# EUROPEAN PATENT APPLICATION

(11) **EP 4 431 524 A2**
(43) Date of publication of application: **18.09.2024**
(21) Application number: 24175650.1
(22) Date of filing: 03.12.2020
(51) Int. Cl.: C07K 14/705

(54) **ENHANCING BLOOD-BRAIN BARRIER DRUG TRANSPORT BY TARGETING ENDOGENOUS REGULATORS**

(30) Priority: 04.12.2019 US 201962943610 P; 25.06.2020 US 202063044048 P
(62) Divisional of application: 20896434.6
(71) Applicant: The Board of Trustees of the Leland Stanford Junior University, Stanford, CA 94305-2038 (US); The United States Government as represented by the Department of Veterans Affairs, Washington DC 20420 (US)
(72) Inventor: Yang, Andrew Chris, Stanford, 20386 (US); Wyss-Coray, Anton, Stanford, 20386 (US)
(74) Representative: Glawe, Delfs, Moll

(57) **Abstract**

Provided herein are methods and compositions for increasing blood-brain barrier permeability in a subject to enhance blood-brain barrier drug transport and methods and composition for identifying agents that regulate blood-brain barrier permeability.

## Description

### CROSS-REFERENCE TO RELATED APPLICATIONS

This application claims the benefit of U.S. Provisional Application No. 62/943,610, filed on December 4, 2019, and U.S. Provisional Application No. 63/044,048, filed on June 25, 2020, the contents of both of which are incorporated by reference herein.

### FIELD

The present invention relates to methods and compositions for increasing blood-brain barrier permeability in a subject to enhance blood-brain barrier drug transport and methods and composition for identifying agents that regulate blood-brain barrier permeability.

### STATEMENT REGARDING FEDERALLY-SPONSORED RESEARCH

This invention was made with Government support under grant numbers DP1 AG053015 and R01 AG059694 awarded by the National Institutes of Health. The Government has certain rights in this invention.

### BACKGROUND

The blood-brain barrier (BBB) maintains the necessary environment for proper brain function, which is thought to occur via the BBB's impermeability to circulating macromolecules. Vascular endothelial cells of the BBB are among the most specialized in the body. With support from abluminal mural cells, the BBB endothelium exhibits unique properties believed to be critical for optimal central nervous system (CNS) function, such as the display of tight junctions and minimal vesicular trafficking (Obermeier et al., Nature Medicine (2013) doi: 10.1038/nm.3407; Ballabh et al., Neurobiol. Dis. (2004) doi:10.1016/j.nbd.2003.12.016; Chow, B. W. & Gu, C., Trends Neurosci. 38, 598-608 (2015); and Blood, T., Barrier, B., Daneman, R. & Prat, A. The Blood-Brain Barrier, 1-23 (2015)). This impermeability throughout healthy life has been reported with various exogenous tracers (Reese, T. S. & Karnovsky, M. J., J. Cell Biol. (1967) doi:10.1083/jcb.34.1.207; Andreone et al., Neuron, 94(3): 581-594 (2017); Park et al., Neuron, 100(1): 167-182.e9 (2018); Yao et al., Nat. Commun., 5: 3413 (2014); Janzer, R. C. & Raff, M. C., Nature, 325(6101): 253-257 (1987); and Saunders et al., Frontiers in Neuroscience, 9: 385 (2015). Several studies have found that the circulatory environment can influence brain function, and specifically regulate neurogenesis, synaptic plasticity, and cognitive performance (Conboy et al., Nature, 433: 760-764 (2005); Villeda et al., Nat. Med. (2014). doi:10.1038/nm.3569; Tingle et al., Nature (2017). doi:doi:10.1038/nature22067; Wyss-Coray, T., Nature, 539: 180-186 (2016); Katsimpardi, L. Science (80-. ). (2014). doi:10.1126/science.1251141; Karnavas et al., J. Exp. Med. (2017). doi:10.1084/jem.20171320; and Gan, K. J. & Südhof, T. C., Proc. Natl. Acad. Sci., 16(25): 12524-12533 (2019). The canonical impermeability of the BBB to exogenous tracers has stimulated exploration regarding the mechanisms of such influence by the periphery on the CNS. At the same time, with each heartbeat, the BBB is endogenously met by a new wave of diverse blood plasma proteins, likely forming a dynamic interface teeming with binding, signaling, and trafficking events.

There remains a need for methods to identify regulators of blood-brain barrier permeability, particularly as regards the aging brain and delivery of therapeutics to treat central nervous system diseases.

### BRIEF SUMMARY OF THE INVENTION

The disclosure provides methods and compositions for increasing blood-brain barrier permeability in a subject. The disclosure further provides methods and compositions for identifying agents that regulate blood-brain barrier permeability.

In some embodiments, the disclosure provides methods and compositions for increasing blood-brain barrier permeability in a subject by administering to the subject an agent that inhibits the activity of alkaline phosphatase protein (ALPL).

The disclosure also provides method for delivering a therapeutic agent to the brain of a subject, which comprises administering to the subject: (a) an agent which inhibits the activity of alkaline phosphatase protein (ALPL); and (b) the therapeutic agent.

The disclosure further provides a method for identifying a protein or other biomolecule that regulates blood-brain barrier permeability in a mammal, which method comprises one or more or each of the steps of: (a) detectably labeling the proteome of blood plasma isolated from a first mammal; (b) introducing the isolated blood plasma comprising the labeled proteome into a second mammal; (c) isolating brain endothelial cells from the second mammal that form the blood-brain barrier; (d) measuring plasma uptake in each of the isolated brain endothelial cells using flow cytometry to produce a population of sorted brain endothelial cells; (e) detecting expression of genes that correlate with plasma uptake in each of the sorted brain endothelial cells; and(f) selecting a protein or other biomolecule encoded by a gene whose expression correlates with increased or decreased plasma uptake in a brain endothelial cell, whereby the protein or other biomolecule regulates blood-brain barrier permeability in the subject.

### BRIEF DESCRIPTION OF THE DRAWING(S)

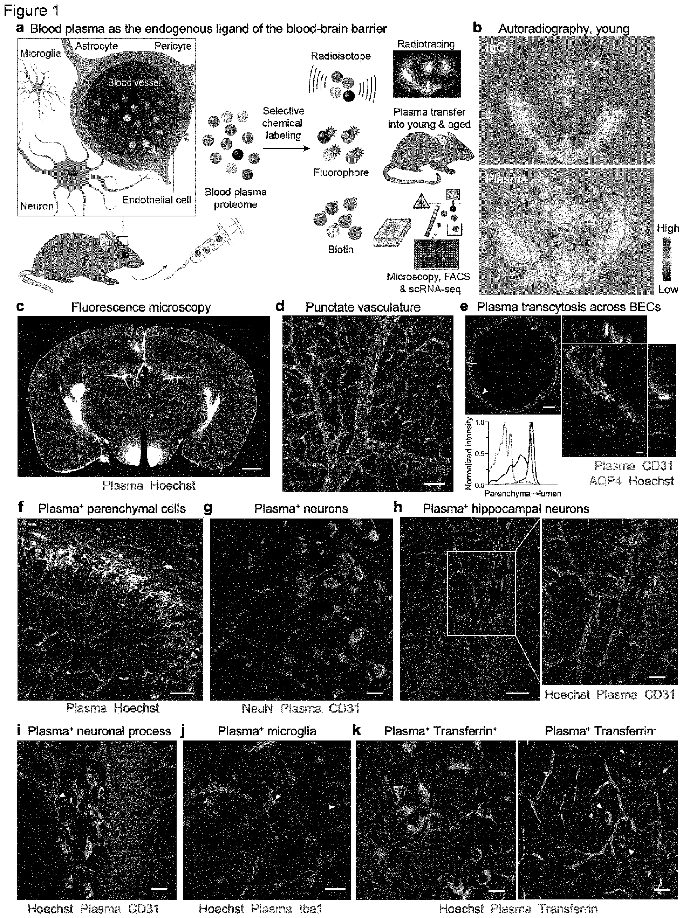

Figure 1a is a schematic diagram of the study rationale and plasma labeling strategy described in Example 1. Figure 1b includes brain audioradiographs of ⁶⁴Cu-labelled IgG (top panel) and Alb/ IgG-depleted plasma (bottom panel) (7.7 MBq matched dose, ~20 µg) injected intravenously into young (3 m.o.) mice and assessed 20 hours later (representative of n=4). ⁶⁴Cu signal ranged from low (black) to high (white) and was overlaid with Nissl staining. Figure 1c is a fluorescence micrograph showing plasma uptake (white) and nuclei (brain) in a coronal brain tile from young (3 m.o.) mice 4 hours after intravenous injection of 150 µL plasma. Scale bar = 1,000 microns (representative image of n=8). Figure 1d is an image showing punctated, plasma⁺ vasculature in the striatum 4 hours after intravenous injection of 150 µL plasma. Scale bar = 20 microns (representative image of n=8 young mice). Figure 1e includes images showing abluminal localization of transcytosed plasma (white) in relation to the CD31+ brain endothelial cell (BECs) layer (red) and AQP4⁺ astrocyte endfeet (green). Figure 1e also includes a histogram depicting fluorescence intensity projections along the white line, normalized within each marker. The inset magnifies the area indicated by the arrowhead. Scale bar = 5 microns (left) and 1 micron (right) (representative image of n=5 young mice). Figure 1f is a fluorescent micrograph showing plasma uptake (white) by parenchymal cells in the hippocampus 20 hours after intravenous injection of 150 µL plasma. Scale bar = 50 microns (representative image of n=8 young mice). Figure 1g is a fluorescent micrograph showing plasma uptake (green) by cortical NeuN⁺ neurons (blue) outside the CD31⁺ vasculature (red) 20 hours after intravenous injection of 150 µL plasma. Scale bar = 20 microns (representative image of n=5 young mice). Figure 1h is a fluorescent micrograph showing plasma uptake (green) by neurons in the dentate gyrus of the hippocampus, beyond the CD31⁺ vasculature (red), 20 hours after intravenous injection of 150 µL plasma. Scale bar = 50 microns (left) and 20 microns (right) (representative image of n=5 young mice). Figure 1i is a fluorescent micrograph showing plasma⁺ (green) neurons adjacent to CD31⁺ vasculature (red) in the dentate gyrus of the hippocampus 20 hours after intravenous injection of 150 µL plasma. White arrowhead indicates a plasma⁺ neuronal process in contact with an endothelial cell. Scale bar = 20 microns (representative image of n=5 young mice). Figure 1j is a fluorescent micrograph showing plasma uptake (green) in cortical Iba1⁺ microglia (red), 20 hours after intravenous injection of 150 µL plasma. Scale bar = 10 microns (representative image of n=5 young mice). Figure 1k includes fluorescent micrographs showing that circulating transferrin (red) reveals neurons double positive for plasma (green) in the cortex (left panel) and neurons solely positive for plasma (right panel). Scale bar = 20 microns (representative image of n=4 young mice). All data were replicated in at least 2 independent experiments. All autoradiography and fluorescence brain sections originated from perfused mice.

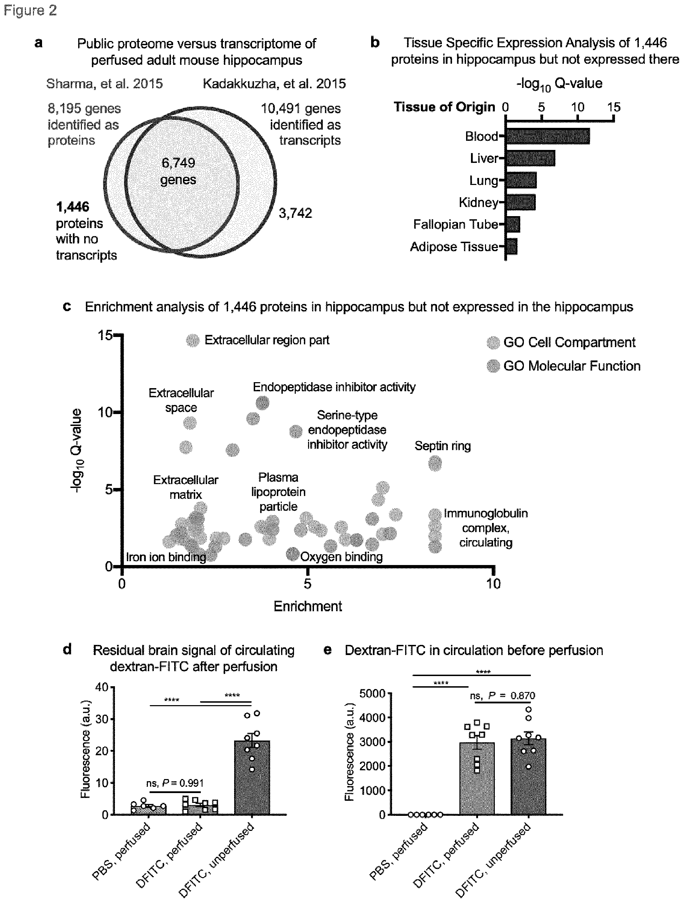

Figure 2a is a schematic diagram showing a comparison of published perfused brain RNA-seq (Kadakkuzha et al., Front. Cell. Neurosci. (2015). doi:10.3389/fncel.2015.00063) and mass spectrometry-based proteomics datasets (Sharma et al., Neurosci., 18: 1819-1831 (2015)), which revealed that 1,446 proteins are present in the hippocampus but corresponding transcripts are not expressed in the hippocampus. These 1,446 proteins then likely migrate from the periphery into the hippocampus. Figure 2b is a graph showing that the 1,446 proteins present but not expressed in the hippocampus in Figure 2a are likely derived from the blood, as assessed by tissue specific expression analysis (Dougherty et al., Nucleic Acids Res. (2010) doi: 10.1093/nar/gkq130). Figure 2c is a plot showing that the 1,446 proteins present but not expressed in the hippocampus in Figure 2a are likely derived from the blood, as assessed by gene ontology analysis (Ashburner et al., Nature Genetics (2000). doi:10.1038/75556; and Gene Ontology Consortium. The Gene Ontology (GO) database and informatics resource. Nucleic Acids Res. (2004). doi:10.1093/nar/gkh036). Figure 2d is a graph depicting results of an assessment of perfusion completeness by measuring residual, intravenously injected 2 mDa dextran-FITC in brain tissue. No significant difference was observed between dextran-FITC-injected and PBS-injected young (3 m.o.) mice after perfusion (n=6-8, one-way ANOVA with Tukey's multiple comparisons correction; mean +/- s.e.m.). Figure 2e is a graph showing circulating 2 mDa dextran-FITC in plasma at the time of sacrifice of mice from Figure 2d (n=6-8, ****P < 0.0001, one-way ANOVA with Tukey's multiple comparisons correction; mean +/s.e.m.). Data in Figures 2d and 2e were replicated in at least 2 independent experiments.

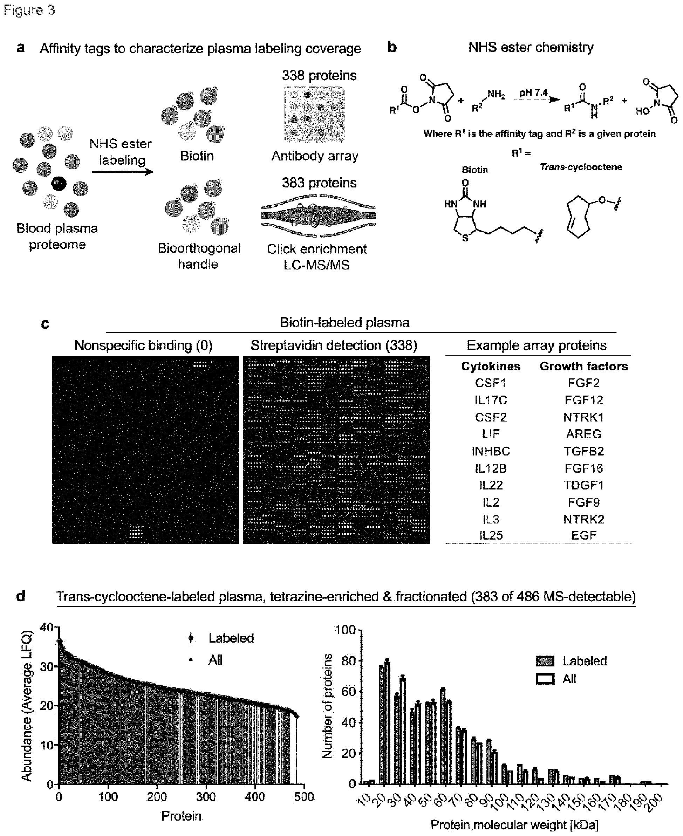

Figure 3a is a schematic diagram depicting an overview of plasma labeling chemistries and detection methods to confirm labeling of hundreds of distinct plasma proteins. Figure 3b is a diagram showing chemoselective labeling of plasma proteins via N-hydroxysuccinimide (NHS) ester chemistry under physiological, non-denaturing conditions (top). Conditions were optimized for each tag to achieve broad and non-perturbative protein labeling. Structures for the small affinity tags biotin and trans-cyclooctene are shown (bottom). Figure 3c includes images of plasma proteins that were labeled with biotin, incubated on an antibody array, and probed with streptavidin. Hundreds of biotinylated proteins were detected across protein groups of diverse activities (n=6). Specific protein-antibody binding indicates that the labeling did not interfere with protein structure. Signal in the unlabeled plasma array (left panel) corresponds to biotinylated positive controls to ensure proper antibody printing. Figure 3d includes graphs illustrating that plasma proteins labeled with the click moiety trans-cyclooctene were enriched on tetrazine beads before fractionation and mass spectrometry-based identification. Labeled proteins spanned abundance and sizes, and showed no overt bias compared to the overall, unlabeled and detectable plasma proteome (n=3, two-way ANOVA; mean +/- s.e.m). Data in Figures 3b and 3c were replicated in at least 2 independent experiments.

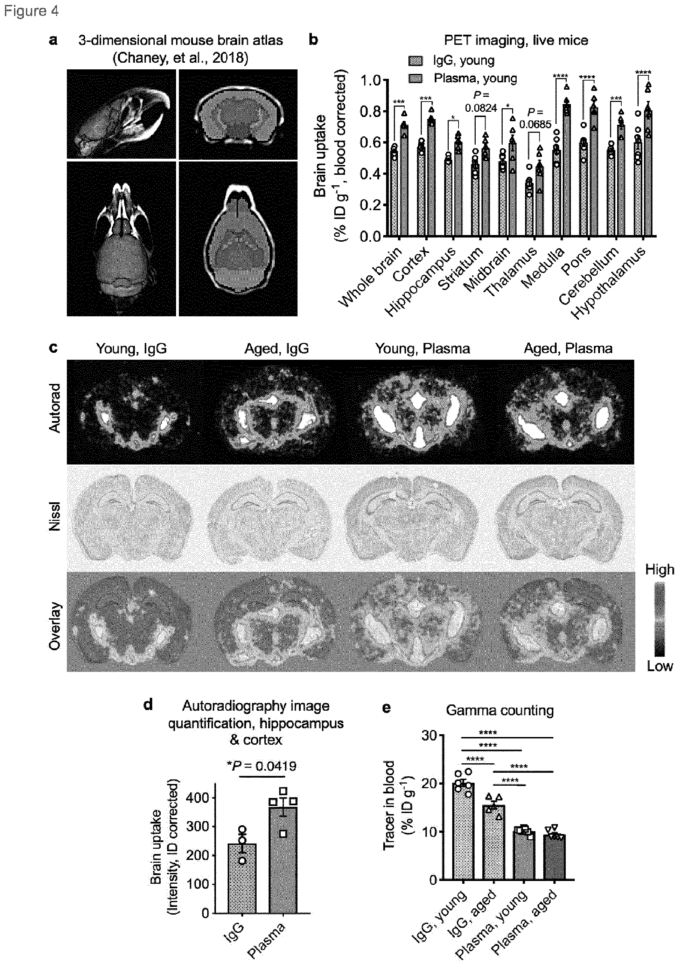

Figure 4a includes images of brain regions demarcated by a 3-dimensional mouse brain atlas rendering for *in vivo* PET signal detection (Chaney et al., J. Nucl. Med. (2018). dol:10.2967/jnumed.118.209155; and Chaney et al., J. Vis. Exp. (2018). doi:10.3791/57243). Figure 4b is a graph showing *in vivo* PET signal detected across brain regions from (a) young (3 m.o.) mice 20 hours after receiving an intravenous dose (ID) of 7.7 MBq (~20 µg) of⁶⁴Cu-labelled IgG or Alb/IgG-depleted plasma. Signal was corrected for activity in corresponding cardiac blood sample at 20 hours (n=7 young IgG, n=6 young plasma, *P < 0.05, ***P < 0.001, ****P < 0.0001, two-way ANOVA; mean +/- s.e.m). Figure 4c includes a series of audioradiographs illustrating *ex vivo* assessment of ⁶⁴Cu-labelled IgG or Alb/IgG-depleted plasma localization in coronal brain sections from injected young (3 m.o.) and aged (22 m.o.) mice. Nissl staining (middle panel) and radioactive signal/Nissl overlay (bottom panel) were used to examine anatomical co-registration (color bar indicates radioactive signal from low (black) to high (white) (n=4 per group). Figure 4d is a graph illustrating quantification of *ex vivo* autoradiography signal intensity in the hippocampus and cortex of young (3 m.o.) mice 20 hours after intravenous injection with 7.7 MBq (~20 µg) of ⁶⁴Cu-labelled IgG or Alb/IgG-depleted plasma (n=3-4, two-sided t-test; mean +/- s.e.m.). Figure 4e is a graph showing ⁶⁴Cu-labelled IgG and Alb/IgG-depleted plasma detected in blood of young (3 m.o.) and aged (22 m.o.) mice 20 hours after injection (n=6 young IgG, n=7 young plasma, n=5 aged IgG, and n=6 aged plasma, ****P < 0.0001, one-way ANOVA with Tukey's correction; mean +/- s.e.m.). All data were replicated in at least two independent experiments.

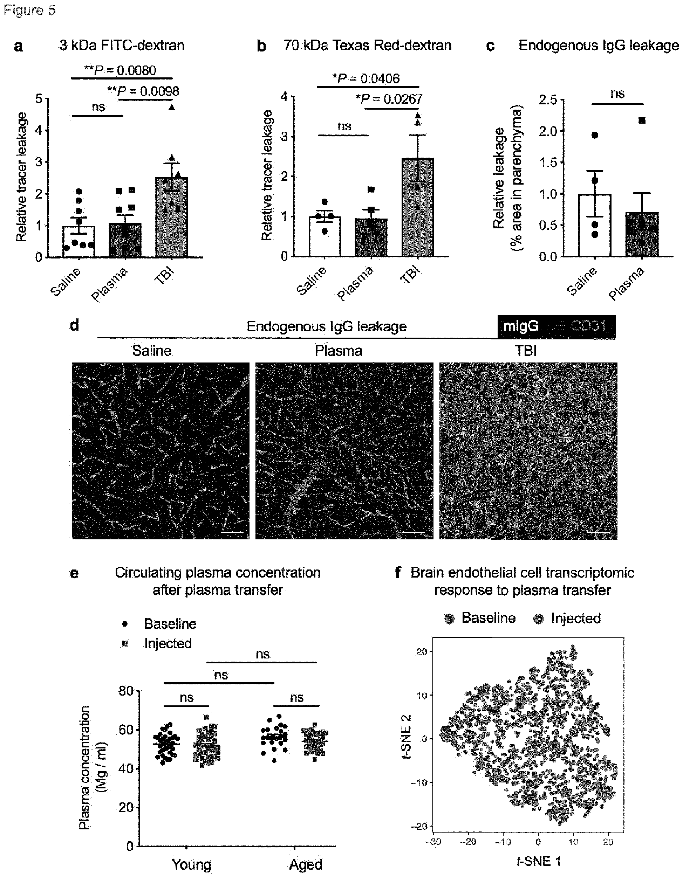

Figure 5a is a graph illustrating BBB permeability after exposure to 150 µL saline or plasma and probing the next day by injections of 3 kDa dextran tracers for quantification by fluorescence plate reader. Figure 5b is a graph illustrating BBB permeability after exposure to 150 µL saline or plasma and probing the next day by injections of 70 kDa dextran tracers for quantification by fluorescence plate reader. Mild traumatic brain injury (TBI) served as a positive control for BBB leakage (for 3 kDa dextran, n=7-9; for 70 kDa dextran, n=4-5, one-way ANOVA with Tukey's multiple comparisons correction; mean +/- s.e.m.). Figure 5c is a graph illustrating BBB permeability after exposure to 150 µL saline or plasma and staining for endogenous immunoglobulin extravasation beyond the vasculature (CD31 area) into the parenchyma (n=4-6, not significant, two-sided t-test; mean +/- s.e.m). Figure 5d includes representative images of endogenous immunoglobulin (white) extravasation into the parenchyma after exposure to 150 µL saline or plasma, or TBI157. Scale bar = 50 microns. Figure 5e is a plot showing plasma protein concentration after injection with 150 µL of plasma at time of sacrifice in young (3 m.o.) and aged (20-24 m.o.) mice (20 hours after injection) used throughout the study compared to plasma concentrations at baseline (n=35 young baseline, n=35 young injected, n=22 aged baseline, n=32 aged injected, not significant, two-way ANOVA; mean +/s.e.m). Figure 5f is a t-SNE plot of brain endothelial cells from young (3 m.o.) mice at baseline and after exposure to 150 µL of plasma, demonstrating no significant perturbation of the cerebrovascular transcriptome upon plasma transfer (n=3). All data were replicated in at least 2 independent experiments.

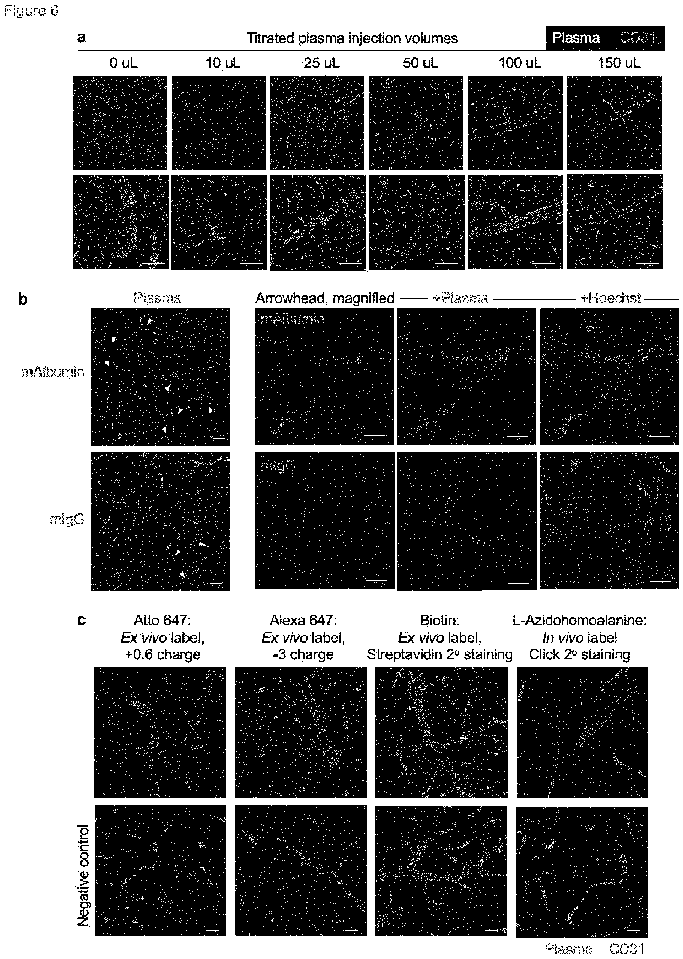

Figure 6a includes representative images of Atto 647N-labeled plasma injected at various volumes, assayed four hours later. 150 µL corresponds to 10-15 mg (0.5 mg/g body weight), with a linear relationship between volume and protein amount (i.e., 15 times less protein injected for 10 µL compared to 150 µL). CD31 marks brain endothelial cells. Scale bar = 100 microns. Figure 6b include images of brain sections from mice injected with Atto 647N-labeled plasma and stained for mouse Albumin (top) and IgG (bottom). For higher magnification images, Albumin and IgG⁺ capillaries were located for imaging (white arrowhead), whereas most of the vasculature was mostly devoid of Albumin and IgG (left). The degree of non-colocalization suggests other plasma factors are responsible for signal detected in the brain. The presence of Albumin and IgG+ capillaries (though a minority) is consistent with the postulation that lysosomal degradation could be an additional mechanism of BBB integrity (Villasenõr et al., Sci. Rep. (2016). doi:10.1038/srep25658). Scale bar = 40 microns (left) and 10 microns (right). Figure 6c includes images of Atto 647N-labeled plasma (left); Alexa Fluor 647-labeled plasma (second to left); biotin-labeled plasma detected by streptavidin 647 slice staining (second to right); and azidohomoalanine-labeled plasma, detected by sDIBO-647 click strain-promoted alkyne-azide cycloadditions (SPAAC) slice staining. Aside from l-azidohomoalanine, plasma was labeled via NHS ester chemistry *ex vivo.* L-azidohomoalanine was performed by feeding mice L-azidohomoalanine in the drinking water and a methionine-deficient chow to enable incorporation of azide-bearing azidohomoalanine in the place of endogenous methionine by the mouse's methionyl-tRNA synthetases (Kiick et al., Proc. Natl. Acad. Sci., 99: 19-24 (2002); and Calve et al., Sci. Rep. (2016). doi:10.1038/srep32377). *In vivo* labeled l-azidohomoalanine plasma was extracted and transfused into separate mice under normal (no L-azidohomoalanine) conditions. Scale bar = 20 microns. All data were replicated in at least 2 independent experiments.

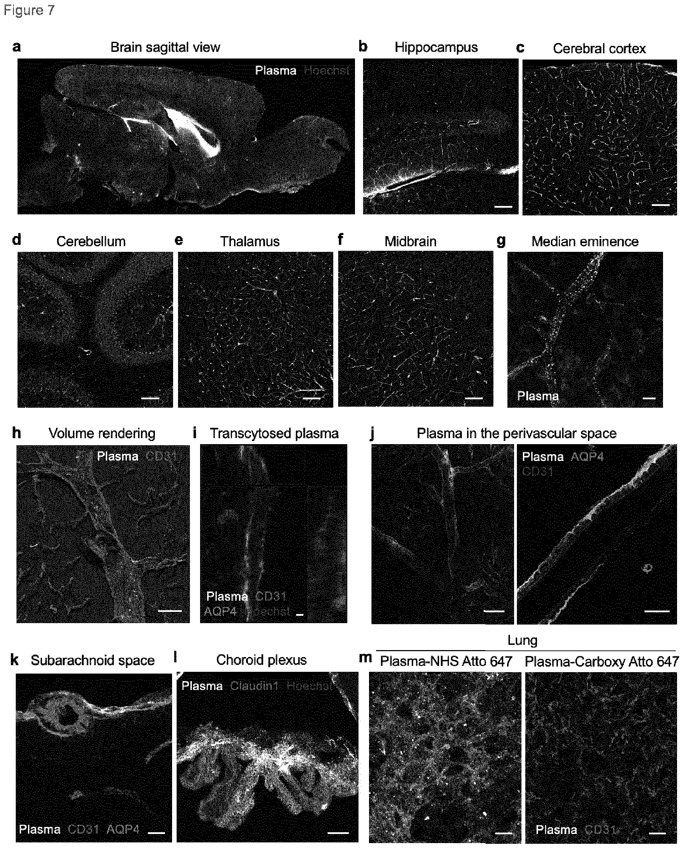

Figure 7a is a representative sagittal image of plasma suffused in the healthy adult brain. Scale bar = 1,000 microns. Figures 7b-7f are images showing plasma permeating the vasculature and parenchyma across brain regions, including hippocampus (Figure 7b), cerebral cortex (Figure 7c), cerebellum (Figure 7d), thalamus (Figure 7e), and midbrain (Figure 7f). Scale bar = 100 microns. Figure 7g is a representative image of plasma in the median eminence. Scale bar = 10 microns. Figure 7h is an image of a representative volume rendering of plasma distribution in a large artery. Plasma accumulates in focal clusters, characteristic of clathrin-mediated endocytosis (Liu et al., J. Cell Biol. (2010). doi:10.1083/jcb.201008117). Scale bar = 50 microns. Figure 7i is a zoomed-in image of a cerebrovascular cross-section denoted by the white line in Figure 1e, showing plasma puncta accumulation within and beyond endothelial cells. Brain endothelial cells are marked by CD31 and astrocytic endfeet by AQP4. Scale bar = 1 micron. Figure 7j includes representative images of plasma in the perivascular space, with plasma tracing the outlines of smooth muscle cells. Scale bar = 50 microns (left panel) and 20 microns (right panel). Figure 7k is a representative image of plasma suffusing the subarachnoid space, localizing beyond endothelial cells (CD31) but within the glia limitans (AQP4). Scale bar = 10 microns. Figure 7l is a representative image of strong plasma signal in the choroid plexus epithelium (Claudin1), suggesting plasma entry into the CSF and a potential role of the glymphatic system in plasma uptake and clearance. Scale bar = 50 microns. Figure 7m includes representative images of plasma accumulation in lung tissue and endothelium (CD31), showing no clear boundary between the vasculature and parenchyma set by the lung endothelium, in contrast to the BBB. There was no signal from residual, nonspecifically-bound free dye even in lung tissue lacking a canonical barrier. Scale bar = 20 microns. All data were replicated in at least 2 independent experiments.

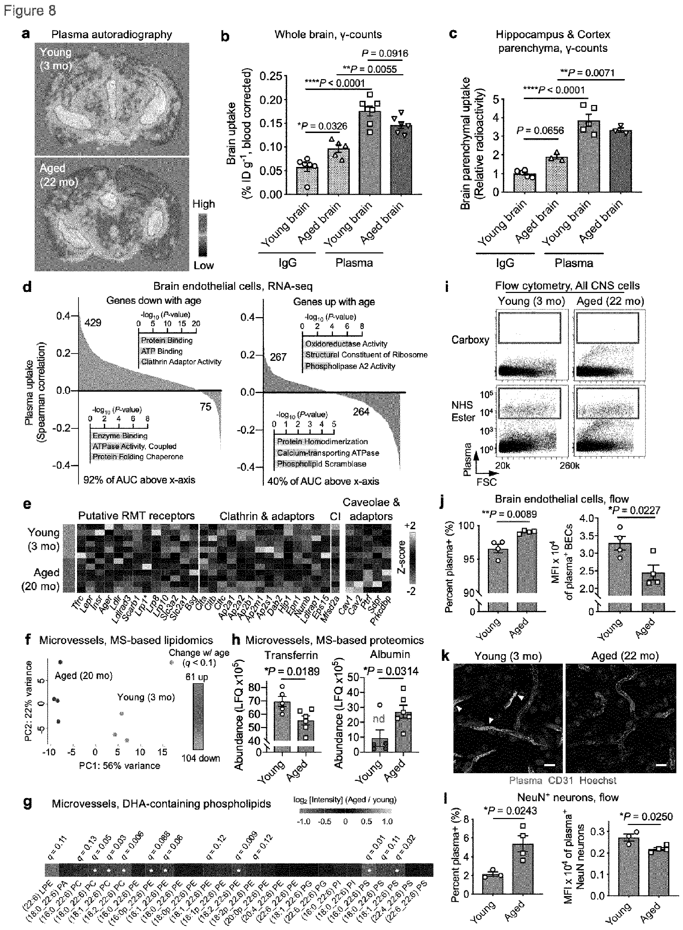

Figure 8a includes brain autoradiographs of ⁶⁴Cu-labelled Alb/IgG-depleted plasma (7.7 MBq matched dose, ~20 µg) injected intravenously into young (3 m.o. - top panel) and aged (22 m.o. - bottom panel) mice and assessed 20 hours later (representative of n=5). ⁶⁴Cu signal ranged from low (black) to high (white) and was overlaid with Nissl staining. Figures 8b and 8c are graphs showing gamma counter quantification of ⁶⁴Cu-labelled IgG and Alb/IgG-depleted plasma in whole brains (Figure 8b) and the de-vascularized hippocampus and cortex parenchyma (Figure 8c) from young (3 m.o.) and aged (22 m.o.) mice 20 hours after intravenous injected dose (ID, 7.7 MBq matched dose, ~20 µg) (n=5-7, one-way ANOVA with Sidak's multiple comparisons correction; mean +/- s.e.m.). Figure 8d is a graph showing brain endothelial cell (BEC) genes downregulated (left, green) and upregulated (right, brown) with age plotted against their contributions to plasma uptake (positive) or inhibition (negative). Figure 8e is an image illustrating gene expression of putative RMT (receptor-mediated transcytosis) receptors, clathrin components, and caveolar components and their inhibitor (CI, caveolar inhibitor) in brain endothelial cells of young (3 m.o.) and aged (20 m.o.) mice (Yousef et al., Nat. Med. (2019). doi:10.1038/s41591-019-0440-4). The asterisk on Lrp1 denotes its abluminal expression (Zlokovic et al., J. Neurochem. (2010). doi:10.1111/j.1471-4159.2010.07002.x). Genes with an average FPKM > 1 in either young or aged endothelial cells are shown. Relative Z-scored values are indicated in graded yellow (high) or blue (low). Figure 8f is a principal component (PC) analysis plot of brain micro vessel lipidomes from young (3 m.o.) and aged (20 m.o.) mice. There were 61 lipids significantly up-regulated and 104 lipids downregulated with age (n=4, each n is a pool of 4 mice (16 young, 16 aged total), q<0.1, Permutation-based FDR of two-sided t-test). Figure 8g is a heatmap showing relative abundance of DHA-containing phospholipids in brain microvessels from aged (20 m.o.) versus young (3 m.o.) mice. The heatmap depicts the log-2 normalized fold change of the MS signal for each lipid species in graded yellow (aged upregulated) or blue (aged downregulated) (n=4, each n is a pool of 4 mice (16 young, 16 aged total), *q<0.1, Permutation-based FDR of two-sided t-test). Figure 8h includes graphs showing relative abundance (label-free quantification, LFQ) of mouse endogenous transferrin (left panel) and albumin (right panel) in brain microvessels from aged (20 m.o.) versus young (3 m.o.) mice. Green dots indicate samples in which albumin was not detected, and values were imputed up to the limit of detection (n=5 young and 6 aged, two-sided t-test; mean +/- s.e.m.). Figure 8i includes representative images of flow cytometry gating of fluorescent plasma uptake across all CNS cells. Plasma fluorescence appears only if plasma is appropriately labeled via NHS ester chemistry prior to intravenous injection (bottom panels), compared to plasma similarly incubated with fluorophore without a conjugative moiety (top panels). Gates were set to mitigate endogenous autofluorescence in fluorescence minus one controls. Figure 8j includes graphs illustrating flow cytometry quantification of plasma uptake by cortical and hippocampal brain endothelial cells (BECs) from young (3 m.o.) and aged (22 m.o.) mice, as assessed by the percent (%) of BECs that are plasma⁺ and mean fluorescence intensity (MFI) of plasma⁺ BECs, 4 hours after intravenous injection of 150 µL plasma (n=4, two-sided t-test; mean +/- s.e.m.). Figure 8k includes images showing plasma uptake (green) in young (3 m.o. - left panel) and aged (22 m.o. - right panel) cortical CD31⁺ vasculature (red) 4 hours after intravenous injection of 150 µL plasma. Arrowheads denote areas with greater accumulation of plasma relative to neighboring cells, indicative of RMT receptor clustering and hierarchical uptake. Scale bar = 10 microns (representative image of n=4 young mice). Figure 8l includes graphs illustrating flow cytometry quantification of plasma uptake by cortical and hippocampal NeuN⁺ neurons from young (3 m.o.) and aged (22 m.o.) mice, as assessed by % of BECs that are plasma⁺ (left panel) and mean fluorescence intensity (MFI) of plasma⁺ BECs (right panel) (n=3-4, two-sided t-test; mean +/- s.e.m.). All data were replicated in at least 2 independent experiments.

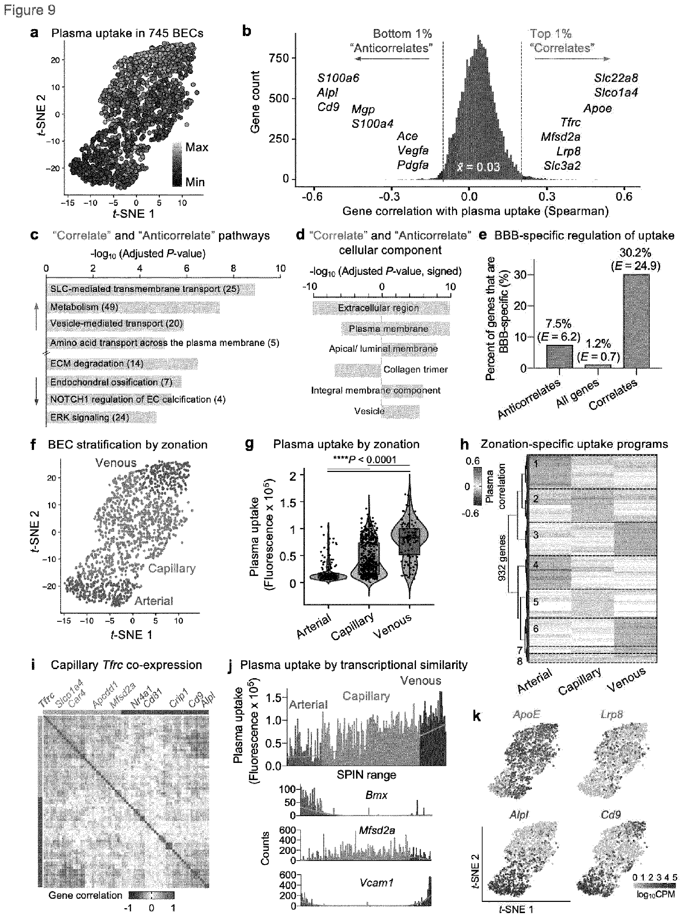

Figure 9a is a t-SNE plot of structured plasma uptake across brain endothelial cells isolated from the cortices and hippocampi of young (3 m.o.) mice (color bar indicates plasma uptake from minimum (black) to maximum (white) (745 BECs from n=3 young mice)). Figure 1b is a graph showing the correlation of 19,899 brain endothelial cell genes with plasma uptake (Spearman). The tail-end top and bottom 1% of genes by correlation are indicated as "Correlates" and "Anticorrelates," respectively, with example genes listed. The distribution median was 0.03. Figure 9c is a schematic diagram illustrating pathway enrichment analysis of "Correlate" and "Anticorrelate" genes, with the number of genes in each pathway listed. Figure 9d is a schematic diagram illustrating gene ontology cellular component enrichment analysis of "Correlate" and "Anticorrelate" genes. Figure 9e is a graph showing enrichment of blood-brain barrier (BBB)-specific genes (Daneman et al., PLoS One (2010). doi:10.1371/journal.pone.0013741) amongst "Correlates" and "Anticorrelates" relative to all genes. Percent of genes and their enrichment (hypergeometric distribution) within "Correlates," "Anticorrelates," and all genes are shown. Figure 9f is a t-SNE plot illustrating brain endothelial cell separation by arterial, capillary, and venous zonation. Figure 9g is a graph illustrating plasma uptake by arterial, capillary, and venous zonation. Figure 9h is a heatmap of gene correlations with plasma uptake (Spearman, red, high; blue, low), which revealed eight major patterns: correlating arterial (1), capillary (2), and venous (3); and anticorrelating arterial (4), capillary (5), venous (6); capillary and venous (7); and global (8). Figure 9i is a matrix illustrating co-expression analysis of "Correlate" (green) and "Anticorrelate" (brown) genes in capillaries expressing the transferrin receptor, Tfrc (Spearman correlation). The matrix was dendrogram-ordered by Euclidian distance and presents genes expressed in at least 15% of capillary cells. Figure 9j includes graphs illustrating plasma uptake across brain endothelial cells sorted by SPIN. Orange curves measure average uptake (LOWESS) within and across vessel segments. Zonation-specific markers are shown in the bottom panels. Figure 9k includes representative t-SNE plots of example "Correlate" and "Anticorrelate" genes. Each dot corresponds to an individual cell, and gene expression levels are indicated by the color spectrum (log10 CPM). All data were replicated in at least 2 independent experiments.

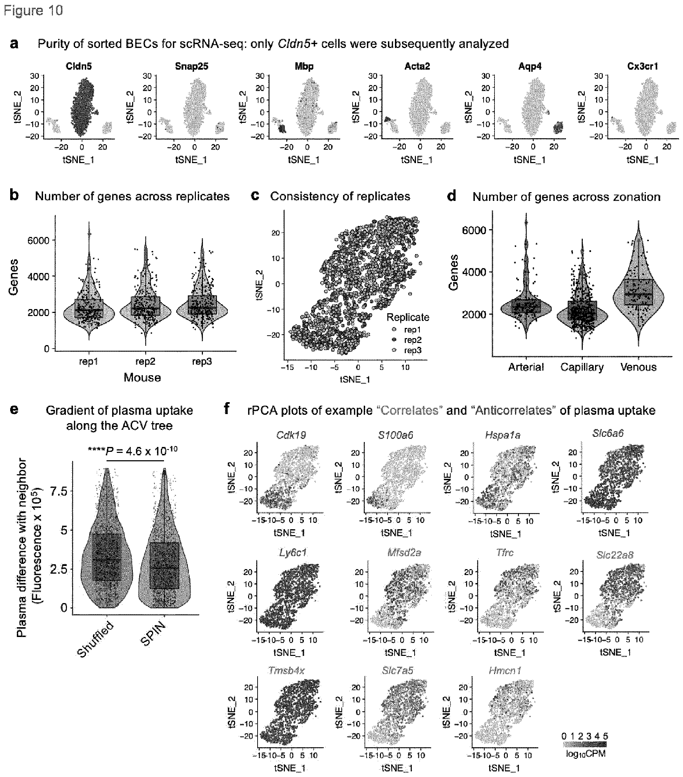

Figure 10a includes t-SNE plots of sorted brain endothelial cells (BECs) from young (3 m.o.) mice that were further filtered to purity using scRNA-seq analysis. Figure 10b is a violin plot of the number of genes expressed per BEC within each biological replicate. Cells in red are outliers. Figure 10c is a t-SNE plot with BECs from separate mice colored distinctly, showing consistency in transcriptomes across replicates. Figure 10d is a violin plot of the number of genes expressed per BEC by zonation. Cells in red are outliers. Figure 10e is a plot illustrating the pairwise difference in plasma uptake across neighboring cells organized by SPIN axis or randomly shuffled. Reduced differences in plasma uptake by SPIN ordering implies a gradation of plasma uptake across the arteriovenous (ACV) tree. Figure 10f includes t-SNE plots illustrating robust PCA (rPCA) plots of "Correlate" and "Anticorrelate" gene expression across BECs. Each dot represents an individual cell, and gene expression levels are indicated by the color spectrum (log10 CPM). All data were replicated in at least 2 independent experiments.

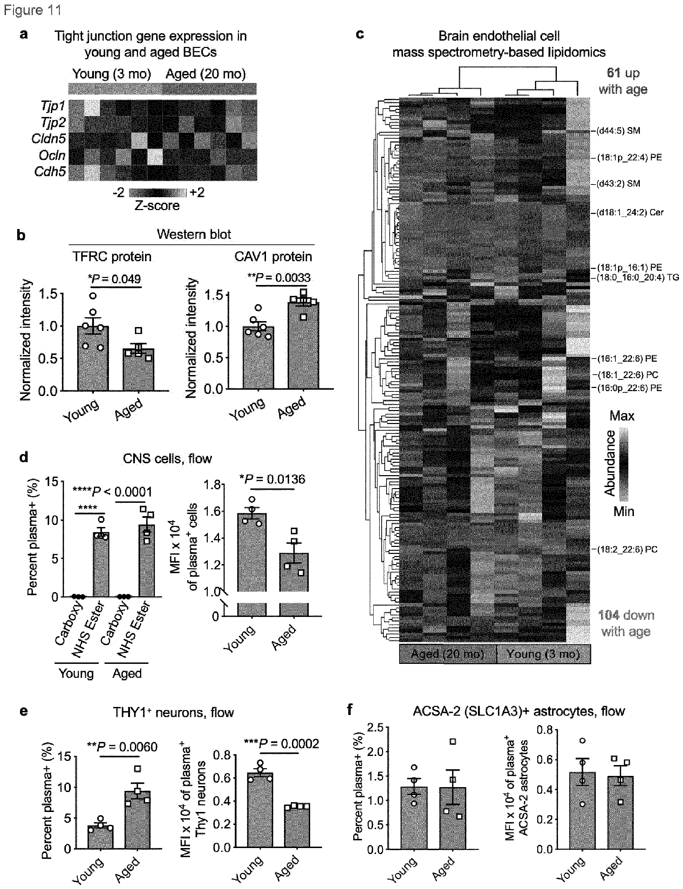

Figure 11a is a heat map illustrating tight junction gene expression from RNA-seq of brain endothelial cells from young (3 m.o.) and aged (20 m.o.) mice (n=6). Relative Z-scored values are indicated in graded yellow (high) or blue (low). Figure 11b includes graphs illustrating Western blot quantification of transferrin receptor (TFRC - left panel) and caveolin 1 (CAV1 - right panel) in young (3 m.o.) and aged (20 m.o.) brain endothelial cells, normalized to histone H3 loading control protein (n=6, two-sided t-test; mean +/- s.e.m.). Figure 11c is a heat map illustrating hierarchical clustering of differentially abundant lipids from unbiased mass spectrometry-based lipidomics of microvessels from young (3 m.o.) and aged (20 m.o.) mice (n=4; each sample is a pool of 4 mice). Data was normalized by Z-score. Lipids with an FDR-corrected q-value below 0.1 were considered significant. Figures 11d-11f include graphs illustrating flow cytometry quantification of plasma uptake across all CNS cells (Figure 11d), Thy1⁺ neurons (Figure 11e), and ACSA-2⁺ astrocytes (Figure 11f) from young (3 m.o.) and aged (22 m.o.) mice (n=4, two-sided t-test; mean +/- s.e.m.). Data were replicated in at least 2 independent experiments.

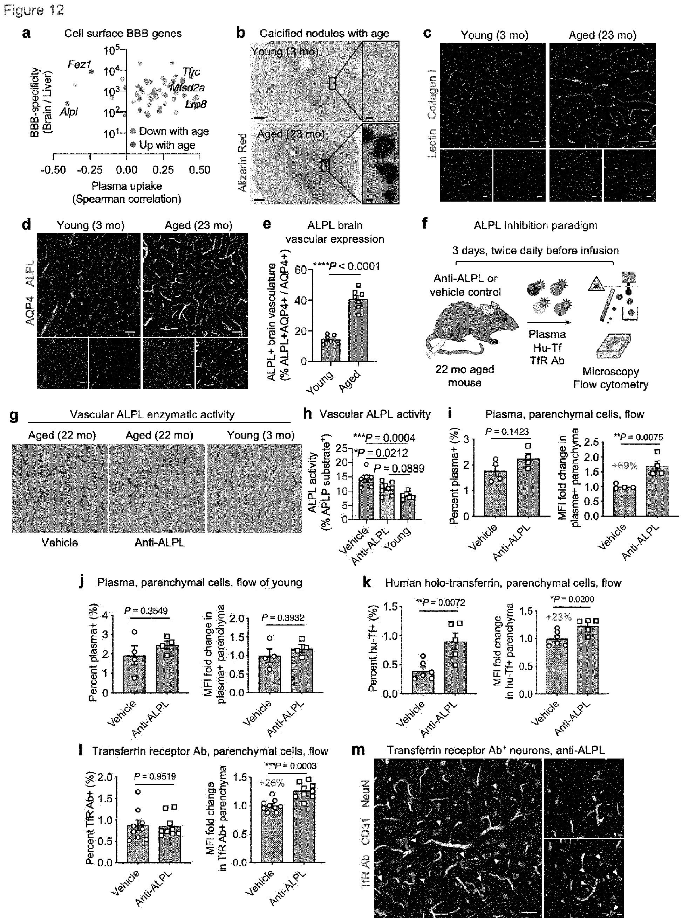

Figure 12a is a dot plot representation of cell-surface, druggable gene candidates on brain endothelial cells. Genes were plotted by their degree of blood-brain barrier specificity (Daneman et al., PLoS One (2010). doi:10.1371/journal.pone.0013741) and correlation with plasma uptake. Genes were colored by their upregulation (brown) or downregulation (green) with age. Figure 12b includes representative images of calcified nodules (Alizarin red staining) detected in aged (23 m.o. - bottom panel) but not young (3 m.o. - top panel) mice, recapitulating nodules seen in pericyte-deficient Pdgfbret/ret mice (Keller et al., Nat. Genet. (2013). doi:10.1038/ng.2723) (representative image of n=5 mice per age). Figure 12c includes representative images of Type I Collagen expression (green) in Lectin⁺ vasculature (red) in the cerebral cortex of young (3 m.o. - left panels) and aged (23 m.o. - right panels) mice. Scale bar = 40 microns. Figure 12d includes representative images of ALPL expression (yellow) in AQP4⁺ vasculature (purple) in the cerebral cortex of young (3 m.o. - left panels) and aged (23 m.o. - right panels) mice. Scale bar = 40 microns. Figure 12e is a graph illustrating quantification of ALPL expression in the cerebral cortex vasculature of young (3 m.o.) and aged (23 m.o.) mice (n=7, two-sided t-test; mean +/- s.e.m.). Figure 12f is a schematic diagram illustrating cerebrovascular ALPL inhibition. Aged (22 m.o.) mice were injected intraperitoneally with vehicle or ALPL inhibitor twice a day for three days before intravenous injection of plasma, human holotransferrin, or transferrin receptor antibody. Uptake in the brain endothelium and parenchyma was assessed by flow cytometry and confocal microscopy of the cortex and hippocampus. Figure 12g includes representative images of ectopic ALPL activity (red) in the cerebral cortex vasculature of aged (22 m.o.) mice treated with ALPL inhibitor (anti-ALPL) or vehicle and in young (3 m.o.) mice. Figure 12h is a graph illustrating quantification of ALPL activity in the cerebral cortex vasculature of aged (22 m.o.) mice treated with ALPL inhibitor (anti-ALPL) or vehicle and in young (3 m.o.) mice (n=6-9, one-way ANOVA with Tukey's multiple comparisons correction; mean +/- s.e.m.). Figures 12i-12l include graphs illustrating flow cytometry quantification of brain parenchymal (CD31-/ CD45-) cell uptake of plasma in aged (22 m.o.) mice (Figure 12i); plasma in young (3 m.o.) mice (Figure 12j); human holo-transferrin (hu-Tf) in aged (22 m.o.) mice (Figure 12k); or transferrin receptor antibody (TfR Ab) in aged (22 m.o.) mice (Figure 12l) after ALPL inhibitor (anti-ALPL) and vehicle treatment, as assessed by the percent (%) of parenchymal cells that are tracer⁺ and the relative mean fluorescence intensity (MFI) of tracer⁺ parenchymal cells (for plasma, n=4; for hu-Tf, n=5-6; for TfR Ab, n=9, two-sided t-test; mean +/- s.e.m.). Tracer refers to plasma, hu-Tf, or TfR Ab Figure 12m includes representative images of transferrin receptor antibody (TfR Ab) in the cortical vasculature (red) and parenchymal cells of ALPL inhibitor-treated aged (22 m.o.) mice. Arrowheads indicate TfR Ab⁺ NeuN neurons. Scale bar = 40 microns (left) and 10 microns (right). Data were replicated in at least 2 independent experiments.

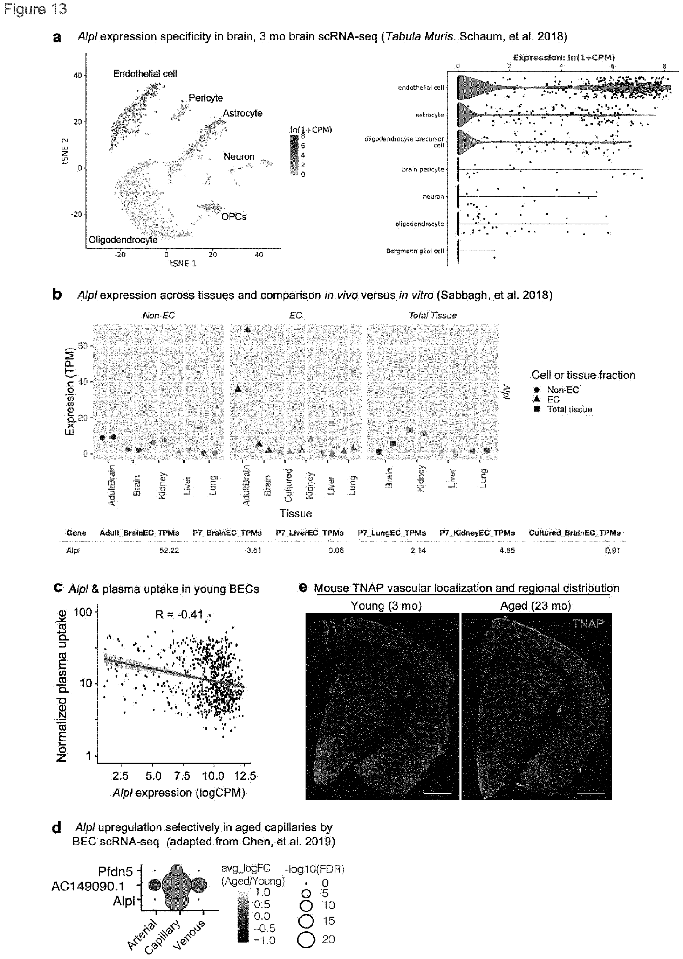

Figure 13a includes t-SNE and violin plots showing scRNA-seq analysis of Alpl expression in CNS cells, log-normalized counts per million reads (CPM). Alpl is expressed mainly in brain endothelial cells (n=7, young mice). Data is from the Tabula Muris Consortium. Figure 13b is a graph showing Alpl expression across endothelial and non-endothelial cells in the CNS, periphery, and tissue culture (transcripts per million, TPM). Alpl is specific to the brain endothelium and expression was lost upon culture. Data is from the Vascular Endothelial Cell Transomics Resource Database (markfsabbagh.shinyapps.io/vectrdb/) (Sabbagh et al., Elife (2018). doi:10.7554/elife.36187). Figure 13c is a plot showing correlation between Alpl expression in young brain endothelial cells (BECs) and plasma uptake from combined flow cytometry index sorting and scRNA-seq. Blue line denotes linear regression, Spearman correlation. Non-Alpl expressing BECs were excluded. Figure 13d is a bubble plot (Chen et al., bioRxiv (2019). doi:10.1101/617258) illustrating that Alpl expression increases specifically in capillaries with age. Figure 13e includes images showing ALPL protein expression in young (3 m.o.) and aged (23 m.o.) brains. Scale bar = 1,000 microns. Data in Figures 13c and 13e were replicated in at least two independent experiments.

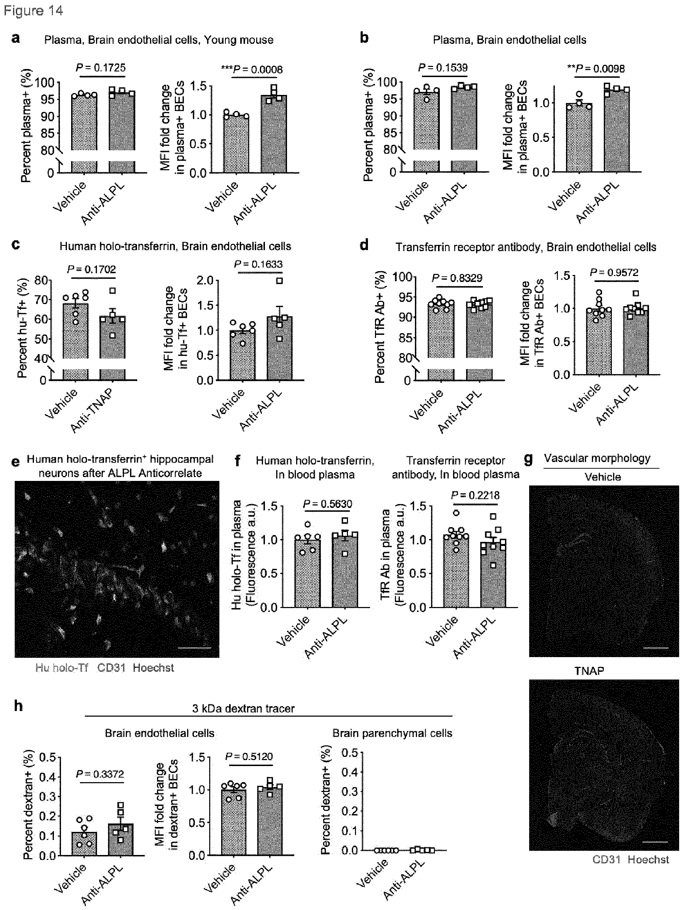

Figures 14a-14d are graphs illustrating flow cytometry quantification of brain endothelial cell uptake of plasma from young mice (3 m.o.) (Figure 14a), plasma from aged mice (22 m.o.) (Figure 14b), human holo-transferrin (hu-Tf) from aged mice (Figure 14c), or transferrin receptor antibody (TfR Ab) from aged mice (Figure 14d) treated with vehicle (grey) or anti-ALPL (green) via IP injections twice daily for three days, as assessed by % of BECs that are tracer⁺ and relative MFI of tracer⁺ BECs (for plasma, n=4; for hu-Tf, n=6 vehicle, n=5 anti-ALPL; for TfR Ab, n=9, two-sided t-test, mean +/- s.e.m.). Tracer refers to plasma, hu-Tf, or TfR Ab. Figure 14e includes representative images of hu- Tr- neurons detected in the cortex of mice treated with ALPL inhibitor. Scale bar = 40 microns. Figure 14f includes graphs showing circulating human holo-transferrin and transferrin receptor antibody in blood plasma upon time of sacrifice to control for and ensure no differences in injected amount (for hu-Tf+, n=6 vehicle, n=5 anti-ALPL; for TfR Ab, n=9, two-sided t-test, mean +/- s.e.m.). Figure 14g include images illustrating overall cerebrovascular morphology, as assayed by CD31 staining, from aged mice (22 m.o.) treated with vehicle or anti-ALPL. Scale bar = 1,000 microns. Data were replicated in at least 2 independent experiments. Figure 14h includes graphs illustrating flow cytometry quantification of brain endothelial cell and brain parenchymal cell uptake of 3 kDa dextran tracer, which probes for disruptions in paracellular permeability, in young mice (3 m.o.) and aged mice (22 m.o.) (n=6 vehicle, n=5 anti-ALPL, two-sided t-test, mean +/- s.e.m.).

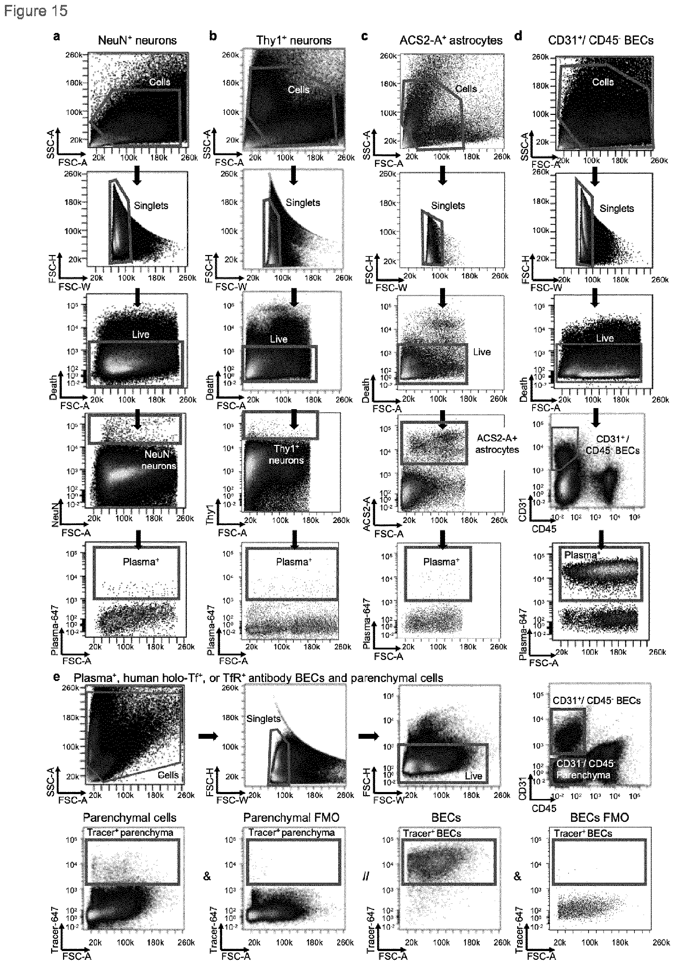

Figure 15a-15d are images illustrating representative gating for plasma⁺, NeuN⁺ neurons (Figure 15a); Thy1 (CD90)⁺ neurons (Figure 15b); ACS2-A⁺ astrocytes (Figure 15c); and CD31⁺/ CD45⁻ brain endothelial cells (BECs) (Figure 15d). For each cell type, plasma⁺ gates were set off FMO negative controls (uninjected) to avoid confounding autofluorescence, especially in aged brains. Figure 15e includes images illustrating representative gating for CD31⁺/ CD45⁻ brain endothelial cells and CD31⁻/CD45⁻ parenchymal cells from aged mice (22 m.o.) after *in vivo* exposure to plasma, human holo-transferrin, or transferrin receptor antibody with or without ALPL inhibitor treatment. Plasma, human holo-transferrin, and transferrin receptor antibody ("tracer") gates were set off FMO negative controls (uninjected) to avoid confounding autofluorescence. Examples shown are from mice injected with transferrin receptor antibody.

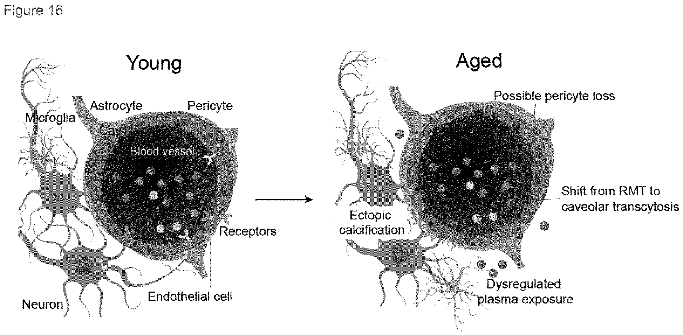

Figure 16 is a schematic diagram illustrating a working model of the shift in BBB transcytosis with age. In healthy adults, brain endothelial cells express high levels of receptors and components of clathrin-coated pits (Simionescu, M. et al. J. Submicrosc. Cytol. Pathol., 20(2): 243-61 (1988); Hervé et al., AAPS J. (2008). doi:10.1208/s12248-008-9055-2) to transport select plasma proteins via receptor-mediated transcytosis (RMT). With age, pericytes might degenerate, promoting vascular calcification and a shift in endothelial transport from ligand-specific receptor-mediated to caveolar transcytosis. Caveolar transcytosis is nonspecific, rendering the aged BBB "leaky" to neurotoxic proteins excluded in health, such as fibrin(ogen), thrombin, and auto-antibodies (Petersen et al., Nature Reviews Neuroscience (2018). doi:10.1038/nrn.2018.13; and Montagne et al., J. Exp. Med. (2017). doi:10.1084/jem.20171406). Loss of RMT receptors complicates drug delivery efforts, including transferrin receptor antibodies in development, and may compromise uptake of peripheral factors necessary for optimal CNS homeostasis.

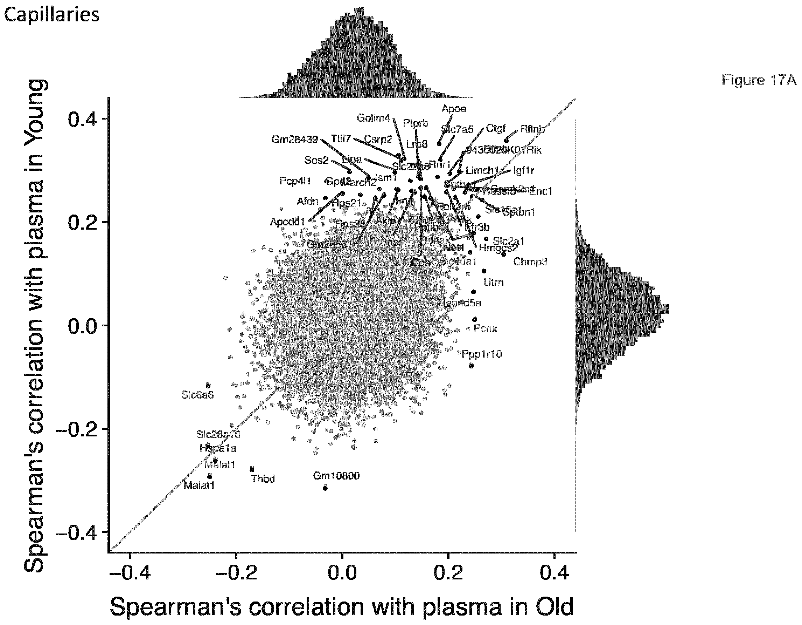

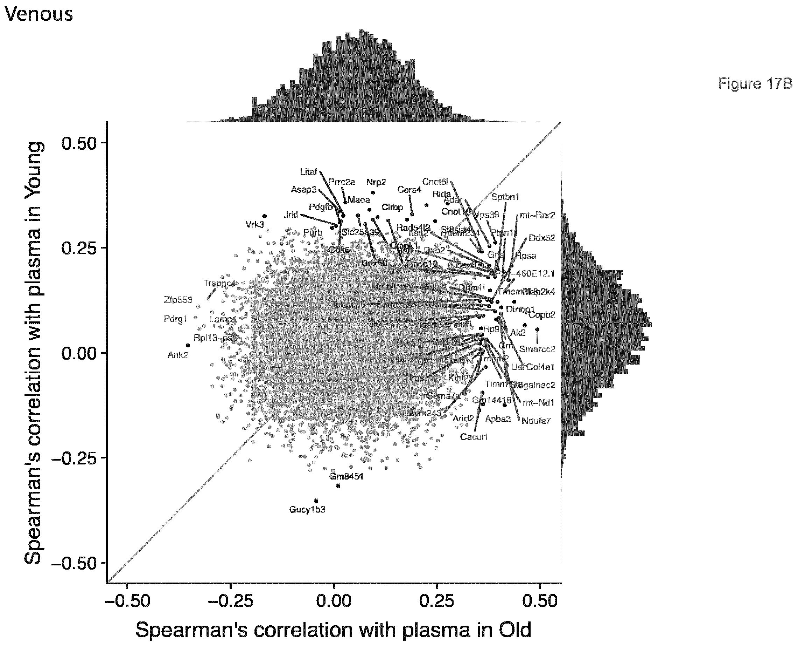

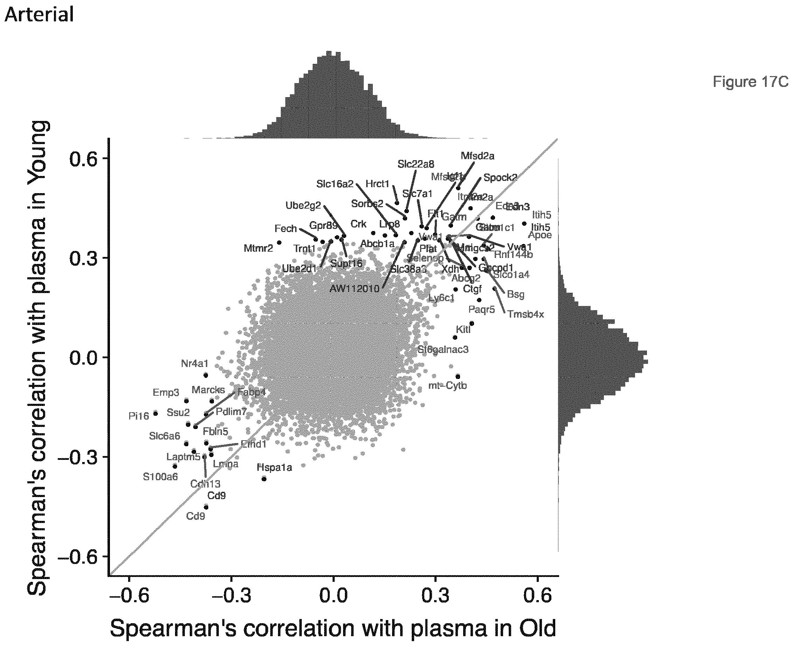

Figures 17A-17C are scatter plots and accompanying histograms showing gene correlates for plasma uptake in brain capillaries (Figure 17A), veins (Figure 17B), and arterioles (Figure 17C) from young and aged mouse brains.

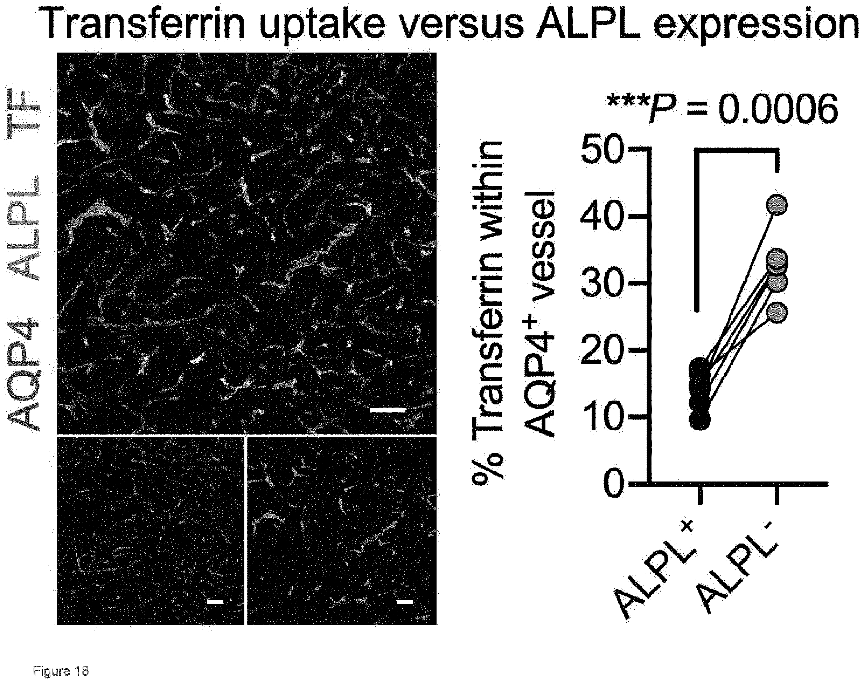

Figure 18 includes images and a graph illustrating enhanced uptake of circulating transferring by ALPL- vaculature in the aged mouse brain (23 mo.) (Scale bar = 40 microns; n=6, paired two-sided t-test; mean +/- s.e.m.).

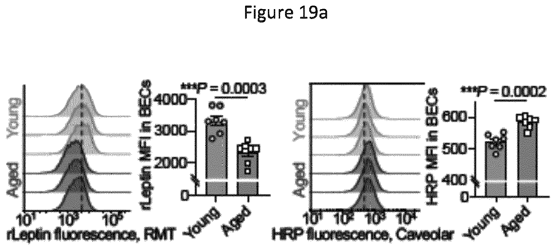

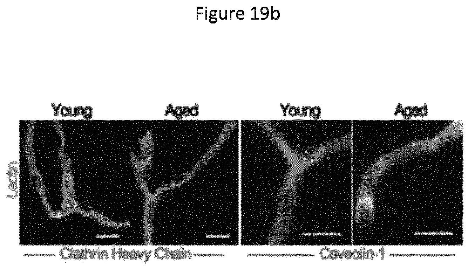

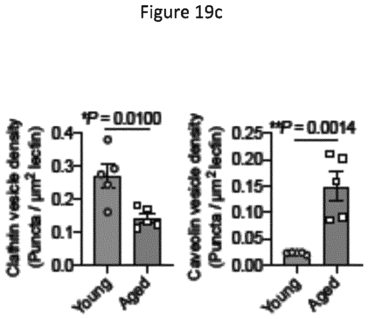

Figure 19a includes histograms and graphs showing MFI recording BEC uptake of recombinant mouse leptin (rLeptin) and horseradish peroxidase (HRP) 4 hours after administration (n=7, two-sided t-test; mean +/- s.e.m.). Figure 19b includes immunofluorescence images of clathrin and caveolar vesicles in acutely isolated microvessels (scale bar = 10 microns). Figure 19c includes graphs showing quantification of clathrin and caveolar vesicles (n=5, two-sided t-test; mean +/- s.e.m.).

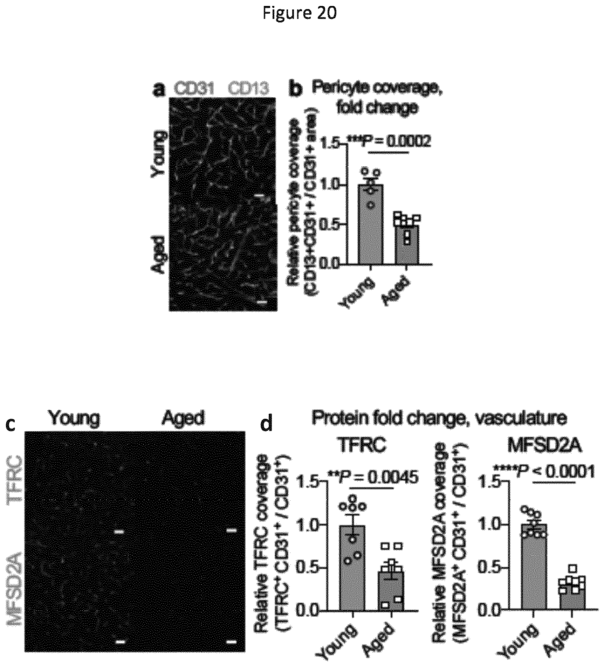

Figure 20a is an image showing cortical CD13+ vascular pericytes and CD31+ endothelial cells in aged and young brains (scale bar = 40 microns). Figure 20b is a graph showing quantification of cortical CD13+ vascular pericytes and CD31+ endothelial cells (n=5 young; n=7 aged, two-sided t-test; mean +/- s.e.m.). Figure 20c is an image showing TFRC and MFSD2A cortical vascular expression (scale bar = 40 microns). Figure 20d is a graph showing quantification of TFRC and MFSD2A cortical vascular expression (n=7 TFRC; n=8 MFSD2A, two-sided t-test; mean +/- s.e.m.).

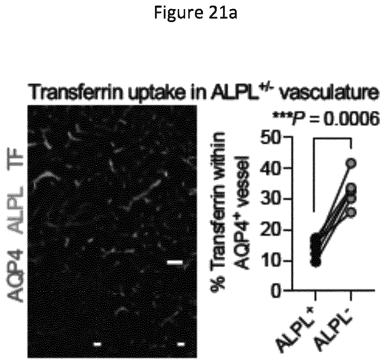

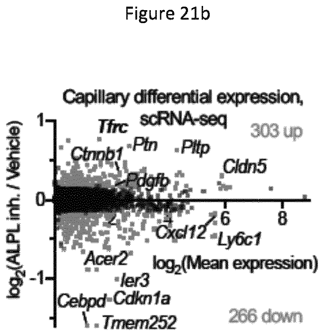

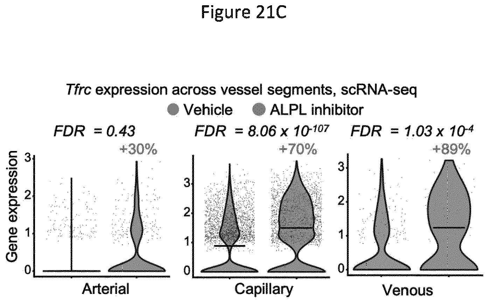

Figure 21a includes an image and histogram showing holo-transferrin uptake in ALPL+/- cortical vasculature (n=6, paired two-sided t-test). Figure 21b is an MA plot of differentially expressed genes (FDR < 0.05) from aged mice after administration of ALPL inhibitor and vehicle (7,036 capillary cells, n=4 mice pooled per group, MAST with Bonferroni correction). Figure 21c includes violin plots showing transferrin receptor (*Tfrc*) expression across vessel segments, following the paradigm in Figure 21b (1,171 arterial, 7,036 capillary, and 430 venous cells, MAST with Bonferroni correction).

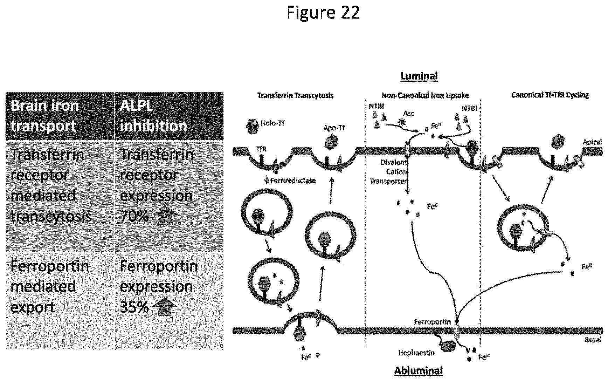

Figure 22 is a schematic diagram illustrating the mechanism of iron transport across the BBB.

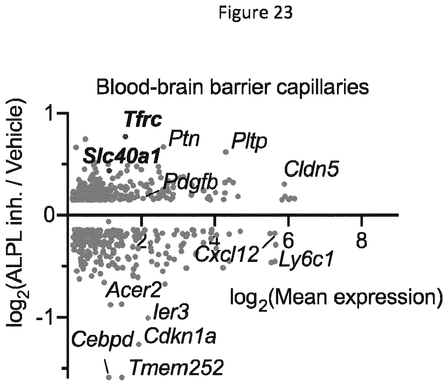

Figure 23 is a graph showing that *Tfrc* and *Slc40a1* are among the top genes upregulated on the BBB upon ALPL inhibition.

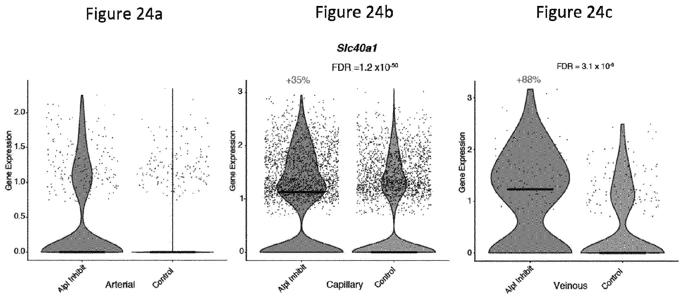

Figures 24a-24c are violin plots showing the magnitude of *Slc40a1* upregulation across brain arterial cells (Figure 24a), capillary cells (Figure 24b), and veinous cells (Figure 24c) upon ALPL inhibition.

### DETAILED DESCRIPTION OF THE INVENTION

The present disclosure is predicated, at least in part, on the development of a screening method to identify proteins and other biomolecules that regulate permeability of the blood-brain barrier. Using the whole blood plasma proteome as a novel discovery tool, as described herein, endogenous proteins and other biomolecules can be directly visualized readily permeating the BBB-protected, healthy adult brain parenchyma. This process is highly regulated by transcriptional programs unique to the brain endothelium, and uptake varies significantly by vessel segment. With age, much of the active regulators of plasma uptake are downregulated, resulting in a shift from receptor-mediated to nonspecific caveolar transcytosis in the BBB, as shown schematically in Figure 16. The present disclosure provides a method for restoring the age-related shift in BBB transcytosis via inhibition of negative regulators of BBB transcytosis.

### Definitions

To facilitate an understanding of the present technology, a number of terms and phrases are defined below. Additional definitions are set forth throughout the detailed description.

As used herein, the terms "nucleic acid," "polynucleotide," "nucleotide sequence," and "oligonucleotide" are used interchangeably and refer to a polymer or oligomer of pyrimidine and/or purine bases, preferably cytosine, thymine, and uracil, and adenine and guanine, respectively (See Albert L. Lehninger, Principles of Biochemistry, at 793-800 (Worth Pub. 1982)). The terms encompass any deoxyribonucleotide, ribonucleotide, or peptide nucleic acid component, and any chemical variants thereof, such as methylated, hydroxymethylated, or glycosylated forms of these bases. The polymers or oligomers may be heterogenous or homogenous in composition, may be isolated from naturally occurring sources, or may be artificially or synthetically produced. In addition, the nucleic acids may be DNA or RNA, or a mixture thereof, and may exist permanently or transitionally in single-stranded or doublestranded form, including homoduplex, heteroduplex, and hybrid states. In some embodiments, a nucleic acid or nucleic acid sequence comprises other kinds of nucleic acid structures such as, for instance, a DNA/RNA helix, peptide nucleic acid (PNA), morpholino nucleic acid (see, e.g., Braasch and Corey, Biochemistry, 41(14): 4503-4510 (2002) and U.S. Patent 5,034,506), locked nucleic acid (LNA; see Wahlestedt et al., Proc. Natl. Acad. Sci. U.S.A., 97: 5633-5638 (2000)), cyclohexenyl nucleic acids (see Wang, J. Am. Chem. Soc., 122: 8595-8602 (2000)), and/or a ribozyme. The terms "nucleic acid" and "nucleic acid sequence" may also encompass a chain comprising non-natural nucleotides, modified nucleotides, and/or non-nucleotide building blocks that can exhibit the same function as natural nucleotides (e.g., "nucleotide analogs").

The terms "peptide," "polypeptide," and "protein" are used interchangeably herein, and refer to a polymeric form of amino acids comprising at least two or more contiguous amino acids, which can include coded and non-coded amino acids, chemically or biochemically modified or derivatized amino acids, and polypeptides having modified peptide backbones.

As used herein, the terms "treatment," "treating," and the like refer to obtaining a desired pharmacologic and/or physiologic effect. Preferably, the effect is therapeutic, i.e., the effect partially or completely alleviates or cures an injury, disease, and/or an adverse symptom attributable to the injury or disease. Similarly, a "therapeutic agent," is any substance, molecule, or compound that is capable of alleviating or curing an injury, disease, and/or adverse symptom when administered to a subject in need thereof. To this end, the methods described herein desirably comprise administering a "therapeutically effective amount" of a therapeutic agent. A "therapeutically effective amount" refers to an amount effective, at dosages and for periods of time necessary, to achieve a desired therapeutic result. The therapeutically effective amount may vary according to factors such as the injury severity, age, sex, and weight of the individual, and the ability of therapeutic agent to elicit a desired response in the individual.

The terms "biomolecule" and "biological molecule," as used herein, refer to any molecule or compound produced by a living organism or cell. Biomolecules typically are organic, and include, for example, large macromolecules (or polyanions) such as proteins, carbohydrates, lipids, and nucleic acids, as well as small molecules such as primary metabolites, secondary metabolites, and natural products.

The term "macromolecule," as used herein, refers to a large polymeric molecule generated by the polymerization of two or more monomers. Macromolecules typically are comprised of thousands of atoms or more. Examples of macromolecules include biopolymers (e.g., nucleic acids, proteins, and carbohydrates) and large non-polymeric molecules (e.g., lipids and macrocycles). Synthetic macromolecules include, for example, common plastics, synthetic fibers, and carbon nanotubes.

The term "immunoglobulin" or "antibody," as used herein, refers to a protein that is found in blood or other bodily fluids of vertebrates, which is used by the immune system to identify and neutralize foreign objects, such as bacteria and viruses. Typically, an immunoglobulin or antibody is a protein that comprises at least one complementarity determining region (CDR). The CDRs form the "hypervariable region" of an antibody, which is responsible for antigen binding (discussed further below). A whole immunoglobulin typically consists of four polypeptides: two identical copies of a heavy (H) chain polypeptide and two identical copies of a light (L) chain polypeptide. Each of the heavy chains contains one N-terminal variable (V_{H}) region and three C-terminal constant (C_{H}1, C_{H}2, and C_{H}3) regions, and each light chain contains one N-terminal variable (V_{L}) region and one C-terminal constant (C_{L}) region. The light chains of antibodies can be assigned to one of two distinct types, either kappa (κ) or lambda (λ), based upon the amino acid sequences of their constant domains. In a typical antibody, each light chain is linked to a heavy chain by disulphide bonds, and the two heavy chains are linked to each other by disulphide bonds. The light chain variable region is aligned with the variable region of the heavy chain, and the light chain constant region is aligned with the first constant region of the heavy chain. The remaining constant regions of the heavy chains are aligned with each other.

The term "monoclonal antibody," as used herein, refers to an antibody produced by a single clone of B lymphocytes that is directed against a single epitope on an antigen. Monoclonal antibodies typically are produced using hybridoma technology, as first described in Köhler and Milstein, Eur. J. Immunol., 5: 511-519 (1976). Monoclonal antibodies may also be produced using recombinant DNA methods (see, e.g., U.S. Patent 4,816,567), isolated from phage display antibody libraries (see, e.g., Clackson et al. Nature, 352: 624-628 (1991)); and Marks et al., J. Mol. Biol., 222: 581-597 (1991)), or produced from transgenic mice carrying a fully human immunoglobulin system (see, e.g., Lonberg, Nat. Biotechnol., 23(9): 1117-25 (2005), and Lonberg, Handb. Exp. Pharmacol., 181: 69-97 (2008)). In contrast, "polyclonal" antibodies are antibodies that are secreted by different B cell lineages within an animal. Polyclonal antibodies are a collection of immunoglobulin molecules that recognize multiple epitopes on the same antigen.

The terms "fragment of an antibody," "antibody fragment," and "antigen-binding fragment" of an antibody are used interchangeably herein to refer to one or more fragments of an antibody that retain the ability to specifically bind to an antigen (see, generally, Holliger et al., Nat. Biotech., 23(9): 1126-1129 (2005)). Any antigen-binding fragment of the antibody described herein is within the scope of the invention. The antibody fragment desirably comprises, for example, one or more CDRs, the variable region (or portions thereof), the constant region (or portions thereof), or combinations thereof. Examples of antibody fragments include, but are not limited to, (i) a Fab fragment, which is a monovalent fragment consisting of the V_{L}, V_{H}, C_{L}, and C_{H1} domains, (ii) a F(ab')₂ fragment, which is a bivalent fragment comprising two Fab fragments linked by a disulfide bridge at the hinge region, (iii) a Fv fragment consisting of the V_{L} and V_{H} domains of a single arm of an antibody, (iv) a Fab' fragment, which results from breaking the disulfide bridge of an F(ab')₂ fragment using mild reducing conditions, (v) a disulfide-stabilized Fv fragment (dsFv), and (vi) a domain antibody (dAb), which is an antibody single variable region domain (V_{H} or V_{L}) polypeptide that specifically binds antigen.

The term "recombinant," as used herein, means that a particular nucleic acid (DNA or RNA) is the product of various combinations of cloning, restriction, polymerase chain reaction (PCR) and/or ligation steps resulting in a construct having a structural coding or non-coding sequence distinguishable from endogenous nucleic acids found in natural systems. DNA sequences encoding polypeptides can be assembled from cDNA fragments or from a series of synthetic oligonucleotides to provide a synthetic nucleic acid which is capable of being expressed from a recombinant transcriptional unit contained in a cell or in a cell-free transcription and translation system. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions, and may act to modulate production of a desired product by various mechanisms. Alternatively, DNA sequences encoding RNA that is not translated may also be considered recombinant. Thus, the term "recombinant" nucleic acid also refers to a nucleic acid which is not naturally occurring, e.g., is made by the artificial combination of two otherwise separated segments of sequence through human intervention. This artificial combination is often accomplished by either chemical synthesis means, or by the artificial manipulation of isolated segments of nucleic acids, e.g., by genetic engineering techniques. Such is usually done to replace a codon with a codon encoding the same amino acid, a conservative amino acid, or a non-conservative amino acid. Alternatively, the artificial combination may be performed to join together nucleic acid segments of desired functions to generate a desired combination of functions. This artificial combination is often accomplished by either chemical synthesis means, or by the artificial manipulation of isolated segments of nucleic acids, e.g., by genetic engineering techniques. When a recombinant polynucleotide encodes a polypeptide, the sequence of the encoded polypeptide can be naturally occurring ("wild type") or can be a variant (e.g., a mutant) of the naturally occurring sequence. Thus, the term "recombinant" polypeptide does not necessarily refer to a polypeptide whose sequence does not naturally occur. Instead, a "recombinant" polypeptide is encoded by a recombinant DNA sequence, but the sequence of the polypeptide can be naturally occurring ("wild type") or non-naturally occurring (e.g., a variant, a mutant, etc.). Thus, a "recombinant" polypeptide is the result of human intervention, but may comprise a naturally occurring amino acid sequence.

The term "small molecule," as used herein, refers to a low molecular weight (< 900 daltons) organic compound that may regulate a biological process, with a size typically on the order of 1 nm. Small molecules exhibit a variety of biological functions and may serve a variety applications, such as in cell signaling, as pharmaceuticals, and as pesticides. Examples of small molecules include, but are not limited to, amino acids, fatty acids, phenolic compounds, alkaloids, steroids, bilins, retinoids, etc.

The term "proteome," as used herein, refers to the complete set of proteins expressed by an organism or a particular set of proteins produced at a specific time in a particular cell or tissue type. The proteome of a particular organism, cell, or tissue actively changes in response to various factors, including the developmental stage of the organism, cell, or tissue, and both internal and external conditions.

The terms "subject" and "patient" are used interchangeably herein and refer to any vertebrate, including, but not limited to, a mammal (e.g., cow, pig, camel, llama, horse, goat, rabbit, sheep, hamsters, guinea pig, cat, dog, rat, and mouse, a non-human primate (for example, a monkey, such as a cynomolgous or rhesus monkey, chimpanzee, etc.) and a human). In some embodiments, the subject may be a human or a non-human. In some embodiments, the subject is a human.

### Blood-Brain Barrier Permeability

The disclosure provides a method for increasing blood-brain barrier permeability in a subject, so as to enhance delivery of macromolecules (e.g., therapeutic agents) to the brain of a subject suffering from a central nervous system (CNS) disease or disorder.

The term "blood-brain barrier (BBB)" is used to describe the unique properties of the microvasculature of the central nervous system. The BBB is a tight-knit layer of endothelial cells (ECs) that coats 400 miles of capillaries and blood vessels in the brain and forms the lumen of the brain microvasculature (Ransohoff et al., Nature Rev. Immun., 3: 569-581 (2003); Abbott et al., Neurobiol. Dis., 3 7: 13-25 (2010)). The BBB achieves a barrier function through tight junctions between endothelial cells that regulate the extravasation of molecules and cells into and out of the central nervous system (CNS) (Abbott et al., *supra*). The presence of specific transport systems within the capillary endothelial cells assures that the brain receives, in a controlled manner, all of the compounds required for normal growth and function. In many instances, these transport systems consist of membrane-associated proteins, which selectively bind and transport certain molecules across the barrier membranes. These transporter proteins are known as solute carrier transporters. This heavily restricting barrier capacity allows BBB ECs to tightly regulate CNS homeostasis, which is critical to allow for proper neuronal function, as well as protect the CNS from toxins, pathogens, inflammation, injury, and disease.

The restrictive nature of the BBB provides an obstacle for drug delivery to the CNS, and major efforts have been made to modulate or bypass the BBB for delivery of therapeutics (Larsen et al., Curr Top Med Chem., 14(9): 1148-60 (2014); and Daneman R. and Prat A., Cold Spring Harb Perspect Biol, 7(1): a020412 (2015)). Indeed, it has been estimated that more than 98% of small-molecule drugs less than 500 Da in size do not cross the BBB (Pardridge, "Brain Drug Targeting: the Future of Brain Drug Development," Cambridge University Press, Cambridge, UK (2001); Pardridge, NeuroRx, 2: 3-14 (2005); and U.S. Patent Application Publication 2013/0224110). Current strategies for CNS drug-delivery fall into three broad categories: chemical BBB disruption, physical BBB disruption, and drug modification.

Loss of some or most of the BBB barrier properties during neurological diseases including stroke, multiple sclerosis (MS), brain traumas, and neurodegenerative disorders, is a major component of the pathology and progression of these diseases (Zlokovic, B.V., Neuron, 57(2): 178-201 (2008); and Daneman R, Ann Neurol. Nov; 72(5):648-72 (2012)). BBB dysfunction also can lead to ion dysregulation, altered signaling homeostasis, as well as the entry of immune cells and molecules into the CNS, all of which can lead to neuronal dysfunction and degeneration.

In some embodiments, the disclosure provides a method for increasing blood-brain barrier permeability in a subject, which comprises administering to the subject an agent which inhibits the activity of alkaline phosphatase protein (ALPL) in the brain. In addition to the endothelial cells of the blood capillaries that make up the BBB, the epithelial cells of the choroid plexus ("CP"), which separate the blood from the cerebrospinal fluid ("CSF") of the central nervous system ("CNS"), together function as the CNS barrier. Thus, in some embodiments, the method for increasing BBB permeability in a subject may increase the permeability of the BBB, the CP, and/or the CNS barrier.

Alkaline phosphatases are membrane-bound glycoproteins that hydrolyze various monophosphate esters at a high pH (Weiss et al., Proc. Nat. Acad. Sci., 83: 7182-7186 (1986)). Liver/bone/kidney alkaline phosphatase, also known as tissue-nonspecific alkaline phosphatase (TNAP), acts physiologically as a lipid-anchored phosphoethanolamine (PEA) and pyridoxal-5-prime-phosphate (PLP) ectophosphatase. Tissue-nonspecific alkaline phosphatase is encoded by the ALPL gene (Weiss et al., *supra*). The deduced 524-amino acid ALPL protein has a presumed signal peptide of 17 amino acids and a predicted molecular mass of 57.2 kD. The ALPL protein shares 52% sequence identity with placental alkaline phosphatase. The ALPL protein is a key effector of bone calcification and is essential for normal skeletal development, as hypomorphic mutations in ALPL lead defective mineralization in hypophosphatasia patients (Sheen et al., J Bone Miner Res., 30(5): 824-836 (2015); Murshed et al., Genes Dev., 19(9): 1093-1104 (2005); Lomashvili et al., Kidney Int., 85(6): 1351-1356 (2014); Savinov et al., J Am Heart Assoc., 4(12): e002499 (2015); Romanelli et al.; PLoS One, 12(10): e0186426 (2017); and Whyte et al., N Engl J Med., 366(10): 904-913 (2012)). As demonstrated herein (see Examples), ALPL is upregulated in the BBB of aged brains and inhibition of ALPL enhances brain uptake of plasma as well as transferrin and a transferrin receptor antibody. The nucleic acid sequence of the ALPL gene is publicly available from, for example, the National Center for Biotechnology Information's (NCBI) GenBank database under Accession No. NG_008940.1. ALPL amino acid sequences are publicly available from, e.g., the GenBank database under Accession Nos. NP_001356734.1, NP_001356733.1, and NP_001356732.1.

Inhibition of ALPL activity may increase BBB permeability by any suitable amount or degree. For example, ALPL inhibition may increase BBB permeability by about 2, 3, 4, 5, 6, 7, 8, 9, 10, 15, 20, or 25-fold, or a range defined by any two of the foregoing values. The increase in BBB permeability may occur for any suitable duration. For example, the increase in BBB permeability may last for 5 minutes, 10 minutes, 15 minutes, 20 minutes, 25 minutes, 30 minutes, 1 hour, 2 hours, 3 hours, 4 hours, 5 hours, 6 hours, 7 hours, 8 hours, 9 hours, 10 hours, 15 hours, 16 hours, 17 hours, 18 hours, 19 hours, 20 hours, 24 hours, or longer.

The phrase "inhibiting ALPL," as used herein, refers to the ability of a substance or method to interfere with the expression and/or biological activity or function of ALPL. The degree of inhibition may be partially complete (e.g., 10% or more, 25% or more, 50% or more, or 75% or more), substantially complete (e.g., 85% or more, 90% or more, or 95% or more), or fully complete (e.g., 98% or more, or 99% or more). Inhibition of ALPL as disclosed herein may involve interfering with or inhibiting the biological activity of ALPL. ALPL biological activity may be inhibited using any suitable agent. In one embodiment, for example, inhibiting ALPL comprises contacting the subject with an agent that inhibits activity of the ALPL protein. Any suitable agent that inhibits alkaline phosphatases may be used in the disclosed method. Such agents include, but are not limited to, phosphate derivatives, phosphonates, vanadate, arsenate, and homoarginine (Fernley, H.N. and Walker, P.G., Biochem. J., 104(3): 1011-1018 (1967); Shirazi et al., Biochem J., 194(3): 803-809 (1981)) or other small molecules that inhibit enzyme activity. Small molecules that inhibit tissue-nonspecific alkaline phosphatase (i.e., the ALPL protein) include, but are not limited to, aryl sulfonamides, such as those described in, e.g., Dahl et al., J. Med. Chem., 52(21): 6919-6925 (2009) and WO 2013/126608, each of which is herein incorporated by reference in its entirety. Other inhibitors include, for example, small molecules containing pyrazole, triazole, or imidazole scaffolds (see, e.g., Chung et al., Molecules, 15.5: 3010-3037 (2010)), small molecules that interfere with ALPL dimerization, and small molecules linked to degrons to induce protein degradation. ALPL inhibitors also include antibodies directed against ALPL (e.g., monoclonal or polyclonal antibodies, or antigen-binding fragments thereof, raised in any suitable animal species).

In some embodiments, a combination of two or more agents that inhibit ALPL may be administered to a subject. For example, any of the small molecule inhibitors described herein may be administered simultaneously or sequentially with other protein ligands that have receptors or transporters on the blood brain barrier (e.g., leptin or recombinant antibodies). Anti-ALPL antibodies, which are commercially available from a variety of sources, may also be administered simultaneously or sequentially with other protein ligands that have receptors or transporters on the blood brain barrier.

In other embodiments, inhibiting ALPL in the subject comprises inhibiting expression of ALPL. Inhibition of ALPL expression can be at the mRNA or protein level and can result from decreased synthesis, increased degradation, or both. In certain embodiments, the method comprises inhibiting expression of the gene encoding ALPL. ALPL gene expression may be inhibited using any suitable agent and/or method known in the art. For example, ALPL gene expression may be inhibited using an inhibitory nucleic acid molecule, including, for example, a small interfering RNA (siRNA), a short hairpin RNA (shRNA), an antisense oligonucleotide, an aptamer, or a ribozyme. Alternatively, ALPL gene expression may be inhibited by introducing one or more mutations (e.g., insertion or deletion of nucleic acids or point mutation) into the ALPL gene which impairs or abolishes transcription. In some embodiments, all or part of the ALPL gene is deleted. Any number of nucleic acids may be deleted from the ALPL gene, so long as the deletion is sufficient to obliterate or impair gene transcription or gene function. Desirably, the entire ALPL gene is removed or deleted in suitable cells (e.g., brain endothelial cells). Any suitable "knock-out" technology for inactivating genes may be used to inhibit ALPL gene expression, a variety of which are known in the art. Such methods include, but are not limited to, homologous recombination, site-specific nucleases (e.g., CRISPR/Cas9 systems, zinc-finger nucleases), and conditional knock-out systems (e.g., Cre/lox technology). Gene editing technology is further described in, e.g., Appasani, K. (ed.), Genome Editing and Engineering: From TALENs, ZFNs and CRISPRs to Molecular Surgery, 1st ed., Cambridge University Press (2018)).

### Delivery of Therapeutic Agents Across the Blood-Brain Barrier

The disclosure also provides a method for delivering a therapeutic agent to the brain of a subject in need thereof, which comprises administering to the subject: (a) an agent which inhibits the activity of alkaline phosphatase protein (ALPL) in the brain; and (b) the therapeutic agent. The therapeutic agent may be administered to the subject simultaneously with the agent that inhibits ALPL activity in the brain. Alternatively, the therapeutic agent may be administered to the subject before or after administration of the agent that inhibits ALPL activity in the brain. When the therapeutic agent and the agent that inhibits ALPL activity are administered separately, administration of each agent may be separated by up to 5 minutes, 10 minutes, 15 minutes, 30 minutes, 1 hour, 2 hours, 3 hours, 4 hours, 5, hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, or 24 hours.

Any suitable therapeutic agent may be administered to the subject. Ideally, the therapeutic agent is indicated for the treatment of a disease or disorder of the central nervous system (CNS). In some embodiments, the therapeutic agent is a macromolecule. The macromolecule may be a biopolymer, such as a nucleic acid sequence, a protein or polypeptide, or a carbohydrate. A macromolecular therapeutic agent may be of any suitable size. For example, a macromolecular therapeutic agent may be about 150 kDa to about 70,000 kDa in size (e.g., about 200 kDa, about 500 kDa, about 1,000 kDa, about 2,000 kDa, about 5,000 kDa, about 10,000 kDa, about 15,000 kDa, about 20,000 kDA, about 30,000 kDa, about 40,000 kDa, about 50,000 kDa, or about 60,000 kDa in size, or a range defined by any two of the foregoing values).

In certain embodiments, the macromolecular therapeutic agent may be a bioactive protein or peptide. Examples of such proteins include antibodies, enzymes, steroids, growth hormone and growth hormone-releasing hormone, gonadotropin-releasing hormone and its agonist and antagonist analogues, somatostatin and its analogues, gonadotropins, peptide T, thyrocalcitonin, parathyroid hormone, glucagon, vasopressin, oxytocin, angiotensin I and II, bradykinin, kallidin, adrenocorticotropic hormone, thyroid stimulating hormone, insulin, glucagon, and analogs or derivatives of any of the foregoing molecules. The protein or peptide can be a synthetic or a naturally occurring peptide, including a variant or derivative of a naturally occurring peptide. A peptide therapeutic agent can be a linear peptide, cyclic peptide, constrained peptide, or a peptidomimetic.

In some embodiments, a protein or peptide therapeutic agent may specifically bind to a target protein or structure associated with a neurological condition. Thus, in accordance with the present disclosure, a protein or peptide therapeutic agent may be useful for the selective targeting of a target protein or structure associated with a neurological condition (e.g., for diagnosis or therapy) (see, e.g., U.S. Patent Application Publication 2009/0238754). Protein or peptide therapeutic agents that specifically bind to a target protein or structure associated with neurological conditions, include, but are not limited to, Aβ-peptide in amyloid plaques of Alzheimer's disease (AD), cerebral amyloid angiopathy (CAA), and cerebral vascular disease (CVD); α-synuclein deposits in Lewy bodies of Parkinson's disease, tau in neurofibrillary tangles in frontal temporal dementia and Pick's disease; superoxide dismutase in amylotrophic lateral sclerosis; and Huntingtin in Huntington's disease and benign and cancerous brain tumors such as glioblastoma's, pituitary tumors, or meningiomas.

In other embodiments, the macromolecular therapeutic agent may be an antibody, such as a monoclonal or polyclonal antibody (as described above). The antibody may specifically bind to a target protein or structure associated with a neurological condition, such as a target protein or structure (such as a specific conformation or state of self-aggregation) associated with an amyloidogenic disease. Such antibodies include, for example, the anti-amyloid antibodies 6E10 and NG8 (see, e.g., Hunter S., Brayne C., J Negat Results Biomed., 16(1): 1 (2017)). Other anti-amyloid antibodies are known in the art, as are antibodies that specifically bind to proteins or structures associated with other neurological conditions, any of which may be used in the methods disclosed herein.

In certain embodiments, the macromolecular therapeutic agent is a monoclonal antibody. Suitable monoclonal antibodies include, but are not limited to, 6E10, PF-04360365, 131I-chTNT-1/B MAb, 131I-L19SIP, 177Lu-J591, ABT-874, AIN457, alemtuzumab, anti-PDGFR alpha monoclonal antibody IMC-3G3, astatine At 211 monoclonal antibody 81C6, bapineuzumab, bevacizumab, cetuximab, cixutumumab, daclizumab, Hu MiK-beta-1, HuMax-EGFr, iodine I 131 monoclonal antibody 3F8, iodine 1131 monoclonal antibody 81C6, iodine I 131 monoclonal antibody 8H9, iodine I 131 monoclonal antibody TNT-1/B, LMB-7 immunotoxin, MAb-425, MGAWN1, Me1-14 F(ab')2, M-T412, natalizumab, neuradiab, nimotuzumab, ofatumumab, panitumumab, ramucirumab, ranibizumab, SDZ MSL-109, solanezumab, trastuzumab, ustekinumab, zalutumumab, tanezumab, aflibercept, MEDI-578, REGN475, muromonab-CD3, abiximab, rituximab, basiliximab, palivizumab, infliximab, gemtuzumab ozogamicin, ibritumomab tiuxetan, adalimumab, omalizumab, tositumomab, tositumomab- 1131, efalizumab, abciximab, certolizumab pegol, eculizumab, AMG-162, zanolimumab, MDX-010, anti0MRSA mAb, pexelizumab, mepolizumab, epratuzumab, anti-RSV mAb, afelimomab, catumaxomab, WX-G250, or combinations thereof. Therapeutic antibodies for the treatment of brain disorders are further described in, e.g., Freskgård P.O., Urich E., Neuropharmacology, 120: 38-55 (2017); and Yu Y.J., and Watts RJ., Neurotherapeutics, 10(3): 459-72 (2013).

In other embodiments, the macromolecular therapeutic agent may be a neurotrophic protein. Suitable neurotrophic proteins include, but are not limited to, nerve growth factor (NGF), brain-derived neurotrophic factor (BDNF), neurotrophin-3 (NT-3), neurotrophin-4 (NT-4), neurotrophin-5 (NT-5), insulin-like growth factors (IGF-I and IGF-II), glial cell line derived neurotrophic factor (GDNF), fibroblast growth factor (FGF), ciliary neurotrophic factor (CNTF), epidermal growth factor (EGF), glia-derived nexin (GDN), transforming growth factor (TGF-α and TGF-β), interleukin, platelet-derived growth factor (PDGF), and S100β protein, and analogs and derivatives thereof.

In other embodiments, the macromolecular therapeutic agent may be a protein associated with membranes of synaptic vesicles, such as calcium-binding proteins and other synaptic vesicle proteins. Calcium-binding proteins include, for example, cytoskeleton-associated proteins such as caldesmon, annexins, calelectrin (mammalian), calelectrin (torpedo), calpactin I, calpactin complex, calpactin II, endonexin I, endonexin II, protein II, synexin I, and enzyme modulators, such as p65. Other synaptic vesicle proteins include inhibitors of mobilization (such as synapsin Ia,b and synapsin IIa,b), synaptophysin, and proteins of unknown function such as p29, VAMP-1,2 (synaptobrevin), VAT1, rab 3A, and rab 3B.

Macromolecular therapeutic agents also include α-, β- and γ-interferon, epoetin, fligrastim, sargramostin, CSF-GM, human-IL, TNF, and other biotechnology drugs. The macromolecular therapeutic agent may also be a peptide, protein, or antibody obtained using recombinant biotechnology methods.

In other embodiments, the therapeutic agent may be a small molecule drug. Any suitable small molecule drug may be administered to the subject. Ideally, the small molecule drug is an agent that is capable of treating a disease or disorder of the central nervous system. Suitable small molecule drugs for treating a disease, disorder, or condition of the CNS include, but are not limited to, acetaminophen, acetylsalicylic acid, acyltransferase, alprazolam, amantadine, amisulpride, amitriptyline, amphetamine-dextroamphetamine, amsacrine, antipsychotics, antivirals, apomorphine, arimoclomol, aripiprazole, asenapine, aspartoacyclase enzyme, atomoxetine, atypical antipsychotics, azathioprine, baclofen, beclamide, benserazide, benserazide-levodopa, benzodiazepines, benztropine, bleomycin, brivaracetam, bromocriptine, buprenorphine, bupropion, cabergoline, carbamazepine, carbatrol, carbidopa, carbidopalevodopa, carboplatin, chlorambucil, chlorpromazine, chlorprothixene, cisplatin, citalopram, clobazam, clomipramine, clonazepam, clozapine, codeine, COX-2 inhibitors, cyclophosphamide, dactinomycin, dexmethylphenidate, dextroamphetaine, diamorphine, diastat, diazepam, diclofenac, donepezil, doxorubicin, droperidol, entacapone, epirubicin, escitalopram, ethosuximide, etoposide, felbamate, fluoxetine, flupenthixol, fluphenazine, fosphenytoin, gabapentin, galantamine, gamma hydroxybutyrate, gefitinib, haloperidol, hydantoins, hydrocordone, hydroxyzine, ibuprofen, ifosfamide, IGF-1, iloperidone, imatinib, imipramine, interferons, irinotecan, KNS-760704, lacosamide, lamotrigine, levetiracetam, levodopa, levomepromazine, lisdexamfetamine, lisuride, lithium carbonate, lypolytic enzyme, mechlorethamine, mGluR2 agonists, memantine, meperidine, mercaptopurine, mesoridazine, mesuximide, methamphetamine, methylphenidate, minocycline, modafinil, morphine, N-acetylcysteine, naproxen, nelfinavir, neurotrin, nitrazepam, NSAIDs, olanzapine, opiates, oseltamivir, oxaplatin, paliperidone, pantothenate kinase 2, Parkin, paroxetine, pergolide, periciazine, perphenazine, phenacemide, phenelzine, phenobarbitol, phenturide, phenyloin, pimozide, Pink1, piribedil, podophyllotoxin, pramipexole, pregabalin, primidone, prochlorperazine, promazine, promethazine, protriptyline, pyrimidinediones, quetiapine, rasagiline, remacemide, riluzole, risperidone, ritonavir, rivastigmine, ropinirole, rotigotine, rufinamide, selective serotonin reuptake inhibitors (SSRIs), selegine, selegiline, sertindole, sertraline, sodium valproate, stiripentol, taxanes, temazepam, temozolomide, tenofovir, tetrabenazine, thiamine, thioridazine, thiothixene, tiagabine, tolcapone, topiramate, topotecan, tramadol, tranylcypromine, , tricyclic antidepressants, trifluoperazine, triflupromazine, trihexyphenidyl, trileptal, valaciclovir, valnoctamide, valproamide, valproic acid, venlafaxine, vesicular stomatitis virus, vigabatrin, vinca alkaloids, zanamivir, ziprasidone, zonisamide, zotepine, zuclopenthixol, and analogs or derivatives of any of the foregoing.

Other therapeutic agents or compounds that may be administered according to the present invention may be of any class of drug or pharmaceutical agent for which crossing the BBB is desired. Such therapeutics include, but are not limited to, antibiotics, anti-parasitic agents, antifungal agents, anti-viral agents, and anti-tumor agents.

A CNS disease, disorder, or condition is any disease, disorder, or condition that affects the brain and/or spinal cord (collectively known as the central nervous system (CNS)). CNS disease, disorder, or conditions that may be treated in accordance with the inventive methods include, but are not limited to, addiction, arachnoid cysts, autism, catalepsy, encephalitis, locked-in syndrome, meningitis, multiple sclerosis (MS), myelopathy, metabolic disease, a behavioral disorder, a personality disorder, dementia, a cancer, a neurodegenerative disorder (e.g., Alzheimer's disease, Huntington's disease, and Parkinson's disease), stroke, pain, a viral infection, a sleep disorder, epilepsy/seizure disorders, acid lipase disease, Fabry disease, Wernicke-Korsakoff syndrome, attention deficit/hyperactivity disorder (ADHD), anxiety disorder, borderline personality disorder, bipolar disorder, depression, eating disorder, obsessive-compulsive disorder, schizophrenia, Barth syndrome, Tourette's syndrome, Canavan disease, Hallervorden-Spatz disease, Lewy Body disease, Lou Gehrig's disease, Machado-Joseph disease, restless Leg syndrome, traumatic brain injury (TBI), and autoimmune disease.

The therapeutic agent and the agent that inhibits the activity of ALPL may be formulated together as a single composition. Alternatively, the therapeutic agent and the agent that inhibits the activity of ALPL may be formulated as separate compositions which may be administered simultaneously or sequentially as described herein. In either case, the composition desirably is a pharmaceutically acceptable (e.g., physiologically acceptable) composition, which comprises a carrier, preferably a pharmaceutically acceptable (e.g., physiologically acceptable) carrier, and the therapeutic agent and/or the ALPL inhibitor. Any suitable carrier can be used within the context of the disclosure, and such carriers are well known in the art. The carrier typically will be liquid, but also can be solid, or a combination of liquid and solid components. The choice of carrier will be determined, at least in part, by the location of the target tissue and/or cells, and the particular method used to administer the composition.

The composition can further comprise any other suitable components, especially for enhancing the stability of the composition and/or its end use. Accordingly, there is a wide variety of suitable formulations of the composition of the invention. The following formulations and methods are merely exemplary and are in no way limiting.

Formulations suitable for parenteral administration include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit dose or multi dose sealed containers, such as ampules and vials, and can be stored in a freeze dried (lyophilized) condition requiring only the addition of a sterile liquid excipient, for example, water, for injections, immediately prior to use. Any suitable carrier can be used within the context of the disclosure, and such carriers are well known in the art. For example, the composition may contain preservatives, such as, for example, methylparaben, propylparaben, sodium benzoate, and benzalkonium chloride. A mixture of two or more preservatives optionally may be used. In addition, buffering agents may be included in the composition. Suitable buffering agents include, for example, glutamic acid (glutamate), citric acid, sodium citrate, phosphoric acid, potassium phosphate, and various other acids and salts. A mixture of two or more buffering agents optionally may be used.

Extemporaneous injection solutions and suspensions can be prepared from sterile powders, granules, and tablets. The composition may be formulated for oral administration. Suitable oral formulations include, for example, tablets, pills, dragees, capsules, liquids, gels, syrups, slurries, suspensions, etc. The composition may be formulated for parenteral administration, e.g., intravenous injection, intraperitoneal injection, intramuscular injection, subcutaneous injection, etc. The compounds or agents of the present invention may also be administered directly to the airways in the form of an aerosol. For use as aerosols, the compounds of the present invention in solution or suspension may be packaged in a pressurized aerosol container together with suitable propellants, for example, hydrocarbon propellants like propane, butane, or isobutane with conventional adjuvants. The composition also may be administered in a non-pressurized form such as in a nebulizer or atomizer. Methods for preparing compositions for pharmaceutical use are known to those skilled in the art and are described in, for example, Remington: The Science and Practice of Pharmacy, Lippincott Williams & Wilkins; 21st ed. (May 1, 2005).

The disclosure also provides a method for identifying a protein or other biomolecule that regulates blood-brain barrier permeability in a mammal (e.g., a mouse, rat, primate, or human). In some embodiments, the method comprises: (a) detectably labeling the proteome or other expressed biomolecules (RNAs) of blood plasma isolated from a first mammal; (b) introducing the isolated blood plasma comprising the labeled proteome or other expressed biomolecules into a second mammal; (c) isolating brain endothelial cells from the second mammal that form the blood-brain barrier; (d) measuring plasma uptake in each of the isolated brain endothelial cells using flow cytometry to produce a population of sorted brain endothelial cells; (e) detecting expression of genes that correlate with plasma uptake in each of the sorted brain endothelial cells; and (f) selecting a protein or other biomolecule encoded by a gene whose expression correlates with increased or decreased plasma uptake in a brain endothelial cell, whereby the protein or other biomolecule regulates blood-brain barrier permeability in the subject. In some embodiments, such identified molecules are utilized for research or clinical purposes to regulate blood-brain barrier permeability. For example, the expression or activity of such molecules is manipulated, as described above for ALPL, to effectuate the desired result.

Blood collection and plasma separation/isolation from whole blood may be accomplished using any desired method. It will be appreciated that serum is the liquid fraction of whole blood that is collected after the blood is allowed to clot. The clot is removed by centrifugation and the resulting supernatant, designated serum, is carefully removed using a pipette. Plasma is produced when whole blood is collected in tubes that are treated with an anticoagulant. The blood does not clot in the plasma tube. The cells are removed by centrifugation. The supernatant, designated plasma, is carefully removed from the cell pellet using a pipette. Systems and methods for blood collection and plasma separation are commercially available from a variety of sources, any of which may be used in the methods described herein.

Methods for detectably labeling the proteome or other biomolecules of blood plasma isolated from a mammal are known in the art and can be used in connection with the methods described herein. For example, chemical labeling with isobaric tandem mass tags, such as isobaric tags for relative and absolute quantification reagents (iTRAQ) and tandem mass tag (TMT) reagents, has been employed in a wide range of different clinically orientated serum and plasma proteomics studies (see, e.g., Moulder et al., Mass Spectrometry Reviews, 37(5): 583-606 (2018)). Fluorescent labeling methods also may be used, such as those described in, e.g., Liu et al., Proteomics, 12(14): 2258-70 (2012); Leclerc et al., Bioconjugate Chem., 29(8): 2541-2549 (2018); Volke, D. and R. Hoffmann, Electrophoresis, 29(22): 4516-4526 (2008); and Obermaier et al., Methods Mol Biol., 1295: 153-65 (2015), herein incorporated by reference in their entireties. In certain embodiments, the plasma proteome is detectably labeled using fluorescent labeling methods.

Once the proteome of the plasma isolated from the first mammal has been detectably labeled, the isolated blood plasma comprising the labeled proteome is then introduced into a second mammal to allow for visualization and measurement of BBB permeability. The second mammal and first mammal desirably are the same type of mammal (e.g., both mice, rats, or non-human primates). The isolated blood plasma comprising the labeled proteome is introduced into the second mammal under conditions that allow for uptake of the plasma comprising the labeled proteome into endothelial cells of the BBB of the second mammal. Following a period of time sufficient for uptake of the plasma comprising the labeled proteome by the BBB of the second mammal, brain endothelial cells that form the blood-brain barrier may be isolated from the second mammal using any suitable method known in the art. Brain endothelial cell isolation protocols are described in, e.g., Assmann et al., Bio Protoc., 7(10): e2294 (2017); Welser-Alves et al., Methods Mol Biol., 1135: 345-56 (2014); Luo et al., Methods Mol Biol., 1135: 357-64 (2014); and Navone et al., Nature Protocols, 8: 1680-1693 (2013), herein incorporated by reference in their entireties.

Plasma uptake by brain endothelial cells of the BBB may be measured using *in vitro* or *in vivo* methods that are used in the art for measuring drug transport across the BBB. *In vivo* methods include, for example, intravenous injection/brain sampling, brain perfusion, quantitative autoradiography, microdialysis, and CSF sampling. *In vitro* methods include, for example, analysis of fresh isolated brain microvessels and endothelial cell culture. Methods for measuring drug and protein transport across the BBB are described in further detail in, e.g., Bickel, U., NeuroRx, 2(1): 15-26 (2005); and Feng, M.R., Curr Drug Metab., 3(6):i 647-57 (2002). In some embodiments, plasma uptake in each of the isolated brain endothelial cells may be measured using flow cytometry to produce a population of sorted brain endothelial cells. Flow cytometry is a technology that rapidly analyzes single cells or particles as they flow past single or multiple lasers while suspended in a buffered salt-based solution. Each particle is analyzed for visible light scatter and one or multiple fluorescence parameters (e.g., from 1 up to 30 or more parameters). Visible light scatter is measured in two different directions: (1) the forward direction (Forward Scatter or FSC) which can indicate the relative size of the cell and (2) at 90° (Side Scatter or SSC) which indicates the internal complexity or granularity of the cell. Light scatter is independent of fluorescence. Samples are prepared for fluorescence measurement through transfection and expression of fluorescent proteins (e.g., green fluorescent protein, GFP), staining with fluorescent dyes (e.g., propidium iodide, DNA) or staining with fluorescently conjugated antibodies (e.g., CD3 FITC) (see, e.g., McKinnon, K.M., Curr Protoc Immunol., 120: 5.1.1-5.1.11 (2018)). Flow cytometry methods for analysis of the blood-brain barrier in particular are described in, e.g., Williams et al., Cytometry A, 87(10): 897-907 (2015), herein incorporated by reference in its entirety.

Performing flow cytometry on the isolated brain endothelial cells results in a population of flow cytometry-sorted (also referred to as "sorted") brain endothelial cells, which are then analyzed to detect expression of genes that correlate with plasma uptake in each of the sorted brain endothelial cells. In this regard, brain endothelial cells may be sorted, and fluorescence recorded for each sorted cell. mRNA expression in each sorted cell may then be detected and quantified using any suitable method known in the art for measuring gene expression. Such methods include, but are not limited to, quantitative or real-time RT-PCR (qRT-PCR), microarray analysis, RNA sequencing, *in situ* hybridization, or Northern blot. In certain embodiments, RNA sequencing (also referred to as "RNA-Seq") is used to detect expression of genes that correlate with plasma uptake. RNA-Seq uses next-generation sequencing (NGS) to detect and quantify RNA in a biological sample at a particular moment, allowing for the analysis of the continuously changing cellular transcriptome (Chu, Y. and Corey D.R., Nucleic Acid Therapeutics, 22 (4): 271-274 (2012); and Wang et al., Nature Reviews Genetics, 10(1): 57-63 (2009)). RNA-Seq facilitates the ability to examine alternative gene spliced transcripts, post-transcriptional modifications, gene fusion, mutations/single nucleotide polymorphisms (SNPs) and changes in gene expression over time (Maher et al., Nature, 458(7234): 97-101 (2009)). In addition to mRNA transcripts, RNA-Seq can be used to analyze different populations of RNA such as small RNA, miRNA, tRNA, and ribosomes. RNA-Seq methods and techniques are described in, e.g., Maekawa et al., Methods Mol Biol., 1164: 51-65 (2014), and techniques for single-cell RNA-Seq are described in, e.g., Chen et al., Front. Genet., 10: 317 (2019).

The expression of a specific gene may be correlated with increased or decreased plasma uptake of proteins or other biomolecules in a particular brain endothelial cell. In this regard, upregulation or downregulation of expression of a specific gene may directly or indirectly lead to increased plasma uptake of proteins (i.e., increased BBB permeability) or to decreased plasma uptake of proteins (i.e., decreased BBB permeability). Expression of a gene is downregulated if the expression is reduced by at least about 20% (e.g., 25%, 30%, 35%, 40%, 45%, 50%, 55%, 60%, 65%, 70%, 75%, 80%, 85%, 90%, 95%, 99% or more) as compared to a reference or control level. Expression of a gene is upregulated if the expression is increased by at least about 20% (e.g., 25%, 30%, 35%, 40%, 45%, 50%, 55%, 60%, 65%, 70%, 75%, 80%, 85%, 90%, 95%, 99% or more) as compared to a reference or control level. A protein or other biomolecule encoded by a gene whose upregulation or downregulation correlates with increased or decreased plasma uptake may be selected as a regulator of blood-brain permeability.

The following examples further illustrate the invention but, of course, should not be construed as in any way limiting its scope.

### EXAMPLES

The following materials and methods were used in the experiments described in the Examples.

### Animals

Aged C57BL/6 mice (20-24 months old) were obtained from the National Institute on Aging rodent colony. Young male C57BL/6 mice (3 months old) were obtained from Jackson Laboratories or Charles River Laboratories. All experiments used male mice. All mice were kept on a 12-h light/dark cycle and provided ad libitum access to food and water. All animal care and procedures complied with the Animal Welfare Act and were in accordance with institutional guidelines and approved by the V.A. Palo Alto Committee on Animal Research and the institutional administrative panel of laboratory animal care at Stanford University.

### Plasma Collection and Labeling

Blood was collected with 250 mM EDTA (Millipore Sigma) as anticoagulant by terminal intracardial bleeding. EDTA-plasma was isolated by centrifugation at 1,000g for 15 min at 4 °C before pooling, aliquoting, flash freezing in liquid nitrogen, and storage at -80 °C. Hemolyzed plasma was discarded. Before labeling, frozen plasma was thawed on ice, gently mixed, and inspected for precipitates. Plasma protein molarity was approximated to be 750 µM. Labeling relied on amine-reactive NHS ester moieties, and labeling ratios and times were determined empirically for each specific label: for radiotracing, NHS-DOTA (1,4,7,10-tetraazacyclododecane-1,4,7,10-tetraacetic acid, Macrocyclics) was added at a 10x molar ratio; for fluorescence, NHS-Atto 647N (Millipore Sigma, ATTO-TEC) added at 1.4x molar ratio; and for characterizing the labeled plasma proteome, NHS-biotin (Thermo Fisher) and NHS-trans-Cyclooctene (Click Chemistry Tools) added at 17.5x molar ratio. NHS-Atto 647N was incubated with plasma for 1.5 h at room temperature before PBS dialysis overnight. The next day, 50 mM Tris pH 8.0 was added for 10 minutes at room temperature, and the labeled plasma washed extensively three times with Amicon Ultra-15 Centrifugal Filter Unit, 3 KDa cutoff (Millipore Sigma, 10 KDa for NHS-Atto 647N), and subsequently washed four times with Zeba Spin Desalting Columns, 7K MWCO cutoff (Thermo Fisher) in chilled PBS. Other conjugations proceeded overnight at 4°C before washing. These steps expanded on washing that consistently yielded >99% radiochemical purity of 64Cu-labelled DOTA (below) to minimize contamination from residual free label. Throughout labeling and washing steps, plasma samples were monitored for signs of aggregation and flocculation. Plasma concentrations were measured using the Nanodrop spectrophotometer (Thermo Fisher), and approximately 10-15 mg (0.5 mg/g body weight, in line with prior studies using horseradish peroxidase5,6,126) was administered intravenously (retro-orbital) per mouse in a volume less than or equal to 150 µL.

### Labeled Plasma Characterization by Mass Spectrometry and Protein Microarray

For characterization by mass spectrometry, labeled plasma was depleted of albumin and IgG using the ProteoPrep Immunoaffinity Albumin & IgG Depletion Kit (Millipore Sigma), enriched with tetrazine agarose overnight at 4°C (Click Chemistry Tools), and extensively washed, as previously described127. In parallel, unlabeled plasma was processed using paramagnetic beads, termed Single-Pot Solid-Phase-enhanced Sample Preparation (SP3), as previously described128. Plasma was fractionated using the Pierce High pH Reversed-Phase Peptide Fractionation Kit (Thermo Fisher) and concatenated fractions cleaned using C18-based STAGE Tips and lyophilized. Peptides were analyzed on an LTQ Orbitrap Fusion Lumos Tribrid Mass Spectrometer (Thermo Fisher Scientific). Peptides were separated by capillary reverse-phase chromatography for 180 min on a 24 cm reversed-phase column (100 µm inner diameter, packed in-house with ReproSil-Pur C18-AQ 3.0 m resin (Dr. Maisch)). A four-step linear gradient was achieved using a Dionex Ultimate 3000 LC-system (Thermo Fisher Scientific) as follows: 97% A + 3% B for 15 min, 75% A +25% B for 135 min, 55% A + 45% B for 15 min, and 5% A + 95% B for 15 min, where buffer A is 0.1 % formic acid in water and buffer B is 0.1% formic acid in acetonitrile. Full MS scans were acquired in the Orbitrap mass analyzer at a resolution of 120,000 (FWHM) and m/z scan ranges 340-1540 in a data-dependent mode. The AGC targets were 4*105 and the maximum injection time for FTMS1 were 50 ms. The most intense ions were then selected for sequencing and fragmented in the Orbitrap mass analyzer using higher-energy collisional dissociation (HCD) with a normalized collision energy of 30% and resolution of 15,000 (FWHM). Monoisotopic precursor selection was enabled and singly charged ion species and ions with no unassigned charge states were excluded from MS2 analysis. Dynamic exclusion was enabled with a repeat count of 2 and ions within ±10 ppm m/z window around ions selected for MS2 were excluded from further selection for fragmentation for 30 sec. AGC target were 5*104 and maximum injection time of 200 ms. The raw data files were processed and analyzed using MaxQuant and Perseus (Max Planck)129,130. In brief, spectra were matched to a Mus musculus database downloaded from uniprot.org, and a contaminant and decoy database. Precursor mass tolerance was set to 4.5 p.p.m., fragment ion tolerance to 10 p.p.m., with fixed modification of Cys residues (carboxyamidomethylation +57.021 Da) and variable modifications of Met residues (Ox +15.995 Da), Lys residues (acetylation +42.011 Da), Asn and Gln residues (deamidation +0.984 Da) and of N termini (carbamylation +43.006 Da). Peptide identifications were calculated with FDR < 0.01, and for protein quantification, minimum ratio count was set to one, with both unique and razor peptides used for quantification.

For characterization by protein microarrays, ELISA-grade antibodies against several hundred secreted and potentially cleaved transmembrane mouse or human signaling proteins were obtained from commercial sources and printed in five replicates onto SuperEpoxy2 glass slides (Arrayit) with a robotic microarrayer (NanoPrint LM210, Arrayit), as previously described13. Arrays were then blocked with 3% (w/v) casein solution before incubation with biotinylated plasma samples overnight at 4 °C. Following washes, arrays were incubated with Alexa Fluor 647-conjugated streptavidin secondary antibodies (Thermo Fisher) and fluorescent signal detected using a GenePix4400A scanner and GenePix Pro7 software (Molecular Devices). Data processing and analysis followed previously described methods13.

### Brain Perfusion

Mice were euthanized with 2.5% (v/v) Avertin and transcardially perfused with at least 50 ml of chilled PBS. Perfusion was performed using a peristaltic pump, with the flow rate not exceeding 10 ml/min to approximate the physiological pressure of the mouse's circulatory system 131-13 3. Mice with perfusate leaking out of the nostrils were not processed further for analysis. For immunohistochemistry (below), mice were subsequently perfused with 4% paraformaldehyde except when hemibrains were taken for flow cytometry (below).

### Immunohistochemistry

Brain tissue processing, immunohistochemistry, and immunofluorescence experiments were performed as described previously12,13. Hemibrains were isolated and post-fixed in 4% (w/v) paraformaldehyde overnight at 4°C before preservation in 30% (w/v) sucrose in PBS. Hemibrains were sectioned coronally or sagittally at a thickness of 50 µm on a freezing-sliding microtome, and sections were stored in cryoprotective medium at -20 °C. Free-floating sections were blocked with appropriate serum before incubation at 4 °C with primary antibodies at the following concentrations for confocal microscopy: goat monoclonal anti-CD31 (1:100, AF3628, R&D), Fluorescein-labeled Lectin (1:200, Vector Laboratories), rabbit monoclonal anti-Aquaporin 4 (1:500, AB2218, Millipore Sigma), rat anti-CD13 (1:100, MCA2183EL, Bio-Rad), goat anti-Alpl/ ALPL (1:100, AF2909, R&D), mouse anti-NeuN (1:400, MAB377, Millipore), goat anti-albumin (1:100, NB600, Novus), rabbit anti-Collagen I (1:100, ab21286, Abcam), and goat anti-Iba1 (1:500, ab5076, Abcam). Sections were washed, stained with Alexa Fluor-conjugated secondary antibodies (1:250), mounted and coverslipped with ProLong Gold (Life Technologies) before imaging on a confocal laser-scanning microscope (Zeiss LSM880). Age-related autofluorescence was quenched with 1 mM CuSO4 in 50 mM ammonium acetate buffer (pH 5), as previously described73. National Institutes of Health ImageJ software was used to quantify the percentage of vasculature (CD31 or AQP4) covered by CD13, AQP4, or ALPL, as described previously63. All analyses were performed by a blinded observer. Alizarin red staining was performed as described previously83, with minor adaptations: sections were incubated for 1 h in 40 mM alizarin red in PBS (pH 7.4) at room temperature, and extensively washed overnight with PBS prior to mounting. Images of brain sections were acquired by conventional light microscopy to detect calcified nodules. Sections with biotinylated plasma were blocked overnight in 6% BSA at room temperature, detected with streptavidin-Alexa Fluor 647 (1:1500, Thermo Fisher) for 2 hours, and washed overnight before mounting. Sections containing L-azidohomoalanine-labeled plasma were blocked overnight in 6% BSA at room temperature, incubated in 45 mM iodoacetamide (Millipore Sigma) in 100% methanol for 1 hour, washed, detected with 1.2 µM sDIBO (Thermo Fisher Scientific) in 100% methanol, and washed overnight before mounting. Wole brain coronal and sagittal sections were processed at 100 µm, incubated in Focusclear (CellExplorer Labs), and imaged in tiles. Vascular ALPL activity was measured using the Red Alkaline Phosphatase Substrate Kit (SK-5100, Vector Laboratories) with 20-minute incubation.

### Gamma Counting, Autoradiography, and PET Scanning

Conjugation of rat IgG2a (400501, BioLegend) and albumin/IgG plasma with 1,4,7,10-tetraazacyclododecane-1,4,7,10-tetraacetic acid (DOTA) was performed using metal-free buffers, as previously described13,134,135. Conjugations proceeded overnight at 4°C with a 10x molar ratio of DOTA-NHS ester in HEPES buffer (0.1 mol l-1, pH 8) and quenched with 50 mM Tris-HCl (Millipore Sigma). Excess DOTA-NHS was removed by Zeba Spin Desalting Columns (0.5 ml, 7K molecular weight cut-off, Thermo Fisher), and the resulting solution was buffer-exchanged into ammonium acetate buffer (0.1 M, pH 5.5) for Cu-64 labelling. DOTA-conjugate solutions were concentrated by ultrafiltration (Amicon 0.5 ml, Millipore Sigma) and kept on ice. Radiolabeling with 64Cu (half-life = 12.7 h) was performed as previously described13. In brief, DOTA-IgG2a or DOTA-plasma (in 25 µg aliquots in 50 µl) was mixed with pH-balanced [64Cu]Cl2 solution (pH 4.5-5.5, University of Wisconsin, Madison) at 37°C with gentle shaking at 400 r.p.m. After incubation (30 min for IgG2a-DOTA and 60 min for plasma-DOTA), 0.1 M EDTA (0.5 M, pH 8.0) was added to a final concentration of 0.01 M and incubated at ambient temperature for 15 min to scavenge unchelated [64Cu]Cl2. Purification was achieved by G25 Sephadex size-exclusion purification (GE Life Sciences). Radiochemical purity was determined by instant thin-layer chromatography with TEC-Control Chromatography strips (Biodex Medical Systems), developed in saline, and size exclusion liquid chromatography with a SEC 3000 column (Phenomenex).

Mice were injected intravenously with 7.7 ± 1.5 MBq of 64Cu-labelled DOTA-IgG2a or 64Cu-labelled DOTA-plasma (radiochemical purity >99%). After 20 h, mice were placed in a dual microPET/ CT scanner (Inveon, Siemens) to capture static images (10 min) for subsequent analysis with VivoQuant software (version 4.0, inviCRO), as previously described136,137. After anaesthetization, blood samples (100-200 µl) were collected by cardiac puncture immediately prior to transcardial perfusion. Tissue-related radioactivity (dose and decay-corrected to time of injection) in blood and brain was assessed following the literature13 procedure with an automated gamma counter (Hidex Oy, calibrated using aliquots of the initial administered dose as standards). Activity in cardiac blood samples at time of sacrifice was used to correct for differential in vivo stability and clearance of plasma vs. IgG. Brain tissue was embedded in optimal-cutting temperature compound (Tissue-Tek), and coronal sections (40 µm) were obtained for ex vivo autoradiography. Autoradiography was conducted using previously described methods13,138: 40 µm sections were mounted, air-dried for 10 min, and then exposed to a digital storage phosphor screen (Amersham Biosciences) for 72 hours at -20 °C. The image plate was analyzed using a Typhoon 9410 Variable Mode Imager (Perkin Elmer). Slides were then Nissl stained and scanned using a Nanozoomer 2.0-RS (Hamamatsu) to enable anatomical co-localization. Images were visualized, processed, and quantified blinded with ImageJ. For quantification, at least 10 consistently-sized hippocampal and cortical areas were drawn for each mouse. Mean pixel intensity was dose and decay corrected for each mouse.

### Primary CNS Cell Isolation for Flow Cytometry Analysis

CNS cell isolation adopted previously described methods42,139,140. Briefly, cortices and hippocampi were microdissected, minced, and digested using the Neural Dissociation Kit (Miltenyi). Suspensions were filtered through a 100 µm strainer and myelin removed by centrifugation in 0.9 M sucrose. The remaining myelin-depleted cell suspension was blocked for ten minutes with Fc preblock (CD16/CD32, BD 553141) on ice and stained for 30 minutes with antibodies to distinguish brain endothelial cells (CD31+/ CD45-), astrocytes (ACSA-2+), and neurons (NeuN+ or CD90.2/Thy1.2+). For analysis of plasma uptake, at least 1,000 cells of each population of interest were analyzed per sample. Antibody dilutions: rat anti-CD31-PE/CF594 (1:100, clone MEC 13.3, BD, cat. No. 563616), rat anti-CD45-PE/Cy7 (1:200, clone 30-F11, Biolegend, cat. no. 103114), rat anti ACSA-2-PE (1:200, clone IH3-18A3, Miltenyi, cat. no. 130-102-365), mouse anti-NeuN-PE (1:100, clone A60, Millipore Sigma, cat. no. FCMAB317PE), and rat anti-CD90.2-FITC (1:100, clone 30-H12, Biolegend, cat. no. 105305).

### Flow Index Sorting and Single-Cell RNA-Sequencing

Cell lysis, first-strand synthesis and cDNA synthesis was performed using the Smart-seq-2 protocol as described previously139-141 in 384-well format, with some modifications. Briefly, brain endothelial cells (CD31+ / CD45-) were sorted using SH800S (Sony) sorters on the highest purity setting ('Single cell'). Plasma-647 fluorescence was recorded for each cell and corresponding sorted well. cDNA synthesis was performed using the Smart-seq2 protocol140-142. After cDNA amplification (23 cycles), concentrations were determined via the PicoGreen quantitation assay. Cells passing quality control were selected through custom scripts and cDNA concentrations normalized to ~0.2 ng/µL using the TPPLabtech Mosquito HTS and Mantis (Formulatrix) robotic platforms. Libraries were prepared and pooled using the Nextera XT kits (Illumina), following the manufacturer's instructions. Libraries were then sequenced on the Nextseq or Novaseq (Illumina) using 2 x 75bp paired-end reads and 2 x 8bp index reads with a 200-cycle kit (Illumina, 20012861). Samples were sequenced at an average of 1.5M reads per cell. Raw sequencing files were demultiplexed with bcl2fastq, reads were aligned using STAR, and gene counts made using HTSEQ version 0.6.1p1. Downstream analysis was performed as previously described58, and plasma fluorescence of each cell correlated (Spearman) with gene expression and zonation59.

### ALPL Inhibitor Treatment

Mice were treated with six doses of ALPL inhibitor (613810, Millipore Sigma; 2,5-Dimethoxy-N-(quinolin-3-yl)benzenesulfonamide, Tissue-Nonspecific Alkaline Phosphatase Inhibitor, MLS-0038949; C₁₇H₁₆N₂O₄S; CAS # 496014-13-2) or 'Vehicle' control over three days, injected intraperitoneally twice per day (8.75 mg/kg). 'Vehicle' treatments consisted of phosphate-buffered saline (PBS) with a matching concentration of DMSO (less than 4% v/v). After the sixth treatment, mice were injected intravenously with 150 µL of Atto 647N-labeled plasma (as above), human holo-transferrin (T4132, Millipore Sigma, 40 mg/kg), transferrin receptor antibody (BE0175, BioXcell, 20 mg/kg), or 3-kDa dextran-FITC (10 mg/kg, Thermo Fisher). Plasma, human holo-transferrin, and transferrin receptor antibody were infused 20 h before sacrifice, while 3-kDa dextran-FITC was given 2 h before sacrifice, as previously described143. After extensive perfusion, CNS cells were isolated as described above. Cell pellets were additionally incubated in Red Blood Cell Lysis Buffer (Millipore Sigma, R7757) for 2 minutes to ensure high CNS cell purity. Brain endothelial cells were distinguished by CD31+ /CD45- staining and parenchymal cells by CD31- / CD45- staining.

### Western Blot

For brain microvessel western blotting of TFRC and CAV1, cortical and hippocampal microvessels were isolated as previously described51,55,144. Protein lysates were prepared by incubating cell pellets on ice in 1% SDS in 50 mM HEPES with complete protease inhibitor cocktail (Thermo Fisher Scientific) and spun at 13,000 x g for 10 minutes. The supernatant was collected, and protein concentration measured with the Pierce BCA Protein Assay Kit (Thermo Fisher Scientific). An aliquot containing 10-20 ug of protein from each sample was mixed with 4X loading buffer (Thermo Fisher Scientific) and boiled for 5 minutes at 95°C before being subjected to SDS-PAGE and transferred to a nitrocellulose membrane (Bio-Rad). The membrane was first blocked with 5% milk and stained overnight at 4°C with primary antibodies at the designated concentrations: rabbit anti-Caveolin-1 (1: 1000, D46G3, CST), rabbit anti-Transferrin Receptor (1:1000, ab214039, Abcam), and rabbit anti Histone H3 (1:4000, ab1791, Abcam). The membrane was washed, stained with IRDye conjugated secondary antibodies (1:15,000, LI-COR) and imaged on the Odyssey CLx (LI-COR). Images were analyzed for band intensities with the ImageStudio software (LI-COR).

### Brain Endothelial Cell Lipidomics, Mass Spectrometry

Brain microvessels were isolated from young (3 m.o.) and old (20 m.o.) mice as previously described51,55,144-148, after overnight fasting (~12h). Sixteen mice of each age were used, with microvessels from 4 mice pooled in each group (n = 4 groups). Lipids were extracted in a randomized order via biphasic separation with cold methyl tert-butyl ether (MTBE), methanol and water, as previously described149. Briefly, 260 µl of methanol and 40 µl of water were added to the brain microvessels and vortexed for 20 s. A lipid internal standard mixture was spiked in each sample (EquiSPLASH LIPIDOMIX, Avanti Polar Lipids (cat #: 330731), and d17-Oleic acid, Cayman chemicals (cat #: 9000432)) to control for extraction efficiency, evaluate LC-MS performance and normalize LC-MS data. Samples were diluted with 1,000 µl of MTBE, vortexed for 10 s, sonicated for 30 s three times in a water bath, and incubated under agitation for 30 min at 4°C. After addition of 250 µl of water, the samples were vortexed for 1 min and centrifuged at 14,000g for 5 min at 20°C. The upper phase containing the lipids was collected and dried down under nitrogen. The dry extracts were reconstituted with 150 µl of 9:1 methanol: toluene.

Lipid extracts were analyzed in a randomized order using an Ultimate 3000 RSLC system coupled with a Q Exactive mass spectrometer (Thermo Fisher Scientific) as previously described150. Each sample was run twice in positive and negative ionization modes. Lipids were separated using an Accucore C18 column 2.1 × 150 mm, 2.6 µm (Thermo Fisher Scientific) and mobile phase solvents consisted in 10 mM ammonium acetate and 0.1% formic acid in 60/40 acetonitrile/water (A) and 10 mM ammonium acetate and 0.1% formic acid in 90/10 isopropanol/acetonitrile (B). The gradient profile used was 30% B for 3 min, 30-43% B in 2 min, 43-55% B in 0.1 min, 55-65% B in 10 min, 65-85% B in 6 min, 85-100% B in 2 min and 100% for 5 min. Lipids were eluted from the column at 0.4 ml/min, the oven temperature was set at 45°C, and the injection volume was 5 µl. Autosampler temperature was set at 20°C to prevent lipid aggregation. The Q Exactive was equipped with a HESI-II probe and operated in data dependent acquisition mode for all the samples. To maximize the number of identified lipids, the 100 most abundant peaks found in blanks were excluded from MS/MS events. External calibration was performed using an infusion of Pierce LTQ Velos ESI Positive Ion Calibration Solution or Pierce ESI Negative Ion Calibration Solution.

LC-MS peak extraction, alignment, quantification and annotation was performed using LipidSearch software version 4.2.21 (Thermo Fisher Scientific). Lipids were identified by matching the precursor ion mass to a database and the experimental MS/MS spectra to a spectral library containing theoretical fragmentation spectra. The most abundant ion for each lipid species was used for quantification i.e. [M+H]+ for LPC, LPE, PC, SM and Cer, [M-H]- for PI, PS, PA and PG, and [M+NH4]+ for CE, DAG and TAG. To reduce the risk of misidentification, MS/MS spectra from lipids of interest were validated as follows: 1) both positive and negative mode MS/MS spectra match the expected fragments, 2) the main lipid adduct forms detected in positive and negative modes are in agreement with the lipid class identified, 3) the retention time is compatible with the lipid class identified and 4) the peak shape is acceptable. The fragmentation pattern of each lipid class was experimentally validated using lipid internal standards. Data were normalized using (i) class-specific internal standards to control for extraction efficiency and (ii) median of all annotated lipids to correct for differential quantity of starting material.

### Brain Endothelial Cell Proteomics, Mass Spectrometry

SP3-prepared and STAGE tip cleaned brain microvessel peptides were resuspended in 0.1% formic acid and analyzed by online capillary nanoLC-MS/MS. Samples were separated on an inhouse made 20 cm reversed phase column (100 µm inner diameter, packed with ReproSil-Pur C18-AQ 3.0 µm resin (Dr. Maisch GmbH)) equipped with a laser-pulled nanoelectrospray emitter tip. Peptides were eluted at a flow rate of 400 nL/min using a four-step linear gradient including 2-4% buffer B in 1 min, 4-25% buffer B in 120 min and 25-40% B in 30 min, 40-98% buffer B in 2 min (buffer A: 0.2% formic acid and 5% DMSO in water; buffer B: 0.2% formic acid and 5% DMSO in acetonitrile) in a Dionex Ultimate 3000 LC-system (Thermo Fisher Scientific). Peptides were then analyzed using an LTQ Orbitrap Elite mass spectrometer (Thermo Fisher Scientific). Data acquisition was executed in data dependent mode with full MS scans acquired in the Orbitrap mass analyzer with a resolution of 60000 and m/z scan range of 340-1600. The top 20 most abundant ions with intensity threshold above 500 counts and charge states 2 and above were selected for fragmentation using collision- induced dissociation (CID) with isolation window of 2 m/z, normalized collision energy of 35%, activation Q of 0.25 and activation time of 5 ms. The CID fragments were analyzed in the ion trap with rapid scan rate. Dynamic exclusion was enabled with repeat count of 1 and exclusion duration of 30 s. The AGC target was set to 1000000 and 5000 for full FTMS scans and ITMSn scans, respectively. The maximum injection time was set to 250ms and 100ms for full FTMS scans and ITMSn scans, respectively. The raw files were analyzed as above, but with a precursor mass tolerance set to 20 p.p.m., fragment ion tolerance set to 0.6 Da, and for protein quantification, minimum ratio count was set to two, with unique peptides used for quantification.

### Blood-Brain Barrier Permeability and Perfusion Assessment

Following intravenous injections of 150 µL of saline or plasma (prepared as above), tracers were injected 20 h later as previously described90,139,143. Briefly, mice were injected with fixable 3-kDa dextran-FITC (10 µg/g, Thermo Fisher), 70-kDa dextran-TMR (100 µg/g, ThermoFisher), or 2-mDa dextran-FITC (100 µg/g, Thermo Fisher) dissolved in saline. After 2 h (for 3-kDa dextran-TMR) or 4 h (for 70-kDa dextran-TMR), mice were anesthetized and perfused, and cortices and hippocampi microdissected. Frozen tissue was thawed and suspended in 300 µL of a custom Lysis Buffer (200 mM Tris, 4% CHAPS, 1M NaCl, 8M Urea, pH 8.0). Tissues were then homogenized using a Branson Digital Sonifier sonicator set to 20% amplitude for 3 seconds, allowed to rest for 30 secs on ice, and repeated 3 times. Samples were centrifuged at 14,000g for 20 minutes at 4°C, and supernatant was extracted for analysis. Fluorescence for FITC and Texas Red were measured with a Varioskan Flash microplate reader (Thermo Fisher Scientific). Fluorescence signal was standardized to quantity of protein per sample using the Pierce BCA Protein Assay Kit (Thermo Fisher Scientific). Brains from separate mice were processed for immunohistochemistry as described above, and endogenous mouse IgG imaged and quantified. As a positive control, cortical mild traumatic brain injury (mTBI, also referred to as concussion) injury was induced in mice using a Benchmark Stereotaxic Impactor (MyNeurolab), as previously described151.

### EXAMPLE 1

This example describes experiments which demonstrate that plasma proteins permeate the healthy adult mouse brain.

The BBB excludes nearly 100% of exogenous macromolecules (Pardridge, W. M. NeuroRx (2005). doi:10.1602/neurorx.2.1.3; Abbott et al., Neurobiol. Dis. (2010). doi:10.1016/j.nbd.2009.07.030; Banks, W. A. BMC Neurology (2009). doi:10.1186/1471-2377-9-S1-S3; St-Amour et al., J. Cereb. Blood Flow Metab. (2013). doi:10.1038/jcbfm.2013.160), from tracers used in preclinical research to therapeutic immunoglobulins (IgG). However, the extent to which the BBB excludes the thousands of endogenous blood proteins it is constitutively exposed to remains largely unknown (Anderson, N. L. & Anderson, N. G., Mol. Cell. Proteomics (2002). doi:10.1074/mcp.R200007-MCP200; and Hood et al., J. Proteome Res. (2005). doi:10.1021/pr050107r). To address this, the mouse blood plasma proteome was chemoselectively labeled (Bragg, P. D. & Hou, C., Arch. Biochem. Biophys. (1975). doi:10.1016/0003-9861(75)90467-1; Sélo, I. et al., J. Immunol. Methods (1996). doi:10.1016/S0022-1759(96)00173-1; and Anderson et al., J. Am. Chem. Soc. (1964). doi: 10.1021/ja01063a037) with a variety of small tags to enable a panel of studies upon transfer into a recipient young or aged mouse (Figure 1a; Figures 2d-2e). After optimizing conditions for amine-reactive N-hydroxysuccinimide ester chemistry, labeling of hundreds of plasma proteins was confirmed across abundance, size, and class using antibody array and mass spectrometry-based proteomics, with coverage largely limited by detection method (Figures 3a-3d). Twenty hours upon transfer into healthy adult mice, radiotracer Cu64-labeled plasma depleted of albumin and IgG accumulated at significantly higher levels than the IgG control in both the leakier, fenestrated circumventricular organs and within the BBB-protected brain tissue (Figure 1b; Figures 4a-4e).

To study plasma uptake at a cellular resolution, plasma was labeled with the small but bright far-red Atto 647N fluorophore used in super-resolution imaging (Dempsey et al., Nat. Methods (2011). doi:10.1038/nmeth.1768; Han et al., Nat. Commun. (2017). doi: 10.103 8/s41467-0 17-01503-6). Fluorescence imaging bore a strong resemblance to autoradiography (Figure 1c). Plasma administration did not induce hyperproteinemia or perturb the BBB, as measured functionally by various fixable and non-fixable tracers and transcriptionally by single-cell RNA sequencing (Figures 5a-5f). Moreover, plasma uptake was detectable across injection volumes down to 10 µL (less than 0.5% of the circulatory volume); seen with various *ex vivo* and *in vivo* labeling chemistries; not co-localized with albumin or IgG even in the minority of vasculature containing them (Figures 6a-6c); and not confounded by residual free dye (Figure 7m; Figure 8i).

At greater magnification, two cardinal features were observed: a punctate vasculature and plasma-containing (plasma⁺) parenchymal cells (Figures 1d-1k; Figure 7a-7h). Indicative of either lysosomal degradation or active transcytosis, a punctate vasculature suggests a surprisingly high level of endocytosis by the BBB endothelium to endogenous plasma proteins not seen with exogenous tracers (Chow, B. W. & Gu, C. Trends Neurosci., 38: 598-608 (2015); Reese, T. S. & Karnovsky, M. J., J. Cell Biol. (1967). doi:10.1083/jcb.34.1.207, St-Amour et al., J. Cereb. Blood Flow Metab. (2013). doi:10.1038/jcbfm.2013.160; Rubin, L. L. & Staddon, J. M., Annu. Rev. Neurosci. (1999). doi:10.1146/annurev.neuro.22.1.11; Triguero et al., Proc. Natl. Acad. Sci. (1989). doi:10.1073/pnas.86.12.4761; and Villasenõr etal., Sci. Rep. (2016). doi:10.1038/srep25658). High-resolution imaging revealed plasma protein transcytosis, with both diffuse and punctate signal on the parenchymal side of the CD31⁺ endothelium (Figure 1e; Figure 7i). Plasma moreover accumulated in the choroid plexus, subarachnoid space, and perivascular space, implicating a potential contribution by the glymphatic system in its uptake, distribution, and clearance (Figure 7j-7l) (Iliff et al., Sci. Transl. Med. (2012). doi:10.1126/scitranslmed.3003748; Louveau et al., Nature, 523: 337-341 (2015); Da Mesquita et al., Nature (2018). doi:10.1038/s41586-018-0368-8). While BBB-permeability for select proteins like transferrin and insulin has been investigated in crude brain homogenate or isolated vessels (Pardridge et al., Metabolism (1987). doi:10.1016/0026-0495(87)90099-0; Poduslo et al., Proc. Natl. Acad. Sci., 91, 5705-5709 (1994); Pardridge, W. M., Journal of NeuroVirology (1999). doi:10.3109/13550289909021285; Pardridge, W. M., Endocr. Rev. (1986). doi:10.1210/edrv-7-3-314; Pardridge, W. M., Drug Discovery Today (2007). doi:10.1016/j.drudis.2006.10.013; and Poduslo et al., Neurobiol. Dis. (2001). doi:10.1006/nbdi.2001.0402), plasma⁺ neurons and microglia could be visualized directly *in situ* across brain regions, including the cortex and hippocampus (Figures 1g-1k). In the hippocampal dentate gyrus, plasma traced the vasculature and adjoining neuronal processes (Figure 1i). By staining for transferrin, the basis of existing BBB drug delivery programs (Villaseñor et al., Cell Rep., 21: 3256-3270 (2017); Zucheroet al., Neuron (2016). doi:10.1016/j.neuron.2015.11.024; Couch et al., Sci. Transl. Med. (2013). doi:10.1126/scitranslmed.3005338; Atwal et al., Sci. Transl. Med. (2011). doi:10.1126/scitranslmed.3002254; Yu et al., Sci. Transl. Med. (2011). doi:10.1126/scitranslmed.3002230; and Niewoehner et al., Neuron (2014). doi:10.1016/j.neuron.2013.10.061), substantial non-colocalized plasma signal was observed in neurons, suggesting that other circulatory factors are similarly BBB-permeable (Figure 1k).

Together, these data show that endogenous circulatory proteins constitutively enter and permeate the healthy adult brain.

### EXAMPLE 2

This example demonstrates the regulation of plasma uptake by the brain vasculature.

Due to the unexpectedly high amount of plasma uptake by the brain, the complex system of genetic regulators (Jefferies et al., Nature (1984). doi: 10.1038/312162a0; Broadwell, R. D.; Acta Neuropathologica (1989). doi:10.1007/BF00294368; Daneman, R., Ann. Neurol. (2012). doi:10.1002/ana.23648; and Zlokovic, B. V., Neuron (2008). doi:10.1016/j.neuron.2008.01.003) that plasma harnesses for enhanced BBB transport was investigated. A `functional transcriptomics' platform was developed that records plasma uptake by endothelial cells via flow cytometry and index sorts them for deep, single-cell RNA sequencing (scRNA-seq, average 1.5 million reads per cell). Linking transcriptomic data and plasma uptake for every cell enabled an unbiased and high-throughput correlation between each gene's expression and degree of plasma uptake. 745 brain endothelial cells were processed from healthy adult mice four hours after administration of fluorescently-labeled plasma to ensure measurement of transcytosis, as previously described (Niewoehner, *supra;* Zuchero et al., Neuron (2016). doi:10.1016/j.neuron.2015.11.024; and Friden et al., Proc. Natl. Acad. Sci. (2006). doi:10.1073/pnas.88.11.4771). Across the 745 brain endothelial cells and 19,899 genes sequenced, a hierarchy of plasma uptake suggestive of regulatory control was observed (Figure 9a; Figure 10a-10d).

Although the vast majority of the 19,899 sequenced genes showed no effect, specific genetic correlates of enhanced or inhibited uptake were identified, as shown in Table 1 and Figure 9b. Certain genes, like transferrin receptor (Tfrc) and CD98hc (Slc3a2), served as validating controls; and as expected, genes that correlated with enhanced plasma uptake ("Correlates") were enriched in the vesicle-mediated transport pathway (Figure 9c). Surprisingly, Correlate genes were also enriched for solute carrier (SLC) transmembrane proteins canonically responsible for shuttling glucose, metal ions, and other small molecules. Their enrichment suggests a possible dual role in protein transcytosis, as recently discovered for the new receptor-mediated transcytosis (RMT) BBB delivery target Slc3a2 encoding CD98hc (Zuchero et al., Neuron (2016). doi:10.1016/j.neuron.2015.11.024). Interestingly, several Correlates have been shown to promote BBB integrity, such as Apoe (Bell et al., Nature (2012). doi:10.1038/nature11087; Zlokovic, JAMA Neurol. (2013). doi:10.1001/jamaneurol.2013.2152) and Mfsd2a (Andreone et al., Neuron, 94: 581-594.e5 (2017); and Ben-Zvi et al., Nature (2014). doi:10.1038/nature13324), suggesting that the mechanisms driving plasma uptake are regulated and not from compromise. It also suggests that these genes either promote uptake of specific plasma proteins via unknown mechanisms or are tightly co-expressed with known receptors that do (Figure 9i). Genes whose expression inversely correlated with plasma uptake ("Anticorrelates") clustered around extracellular vascular and soft tissue calcification and ossification pathways (Figure 9c). Both Correlates and Anticorrelates localized to the cell surface, suggesting plasma uptake is mediated by direct binding to receptors; and is inhibited by cell surface proteins acting in cis or sequestration in the extracellular matrix (Fig. 2d). Finally, a strong enrichment of BBB-specific genes (Daneman et al., PLoS One (2010). doi:10.1371/journal.pone.0013741) was found among Correlates and Anticorrelates, suggesting that these regulators of plasma uptake and transcytosis may be unique to the BBB (Figure 9e). The identification of cell surface Correlates and Anticorrelates offers new molecular targets to modulate BBB transport.

**Table 1**

| | | | | | |
|---|---|---|---|---|---|
| **Gene** | **Summary** | **Disease assoc.** | **Corr.** | **Percentile** | **References** |
| Slc7a5 | Forms heterodimer with Denali's Slc3a2. Also transports L-DOPA, pregabalin, and other CNS drugs. Also called CD98 and LAT1. Enriched on BBB. | Phenylketonuria | 0.48 | 99.98% | doi: 10.1007/s11095-018-2532-0 |
| | | | | | doi: 10.1073/pnas.96.21.12079 |
| Slc22a8 | Transports neurosteroids and several (non-CNS) drugs. Also known as OAT3. Canonically considered an efflux transporter. | N/A | 0.46 | 99.97% | doi: 10.3389/fphar.2018.00354 |
| | | | | | doi: 10.1002/bdd.693 |
| Lrp8 | Also known as APOER2. Decreased expression associated with some neurological diseases. Important for brain development and LTP. Receptor for Clusterin. Transports several proteins, including APOE. Expression decreases with AD. Involved in Abeta processing. Also implicated in major depressive disorder and antiphospholipid syndrome. | Alzheimer's | 0.32 | 99.75% | doi: 10.3390/ijms19103090 |
| | | Disease, | | | doi: 10.1186/1750-1326-6-30 |
| | | Major Depressive | | | doi: 10.1016/j.neuint.2006.07.007 |
| | | Disorder, | | | doi: 10.1016/j.semcdb.2008.10.005 |
| | | Antiphospholipid | | | doi: 10.1016/j.pnpbp.2010.05.014 |
| | | Syndrome | | | doi: 10.1111/j.1538-7836.2005.01359.x |
| Ctgf/Ccn2 | Strongly upregulated in AD. Interacts with VEGF and members of TGFb family. BECs in knockout show impaired interaction with pericytes. | Fibrotic Disease Cancer | 0.29 | 99.58% | doi: 10.1096/fj.05-4359fje |
| | | | | | doi: 10.1096/fj.01-0332fje |
| | | | | | doi: 10.1038/nrd3599 |
| | | | | | doi: 10.1371/joumal.pone.0030562 |
| | | | | | doi: 10.1007/s00018-011-0782-7 |
| Kdr | Also known as VEGFR2. Known to affect BBB permeability. | Corneal neurovascularization | 0.34 | 99.80% | doi: 10. 1073/pnas. 142224299 |

| **Gene** | **Summary** | **Disease assoc.** | **Corr.** | **Percentile** | **References** |
|---|---|---|---|---|---|
| Igf1r | IGF-1 receptor. Knockout or haploinsufficiency causes delayed Abeta accumulation in AD mouse models. Impaired brain Igf1r increases lifespan. rhIGF-1 reduces permeability of BBB | Alzheimer's disease | 0.33 | 99.78% | doi: 10.2174/18715273113126660141 |
| | | | | | doi: 10.1371/journal.pbio.0060254 |
| | | | | | doi: 10.1016/j.expneurol.2018.11.009 |
| Slc16a1 | Monocarboxylate transporter 1. Similar densities on luminal and abluminal side. Involved in lactate transport. | ALS (1) | 0.44 | 99.95% | doi: 10.1038/jcbfm.2014.206 |
| Slc38a5 | Sodium-coupled amino acid transporter (glutamine, asparagine, histidine, serine, alanine, and glycine). Strongly enriched on BBB vs other EC compartments. | Autism (2) | 0.47 | 99.98% | doi: 10.1111/j.1471-4159.2009.06481.x |
| | | | | | doi: 10.1016/j.cell.2016.11.013 |
| Spare | Also known as osteonectin. Glycoprotein that binds calcium. Involved in bone mineralization. Spare is "anti-adhesive" - does not support cell attachment and inhibits cell spreading. Regulates EC proliferation (expression correlates with proliferation). Knockout mice available. Expression goes up in response to LPS and Tnfa. Thought to increase BBB permeability. | | 0.40 | 99.91% | doi: 10.1097/00003086-199208000-00042 |
| | | | | | doi: 10.1186/s12974-016-0657-9 |
| Bsg | Also known as extracellular matrix metalloproteinase inducer and CD147. Associates with MCTs (like Slc16a1). Discussed as shuttle target. | Covid-19 (2) | 0.26 | 99.42% | doi: 10.1016/j.neuron.2015.11.024 |
| | | | | | doi:10.1101/2020.03.14.988345 |
| Car4/Ca4 | Carbonic anhydrase 4. Carbonic anhydrases reversibly hydrate carbon dioxide and are involved in calcification. Possibly functions as CO2/HCO3 transporter. Associates with MCTs and Bsg. Only expressed on luminal side. | | 0.39 | 99.90% | doi: 10.1007/978-94-007-7359-2_9 |
| | | | | | doi: 10.1074/jbc.RA118.005536 |
| | | | | | doi: 10.1074/jbc.M113.537043 |
| | | | | | doi: 10.1002/hep.510240521 |
| Tmem98 | Involved with Wnt/ß-catenin signalling. Reduces Wnt/B-catenin signaling. Involved in oligodendrocyte differentiation. | Multiple Sclerosis | 0.35 | 99.84% | doi: 10.1371/journal.pone.0227435 |
| | | | | | doi: 10.1523/JNEUROSCI.0154-18.2018 |
| | | | | | doi: 10.1073/pnas.1609905114 |
| Slco1c1 | Responsible for uptake of thyroid hormones into brain tissue. | Hyperthyroidism Depression Fatigue | 0.34 | 99.79% | doi: 10.1016/j.beem.2007.03.004 |
| | | | | | doi: 10.1111/j.1365-2265.2008.03267.x |
| Slc7a1 | Glucose and amino acid transporter. Important for arginine transport. | | 0.39 | 99.89% | doi: 10.1038/s41598-017-04965-2 |
| Itm2a | Involved in osteo- and chondrogenic cellular differentiation, as well as T cell activation. Structurally related protein Itm2b is involved in a familial form of cerebral amyloid angiopathy. | Graves Disease | 0.41 | 99.93% | doi:10.1074/jbc.271.32.19475 |
| | | | | | doi:10.1084/jem.190.2.217 |
| | | | | | doi: 10.1210/jc.2016-2625 |
| | | | | | doi:10.1007/s00018-005-5092-5 |
| Cd9 | Known involvement in exosome transport (exosome marker). Colocalizes with VCAM1 and ICAM1. Known to be important for leukocyte adhesion and extravasation. Knockdown reduces VCAM1 and ICAM1 expression. Blocking CD9 strongly enhanced the barrier function of the BBB in vitro. | HIV Multiple Sclerosis | -0.38 | Bottom 99.97% | doi: 10.1074/jbc.273.32.20121 |
| | | | | | doi: 10.3389/fimmu.2018.00863 |
| | | | | | doi: 10.3389/fimmu.2018.02316 |
| | | | | | doi: 10.1002/glia.22565 |
| | | | | | doi: 10.4049/jimmunol.177.8.5129 |
| Cdh 13 | Unique member of the cadherin family known as T-cadherin. Acts as negative regulator of axon growth during neural differentiation. Protects vascular endothelial cells from apoptosis due to oxidative stress, and is associated with resistance to atherosclerosis. Upregulated in response to oxidative stress. | ADHD | -0.43 | Bottom 100% | doi: 10.1016/j.euroneuro.2012.06.009 |
| | | | | | doi: 10.1096/fj.05-3834fje |
| Thbd | Thrombomodulin. Binds thrombin and activates Protein C to reduce blood coagulation. Important for myelination. Anti-coagulant and anti-inflammatory. Expression reduced and shedding increased by IL-6, TNF-a. Increased expression with laminar shear stress. | | -0.09 | Bottom 98.24% | doi:10.1055/s-0038-1657558 |
| | | | | | doi: 10.1186/1423-0127-19-34 |
| | | | | | doi: 10.1016/j.mvr.2014.09.003 |
| Mcam | Also known as CD146. Receptor for laminin alpha 4. Also expressed by pericytes. Two isoforms exist. Blockade impedes brain T-cell infiltration. Coordinates brain endothelial cell-pericyte communication for blood-brain barrier development. Important for ECs to initiate pericyte association. After pericyte-EC connection, ECs no longer express but pericytes maintain expression. Interacts with PDGFRB. Soluble CD146 in CSF is a marker of BBB dysfunction and inflammation. | | -0.16 | Bottom 99.31% | doi:10.1371/journal.pone.004044 |
| | | | | | doi: 10.1007/s13238-017-0484-5 |
| | | | | | doi: 10.1186/s12974-018-1276-4 |
| | | | | | doi: 10.1073/pnas.1710848114 |
| | | | | | doi: 10.7150/thno.37142 |
| Gcnt1 | Member of the beta-1,6-N-acetylglucosaminyltransferase gene family. It is essential to the formation of Gal beta 1-3(GlcNAc beta 1-6)GalNAc structures and the core 2 O-glycan branch. Involved in Notch signaling in T cells | | -0.27 | Bottom 99.80% | doi: 10.4049/jimmunol.1901194 |
| Ccn3 | Also called NOV. Has homologies with von Willebrand factor type C. Directly binds integrin receptors. Involved in adhesion, migration, proliferation, differentiation, and survival. Can also bind BMP2 (connections with TGF superfamily). Regulates endothelial inflammation. Knockdown increased VCAM1 expression. Ccn3 reduces monocyte adhesion. Expression regulated by Klf2. Negatively regulates Nf-kB. | | -0.34 | Bottom 99.95% | doi: 10.1016/j.biocel.2008.07.025 |
| | | | | | doi: 10.1074/jbc.M404903200 |
| | | | | | doi: 10.1016/j.bbrc.2007.01.029 |
| | | | | | doi: 10.1007/s12079-010-0095-x |

The brain vasculature follows an arteriovenous zonation (Vanlandewijck et al., Nature, 554: 475-480 (2018); Chen et al., bioRxiv (2019). doi:10.11 01/617258; Simionescu et al., J. Cell Biol. (1976). doi:10.1083/jcb.68.3.705) (Figure 9f). Unexpectedly, plasma uptake varied distinctly by vessel segment, with venous cells endocytosing the most plasma, arterial cells the least, and capillaries in between but exhibiting the widest distribution (Figure 9g). Plasma uptake thus inversely correlated with the blood pressure felt by the vessel segment. Each vessel segment exhibited a unique transcriptional program correlated with plasma uptake and its inhibition (Figure 9h). Within capillaries, *Tfrc* expression paralleled other Correlates' such as *Mfsd2a,* forming a co-expression module that suggests co-orchestration by a common upstream signaling pathway (Figure 9i). Expression of this *Tfrc* module was mutually exclusive with that of strong Anticorrelates like *Alpl*, marking mutually exclusive populations within capillaries. To further investigate variable plasma uptake across capillaries, endothelial cells were ordered into a one-dimensional axis by SPIN (sorting points into neighborhoods) (Vanlandewijck et al., *supra*; and Tsafrir et al., Bioinformatics (2005). doi:10.1093/bioinformatics/bti329). Capillaries more transcriptionally similar to arterial cells endocytosed plasma like an arterial cell, and vice versa for venous-like capillaries (Figure 9j; Figure 10e). This suggests a transcriptional and perhaps spatial gradient of plasma uptake along the cerebral arteriovenous tree. Although individual genetic correlates of plasma uptake are expressed throughout arteriovenous zonation (Figure 9k; Figure 10f), their assembly into distinct co-expression modules likely determines vessel segment differences in uptake.

Together, these data suggest that in health, BBB-specific and therapeutically relevant transcriptional programs actively regulate plasma transport and are unique to each vessel segment.

### EXAMPLE 3

This example describes an age-related shift in BBB transcytosis.

With age, the BBB is 'leakier' to exogenous tracers (Montagne et al., Neuron (2015). doi:10.1016/j.neuron.2014.12.032). IgG brain uptake was detected at levels consistent with prior work (Poduslo et al., Proc. Natl. Acad. Sci., 91: 5705-5709 (1994)), and concordantly, an increase in IgG accumulation with age was observed by both autoradiography and quantitative gamma counting (Figure 4c; Figures 8b-8c). Surprisingly, however, plasma uptake diminished in the aged brain (Figures 8a-8b; Figure 4c). This held true whether whole brains or de-vascularized cortical and hippocampal parenchymal homogenate were analyzed (Figures 8b-8c). Nevertheless, plasma uptake consistently exceeded that of IgG, further suggesting a caveolae-independent mechanism mediating enhanced plasma transport across the BBB.

To investigate the mechanisms responsible for the age-related impairment in plasma transport, the scRNA-seq data was combined with a previously published brain endothelial cell transcriptomic ageing dataset (Yousef et al., Nat. Med. (2019). doi:10.1038/s41591-019-0440-4) (Figure 8d). Of the genes downregulated with age, a remarkable 85% correlated with plasma uptake in youth (92% of the area under the curve). Enrichment analysis highlighted a loss of clathrin adapter activity and ATP utilization, hinting at defects in active RMT. Indeed, a global shift from ligand-specific RMT to nonspecific caveolar transcytosis with age was observed in brain endothelial cells (Figure 8e; Figures 11a-11b). Putative RMT receptors, including Tfrc, decreased with age at both the transcript and protein level, as did downstream clathrin components and its adapters. Cav1, obligatory for caveolae formation (Andreone et al., *supra*), increased with age at both the transcript and protein level, while Mfsd2a, which suppresses caveolar transcytosis, decreased with age.

To functionally validate this age-related shift in endothelial transcytosis, three independent approaches were employed: lipidomics, proteomics, and flow cytometry. Recent work has shown that the unique lipid composition of brain endothelial cells underlies their low rates of caveolar transcytosis; and that Mfsd2a maintains these properties by embedding omega-3 fatty acid docosahexaenoic acid (DHA)-containing phospholipids into the luminal plasma membrane to preclude the formation of caveolae (Andreone et al., Neuron (2017). doi:10.1016/j.neuron.2017.03.043, Ben-Zvi et al., Nature (2014). doi:10.1038/nature13324; and Nguyen et al., Nature (2014). doi:10.1038/nature13241). Consistent with diminished Mfsd2a expression with age, unbiased lipidomic analysis revealed an age-related reduction in DHA-containing phospholipid species in the lysophosphatidylethanolamine (LPE), phosphatidylcholine (PC), phosphatidylethanolamine (PE), and phosphatidylserine (PS) classes (Figures 8f-8g; Figure 11c). These were the very DHA-containing classes differentially enriched in brain over peripheral endothelial cells (Andreone et al., Neuron (2017). doi:10.1016/j.neuron.2017.03.043). Among differentially 160 abundant lipids, an age-upregulation of specific caveolae-related lipids, such as sphingomyelins, ethanolamine plasmalogens (PE-p), and ceramides (Extended Data Fig. 8c), also was observed.

Transferrin and albumin are canonical ligands for protein RMT and caveolar transcytosis, respectively (Fishman et al., J. Neurosci. Res. (1987). doi:10.1002/jnr.490180206; Roberts et al., J. Cell Sci. (1993); Lajoie, J. M. & Shusta, E. V., Annu. Rev. Pharmacol. Toxicol. (2014). doi:10.1146/annurevpharmtox-010814-124852; and Schubert et al., J. Biol. Chem. (2001). doi:10.1074/jbc.C100613200). By mass spectrometry-based proteomics, a decrease in endogenous transferrin and a concomitant increase in albumin abundance was observed within aged microvessels (Figure 8h). This result is consistent with an age-related decline in *Tfrc* and clathrin expression, and an increase in ligand-nonspecific caveolae. Although the abundance of endogenous albumin was below the limit of detection in most microvessel samples from young mice, it was consistently detected with age. Transferrin abundance exceeded that of albumin, but the difference narrowed with age, supporting the hypothesis that RMT is the predominant route of transcytosis across the young BBB but transitions to caveolar transcytosis with age. Similar patterns were observed using the injected RMT ligand leptin and caveolae-transported ligand horseradish peroxidase (HRP), with the young endothelium taking up more of the leptin but the aged more of the HRP (Figure 19a).

Finally, since an age-related change in functional transport was measured consistent with the predicted shift from receptor-mediated (exemplified by transferrin, leptin) to caveolar (IgG, albumin, HRP) transcytosis, the frequency of clathrin and caveolar vesicles was visualized and quantified with age. Through high-resolution microscopy of antibody-stained microvessels, a greater density of clathrin vesicles was observed in the young brain endothelium but more caveolar vesicles were observed in the aged brain (Figures 19b and 19c).

In contrast to RMT, which was hierarchical across endothelial cells (Figure 9a), caveolar transport pathways exhibit broad, ligand-nonspecific uptake (Nabi et al., Journal of Cell Biology (2003). doi:10.1083/jcb.200302028; and Villaseñor et al., Cellular and Molecular Life Sciences (2019). doi:10.1007/s00018-018-2982-x). It was thus hypothesized that with age, endothelial cells should have a more uniform pattern of uptake. Using flow cytometry, first it was confirmed that the detected plasma signal was specific, enabling reliable quantification that circumvents age-related autofluorescence (Spitzer et al., J. Neurosci. Methods (2011). doi:10.1016/j.jneumeth.2011.01.029; Schnell et al., J. Histochem. Cytochem. (1999). doi:10.1177/002215549904700601 (Fig. 3i; Extended Data Fig. 8d; Extended Data 177 Fig. 11a-e). An increase in the proportion of plasma⁺ endothelial cells with age was observed, which is consistent with the non-specificity of caveolar transport (Figure 8j). In contrast, the average amount of plasma within endocytosing cells decreased with age, which is in line with autoradiography and gamma counting data (Figures 8a-8b) and suggests that RMT is a more productive pathway than caveolar for plasma protein transcytosis. This age-related shift also was identified by microscopy, with a more heterogenous and hierarchical pattern of plasma uptake observed in the young vasculature compared to a more diffuse and uniform signal observed with age (Figure 8k). This shift was also evident in both NeuN⁺ and Thy1⁺ neurons (Figure 8l; Figure 11e), but not in ACS2-A⁺ astrocytes (Figure 11f).

The data described above suggest that the aged BBB undergoes a shift from receptor-mediated to caveolar transcytosis, and that this results in both an altered composition of BBB-permeable proteins and an overall decrease in plasma uptake. A model of the shift in BBB transcytosis with age is schematically depicted in Figure 16.

### EXAMPLE 4

This example describes age-related calcification in the CNS and that ALPL inhibition restores BBB transcytosis.

To discover overt anatomical changes associated with the age-related shift in brain endothelial transport, vascular density, astrocyte coverage, and pericyte coverage were assessed with age (Figures 20a and 20b). A specific loss of pericyte coverage was observed (Figures 20A and 20b), with several pericyte-induced genes, such as *Tfrc*, *Lrp8*, and *Mfsd2a,* decreasing both in pericyte-deficient Pdgf^{*ret*/*re*t} transgenic models (Armulik et al., Nature, 468: 557-561 (2010)) and normal ageing (Figures 20b and 20c). This suggests a role for pericytes in maintaining mature BBB function. Pericyte deficient transgenic models display ectopic calcifications, manifesting as bone-dense nodules and vascular spheroids (Keller et al., Nat. Genet. (2013). doi:10.1038/ng.2723; and Zarb et al., Brain (2019). doi:10.1093/brain/awz032). Remarkably, such calcifications were phenocopied in normal, disease-free ageing (Figure 12b). Thus, pericyte loss and concomitant ectopic brain calcifications and BBB dysfunction may be normal features of brain ageing. Molecularly, calcifications arise from the co-localization of Type I collagen and the alkaline phosphatase ALPL (Murshed et al., Genes Dev. (2005). doi:10.1101/gad.1276205), both of which increased in parallel with age (Figures 12d and 12e).

Several major drug and clinical development programs to enhance CNS drug delivery rely on BBB RMT, with the majority targeting the transferrin receptor, TFRC (Atwal et al., Sci. Transl. Med. (2011). doi:10.1126/scitranslmed.3002254; Yu et al., Sci. Transl. Med. (2011). doi:10.1126/scitranslmed.3002230; Zuchero et al., Neuron (2016). doi:10.1016/j.neuron.2015.11.024; Sonoda et al., Mol. Ther. (2018). doi:10.1016/j.ymthe.2018.02.032; Bien-Ly et al., J. Exp. Med. (2014). doi:10.1084/jem.20131660; and Oller-Salvia et al., Chemical Society Reviews (2016). doi:10.1039/c6cs00076b). Having observed a loss of BBB RMT with age, a search for molecular targets to enhance aged BBB transcytosis (Figure 12a) was performed by filtering for genes that were (1) BBB-enriched (Figures 13a-13b); (2) expressed on the endothelial cell surface (Figure 12a); (3) upregulated with age (Figures 12d-12e; Figures 13d-13e); (4) Anticorrelates of plasma uptake (Figure 9b; Figure 13c); and (5) amenable to inhibition with FDA-approved small molecules and improved derivatives (Debray et al., Bioorganic Med. Chem. (2013). doi:10.1016/j.bmc.2013.09.053; and Sheen et al., J. Bone Miner. Res. (2015). doi:10.1002/jbmr.2420). Only the alkaline phosphatase gene, Alpl, met these criteria.

Yet, Alpl's canonical role is the key effector of bone calcification (Sheen et al., J. Bone Miner. Res. (2015); Murshed et al., Genes Dev. (2005). doi:10.1101/gad.1276205; Villa-Bellosta, R. & O'Neill, W. C., Kidney International (2018). doi:10.1016/j.kint.2017.11.035; Savinov et al., J. Am. Heart Assoc. (2015). doi:10.1161/JAHA.115.002499; Romanelli, F., PLoS One (2017). doi:10.1371/journal.pone.0186426)). Remarkably, alizarin red staining detected calcification (bone-dense nodules) in normally aged brains (Figure 12b). These calcifications recapitulated those observed in the pericyte-deficient Pdgfret/ret mouse model (Keller, A. et al., Nat. Genet. (2013). doi:10.1038/ng.2723; Vanlandewijck, M. et al., PLoS One (2015). doi:10.1371/journal.pone.0143407; Zarb et al., Brain (2019). doi:10.1093/brain/awz032; Sagare et al., Nat. Commun. (2013). doi:10.1038/ncomms3932; Montagne et al., Nat. Med. (2018). doi:10.1038/nm.4482; and Bell et al., Neuron (2010). doi:10.1016/j.neuron.2010.09.043), though pericyte loss has not been demonstrated in normal, disease-free ageing. Molecularly, vascular calcification is marked by the co-localization of Type I collagen and ALPL (encoded by Alpl), as it is only in this condition that extracellular matrix mineralization occurs (Murshed et al., Genes Dev. (2005). doi:10.1 101/gad.1276205; and Villa-Bellosta, R. & O'Neill, W. C., Kidney International (2018). doi:10.1016/j.kint.2017.11.035). Vascular overexpression of ALPL, which hydrolyzes the potent inhibitor of mineralization - pyrophosphate, is sufficient to induce ectopic vascular calcification (Sheen et al., J. Bone Miner. Res. (2015); Savinov et al., J. Am. Heart Assoc. (2015). doi:10.1161/JAHA.115.002499; and Romanelli, F., PLoS One (2017). doi:10.1371/journal.pone.0186426). Thus, the surprising increase in both Type I collagen and ALPL in the aged brain endothelium is consistent with an increasingly osteogenic environment and vascular calcification with age (Figure 12c-12e).

Consistent with the non-overlapping expression of *Alpl* and *Tfrc* in capillaries (Figure 9i), holo-transferrin was preferentially taken up by ALPL-deficient capillaries (Figure 21a). To determine whether this correlation hinted at a causal mechanism, scRNA-seq of aged brain endothelial cells was performed after pharmacologic ALPL inhibition. Remarkably, the top upregulated gene after ALPL inhibition was the transferrin receptor, with a ~70% expression increase in capillaries and ~89% increase in venous cells (Figures 21b and 21c).

Administration of a selective ALPL inhibitor (Dahl et al., J. Med. Chem. (2009). doi:10.1021/jm900383s; herein incorporated by reference in its entirety) decreased ALPL activity and thus, mineralization activity in the aged brain vasculature (Figure 12f-12g). The effect of ALPL expression on transferrin uptake in the aged mouse brain (23 mo.) is shown in Figure 18.

To validate a pleiotropic role for Alpl in BBB protein uptake, an injection paradigm from prior work (Armulik et al., Nature, 468: 557-561 (2010) was adopted (Figure 12f), and increased plasma uptake was observed in brain endothelial cells from both young and aged mice upon selective ALPL inhibitor treatment (Figures 14a-14b; Figure 15e). In aged mice, increased plasma transport across the endothelium to brain parenchymal cells was observed (Figure 12i). The percentage of plasma⁺ cells did not change, yet the amount of plasma taken up by participating cells increased. Thus, based on prior flow cytometry and imaging data (Figures 8i-8l; Figures 11d-11e), ALPL inhibition likely does not alter age-upregulated caveolar transport (Figure 14h), but instead promotes protein RMT. Interestingly, ALPL inhibition trended towards, but did not significantly enhance, plasma transcytosis across the young BBB to the parenchyma, despite greater uptake in the endothelium (Figure 12j; Figure 14a). This could be due to restricted expression of ALPL to arterial cells in the young BBB, which constitute a minority of the vasculature, and thus are insufficient in number to confer significantly enhanced transcytosis upon ALPL inhibition (Figures 12d-12e; Figures 13d-13e). It is possible that inhibition of ALPL phosphatase activity works in opposition to imatinib, a tyrosine kinase inhibitor previously shown to arrest BBB transcytosis (Armulik et al., Nature (2010). doi:10.1038/nature09522; and Su et al., Nat. Med. (2008). doi:10.1038/nm178791,92)

Because existing transferrin receptor antibodies in clinical development similarly cross the BBB via RMT, it was investigated whether ALPL inhibition could exemplify a new class of general "adjuvants" to boost brain uptake. Indeed, increased parenchymal uptake was observed after ALPL inhibition for both human holo-transferrin and a high affinity, commercially-available transferrin receptor antibody (Figures 12k-12l; Figures 14c-14f). Enhancement of transferrin receptor antibody uptake was not as strong as that of holo-transferrin, consistent with prior work showing that such high-affinity, bivalent antibody variants are preferentially trapped and degraded in the endothelium (Yu et al., Sci. Transl. Med. (2011). doi:10.1126/scitranslmed.3002230; Niewoehner et al., Neuron (2014). doi:10.1016/j.neuron.2013.10.061, Bien-Ly et al., J. Exp. Med. (2014). doi:10.1084/jem.20131660, Haqqani et al., J. Neurochem. (2018). doi:10.1111/jnc.14482; and Johnsen et al., Theranostics (2018). doi:10.7150/thno.25228). Parenchymal access of holo-transferrin and the transferrin receptor antibody after ALPL inhibition was visually confirmed *in situ*, with accumulation in several cell types such as cortical NeuN⁺ neurons (Figure 12m; Figure 14e). ALPL inhibition did not perturb vascular morphology or compromise paracellular tight junctions (Figures 14g-14h).

The results of this example show that brain calcification is an attribute of normal ageing, and that inhibition of age-upregulated, calcification-promoting ALPL in the vasculature can enhance uptake of plasma and therapeutically relevant biologics.

### EXAMPLE 5

Gene correlates of plasma uptake were determined for brain capillaries, veins, and arterioles in younger and older subjects, and the results are shown in Figures 17A-17C, respectively. This information is useful for targeting a gene and/or vessel type that is specifically involved in a particular disease or other context to potentially minimize toxicity.

### EXAMPLE 6

This example demonstrates that ALPL inhibition upregulates blood-brain barrier iron transporters Tfrc and Slc40a1, and provides a therapeutic approach for treating restless legs syndrome (RLS).

Restless Legs Syndrome (RLS) is a serious movement-related sleep disorder affecting 7-10% of the general population, with 3% requiring therapeutic intervention (Allen et al., Arch. Intern. Med. (2005). doi:10.1001/archinte.165.11.1286; Innes, K. E., Selfe, T. K. & Agarwal, P. Sleep Medicine (2011). doi:10.1016/j.sleep.2010.12.018; Ohayon et al., Sleep Medicine Reviews (2012). doi:10.1016/j.smrv.2011.05.002). Both prevalence and severity are exacerbated with age (Milligan, S. A. & Chesson, A. L., Drugs and Aging (2002). doi:10.2165/00002512-200219100-00003). Although the exact pathogenesis of RLS is unclear, clinical studies consistently indicate that brain iron insufficiency is associated with RLS. Yet, peripheral iron supplementation is hampered by iron's limited permeability across the blood-brain barrier (BBB), necessitating facilitated delivery via dedicated transporters (Mills et al., Future Medicinal Chemistry (2010). doi:10.4155/fmc.09.140; and Chiou, B. et al., PLoS One (2018) doi:10.1371/journal.pone.0198775). Recent research has revealed impaired expression of the very genes mediating homeostatic iron transport across the BBB in RLS patients (Mizuno et al., J. Sleep Res. (2005). dol:10.1111/j.1365-2869.2004.00403.x; and Connor et al., Brain (2011). doi:10.1093/brain/awr0127,8). Thus, there remains a significant unmet need for therapeutics that can promote iron delivery to the brain to restore iron insufficiency.

It has been found that intravenous administration of a selective ALPL inhibitor employing the methodologies described herein is sufficient to enhance the iron transport pathway across the BBB (shown schematically in Figure 22). Specifically, iron transport is determined by two critical genes: the transferrin receptor (TFRC) for import of iron into the brain endothelium and ferroportin for productive channeling of imported iron into the brain parenchyma proper. ALPL inhibition strongly upregulated both TFRC and ferroportin expression on the aged BBB (Figure 23). Indeed, across the entire genome, TFRC was the strongest upregulated receptor and ferroportin was the strongest upregulated transporter upon ALPL inhibition as shown in Tables 2 and 3 and Figure 23. ALPL inhibition promoted TFRC and ferroportin expression in BBB capillaries and venous cells, where they are normally expressed (Figures 24a-24c).

**Table 2**

| **Receptors** | **% increase** | **pct, ALPL inh.** | **pct, Control** | **P_val_adj** |
|---|---|---|---|---|
| Tfrc | 70.1% | 0.723 | 0.508 | 8.06E-107 |
| Lpar6 | 37.0% | 0.446 | 0.283 | 2.99E-42 |
| Fcgrt | 24.2% | 0.849 | 0.732 | 6.16E-39 |
| Scarb1 | 11.1% | 0.45 | 0.365 | 1.34E-08 |

**Table 3**

| **Transporters** | **% increase** | **pct, ALPL inh.** | **pct, Control** | **P_val_adj** |
|---|---|---|---|---|
| Slc40a1 | 35.0% | 0.62 | 0.44 | 1.22E-50 |
| Slc6a6 | 24.9% | 0.99 | 0.97 | 2.39E-64 |
| Slc16a4 | 18.8% | 0.42 | 0.29 | 2.91E-28 |
| Slc26a10 | 17.8% | 0.19 | 0.14 | 1.83E-06 |
| Sle16a2 | 16.5% | 0.66 | 0.55 | 6.72E-18 |
| Slco1c1 | 15.7% | 0.97 | 0.94 | 1.46E-22 |
| Slc7a1 | 12.2% | 0.72 | 0.66 | 1.31E-03 |
| Slc39a10 | 12.1% | 0.89 | 0.82 | 1.23E-12 |
| Slc22a8 | 11.0% | 0.71 | 0.66 | 9.75E-03 |
| Slc39a8 | 10.9% | 0.34 | 0.27 | 1.24E-04 |
| Slc25a1 | 10.7% | 0.24 | 0.17 | 1.15E-08 |

These findings demonstrate that ALPL inhibition provides a pharmacologic approach to upregulate the determinants of iron transport into the brain, offering a therapeutic approach to RLS.

In some embodiments, ALPL inhibition is used in combination with other treatments or therapies for RLS. For example, in some embodiments, ALPL inhibition is combined with ropinirole (REQUIP), rotigotine (NEUPRO), pramipexole (MIRAPEX), gabapentin (NEURONTIN, GRALISE), gabapentin enacarbil (HORIZANT), pregabalin (LYRICA), tramadol (ULTRAM, CONZIP), codeine, oxycodone (OXYCONTIN, ROXICODONE), hydrocodone (HYSINGLA ER, ZOHYDRO ER), muscle relaxants, and sleep medications.

All references, including publications, patent applications, and patents, cited herein are hereby incorporated by reference to the same extent as if each reference were individually and specifically indicated to be incorporated by reference and were set forth in its entirety herein.

The use of the terms ^{"}a^{"} and ^{"}an^{"} and ^{"}the^{"} and ^{"}at least one^{"} and similar referents in the context of describing the invention (especially in the context of the following claims) are to be construed to cover both the singular and the plural, unless otherwise indicated herein or clearly contradicted by context. The use of the term ^{"}at least one^{"} followed by a list of one or more items (for example, ^{"}at least one of A and B^{"}) is to be construed to mean one item selected from the listed items (A or B) or any combination of two or more of the listed items (A and B), unless otherwise indicated herein or clearly contradicted by context. The terms ^{"}comprising,^{" "}having,^{" "}including,^{"} and ^{"}containing^{"} are to be construed as open-ended terms (i.e., meaning ^{"}including, but not limited to,^{"}) unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., ^{"}such as^{"}) provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

The following embodiments are provided:
1. A method for increasing blood-brain barrier permeability in a subject, which comprises administering to the subject an agent which inhibits the activity of alkaline phosphatase protein (ALPL).
2. A method for delivering a therapeutic agent to the brain of a subject in need thereof, which comprises administering to the subject: (a) an agent which inhibits the activity of alkaline phosphatase protein (ALPL) in the brain; and (b) the therapeutic agent.
3. The method of embodiment 2, wherein the therapeutic agent is a macromolecule.
4. The method of embodiment 3, wherein the macromolecule is a protein.
5. The method of embodiment 4, wherein the macromolecule is an antibody.
6. The method of embodiment 2, wherein the therapeutic agent is a small molecule drug.
7. The method of any one of embodiments 1-6, wherein the agent is a small molecule inhibitor of alkaline phosphatase protein (ALPL).
8. The method of any one of embodiments 1-7, wherein the subject suffers from a central nervous system (CNS) disease.
9. The method of embodiment 8, wherein the central nervous system disease is a metabolic disease, trauma, a behavioral disorder, a personality disorder, dementia, a cancer, an infection, an autoimmune disease, a vascular disease, a sleep disorder, or a neurodegenerative disease.
10. The method of embodiment 8 or embodiment 9, wherein the central nervous system disease is restless leg syndrome (RLS).
11. The method of any one of embodiments 1-10, wherein the subject is 50 years of age or older.
12. A method for identifying a biomolecule that regulates blood-brain barrier permeability in a mammal, which method comprises:
   (a) detectably labeling biomolecules of blood plasma isolated from a first mammal;
   (b) introducing the isolated blood plasma comprising the labeled biomolecules into a second mammal;
   (c) isolating brain endothelial cells from the second mammal that form the blood-brain barrier;
   (d) measuring plasma uptake in each of the isolated brain endothelial cells using flow cytometry to produce a population of sorted brain endothelial cells;
   (e) detecting expression of genes that correlate with plasma uptake in each of the sorted brain endothelial cells; and
   (f) selecting a biomolecule encoded by a gene whose expression correlates with increased or decreased plasma uptake in a brain endothelial cell, whereby the biomolecule regulates blood-brain barrier permeability in the subject.
13. The method of embodiment 12, wherein the biomolecules of the isolated blood plasma are fluorescently labeled.
14. The method of embodiment 12 or embodiment 13, wherein expression of genes is detected using single cell RNA sequencing (scRNA-seq).
15. The method of any one of embodiments 12-14, wherein the mammal is a mouse.
16. The method of any one of embodiments 12-15, wherein the biomolecule increases blood-brain barrier permeability.
17. The method of any one of embodiments 12-15, wherein the biomolecule reduces blood-brain barrier permeability.
18. The method of any one of embodiments 12-17, wherein the biomolecule is a protein or peptide.
19. The method of any one of embodiments 12-17, wherein the biomolecule is a nucleic acid.

### References

1. Obermeier, B., Daneman, R. & Ransohoff, R. M. Development, maintenance and disruption of the blood-brain barrier. Nature Medicine (2013). doi:10.1038/nm.3407
2. Ballabh, P., Braun, A. & Nedergaard, M. The blood-brain barrier: an overview: structure, regulation, and clinical implications. Neurobiol. Dis. (2004). doi:10.1016/j.nbd.2003.12.016
3. Chow, B. W. & Gu, C. The Molecular Constituents of the Blood-Brain Barrier. Trends Neurosci. 38, 598-608 (2015).
4. Blood, T., Barrier, B., Daneman, R. & Prat, A. The Blood -Brain Barrier. 1-23 (2015).
5. Reese, T. S. & Karnovsky, M. J. Fine structural localization of a blood-brain barrier to exogenous peroxidase. J. Cell Biol. (1967). doi:10.1083/jcb.34.1.207
6. Andreone, B. J. et al. Blood-Brain Barrier Permeability Is Regulated by Lipid Transport-Dependent Suppression of Caveolae-Mediated Transcytosis. Neuron (2017). doi:10.1016/j.neuron.2017.03.043
7. Park, M. H. et al. Vascular and Neurogenic Rejuvenation in Aging Mice by Modulation of ASM. Neuron (2018). doi:10.1016/j.neuron.2018.09.010
8. Yao, Y., Chen, Z. L., Norris, E. H. & Strickland, S. Astrocytic laminin regulates pericyte differentiation and maintains blood brain barrier integrity. Nat. Commun. (2014). doi:10.1038/ncomms4413
9. Janzer, R. C. & Raff, M. C. Astrocytes induce blood-brain barrier properties in endothelial cells. Nature (1987). doi:10.1038/325253a0
10. Saunders, N. R., Dziegielewska, K. M., Mollgard, K. & Habgood, M. D. Markers for blood-brain barrier integrity: How appropriate is Evans blue in the twenty-first century and what are the alternatives? Frontiers in Neuroscience (2015). doi:10.3389/fnins.2015.00385
11. Conboy, I. M. et al. Rejuvenation of aged progenitor cells by exposure to a young systemic environment. Nature 433, 760-764 (2005).
12. Villeda, S. A. et al. Young blood reverses age-related impairments in cognitive function and synaptic plasticity in mice. Nat. Med. (2014). doi:10.1038/nm.3569
13. Tingle et al. Human umbilical cord plasma proteins revitalize hippocampal function in aged mice. Nature (2017). doi:doi:10.1038/nature22067
14. Wyss-Coray, T. Ageing, neurodegeneration and brain rejuvenation. Nature 539, 180-186 (2016).
15. Katsimpardi, L. et al. Vascular and neurogenic rejuvenation of the aging mouse brain by young systemic factors. Science (80-. ). (2014). doi:10.1126/science.1251141
16. Karnavas, T. et al. Gpr158 mediates osteocalcin's regulation of cognition. J. Exp. Med. (2017). doi:10.1084/jem.20171320
17. Gan, K. J. & Sudhof, T. C. Specific factors in blood from young but not old mice directly promote synapse formation and NMDA-receptor recruitment. Proc. Natl. Acad. Sci. 116, 201902672 (2019).
18. Pardridge, W. M. The blood-brain barrier: Bottleneck in brain drug development. NeuroRx (2005). doi:10.1602/neurorx.2.1.3
19. Abbott, N. J., Patabendige, A. A. K., Dolman, D. E. M., Yusof, S. R. & Begley, D. J. Structure and function of the blood-brain barrier. Neurobiol. Dis. (2010). doi:10.1016/j.nbd.2009.07.030
20. Banks, W. A. Characteristics of compounds that cross the blood-brain barrier. in BMC Neurology (2009). doi:10.1186/1471-2377-9-S1-S3
21. St-Amour, I. et al. Brain bioavailability of human intravenous immunoglobulin and its transport through the murine blood-brain barrier. J. Cereb. Blood Flow Metab. (2013). doi:10.1038/jcbfm.2013.160
22. Anderson, N. L. & Anderson, N. G. The Human Plasma Proteome. Mol. Cell. Proteomics (2002). doi:10.1074/mcp.R200007-MCP200
23. Hood, B. L. et al. Investigation of the mouse serum proteome. J. Proteome Res. (2005). doi:10.1021/pr050107r
24. Bragg, P. D. & Hou, C. Subunit composition, function, and spatial arrangement in the Ca2+- and Mg2+-activated adenosine triphosphatases of Escherichia coli and Salmonella typhimurium. Arch. Biochem. Biophys. (1975). doi:10.1016/0003-9861(75)90467-1
25. Selo, I., Negroni, L., Creminon, C., Grassi, J. & Wal, J. M. Preferential labeling of α-amino N terminal groups in peptides by biotin: Application to the detection of specific anti-peptide antibodies by enzyme immunoassays. J. Immunol. Methods (1996). doi:10.1016/S0022-1759(96)00173-1
26. Anderson, G. W., Zimmerman, J. E. & Callahan, F. M. The Use of Esters of N-Hydroxysuccinimide in Peptide Synthesis. J. Am. Chem. Soc. (1964). doi:10.1021/ja01063a037
27. Dempsey, G. T., Vaughan, J. C., Chen, K. H., Bates, M. & Zhuang, X. Evaluation of fluorophores for optimal performance in localization-based super-resolution imaging. Nat. Methods (2011). doi:10.1038/nmeth.1768
28. Han, Y., Li, M., Qiu, F., Zhang, M. & Zhang, Y. H. Cell-permeable organic fluorescent probes for live-cell long-term super-resolution imaging reveal lysosome-mitochondrion interactions. Nat. Commun. (2017). doi:10.1038/s41467-017-01503-6
29. Rubin, L. L. & Staddon, J. M. THE CELL BIOLOGY OF THE BLOOD-BRAIN BARRIER. Annu. Rev. Neurosci. (1999). doi:10.1146/annurev.neuro.22.1.11
30. Triguero, D., Buciak, J. B., Yang, J. & Pardridge, W. M. Blood-brain barrier transport of cationized immunoglobulin G: enhanced delivery compared to native protein. Proc. Natl. Acad. Sci. (1989). doi:10.1073/pnas.86.12.4761
31. Villasenor, R. et al. Trafficking of Endogenous Immunoglobulins by Endothelial Cells at the Blood-Brain Barrier. Sci. Rep. (2016). doi:10.1038/srep25658
32. Iliff, J. J. et al. A paravascular pathway facilitates CSF flow through the brain parenchyma and the clearance of interstitial solutes, including amyloid β. Sci. Transl. Med. (2012). doi:10.1126/scitranslmed.3003748
33. Louveau, A. et al. Structural and functional features of central nervous system lymphatic vessels. Nature 523, 337-341 (2015).
34. Da Mesquita, S. et al. Functional aspects of meningeal lymphatics in ageing and Alzheimer's disease. Nature (2018). doi:10.1038/s41586-018-0368-8
35. Pardridge, W. M., Eisenberg, J. & Yang, J. Human blood-brain barrier transferrin receptor. Metabolism (1987). doi:10.1016/0026-0495(87)90099-0
36. Poduslo, J. F., Curran, G. L. & Berg, C. T. Macromolecular permeability across the blood-nerve and blood-brain barriers. Proc. Natl. Acad. Sci. 91, 5705-5709 (1994).
37. Pardridge, W. M. Blood-brain barrier biology and methodology. Journal of NeuroVirology (1999). doi:10.3109/13550289909021285
38. Pardridge, W. M. Receptor-mediated peptide transport through the blood-brain barrier. Endocr. Rev. (1986). doi:10.1210/edrv-7-3-314
39. Pardridge, W. M. Blood-brain barrier delivery. Drug Discovery Today (2007). doi:10.1016/j.drudis.2006.10.013
40. Poduslo, J. F., Curran, G. L., Wengenack, T. M., Malester, B. & Duff, K. Permeability of proteins at the blood-brain barrier in the normal adult mouse and double transgenic mouse model of Alzheimer's disease. Neurobiol. Dis. (2001). doi:10.1006/nbdi.2001.0402
41. Villasenor, R., Schilling, M., Sundaresan, J., Lutz, Y. & Collin, L. Sorting Tubules Regulate Blood-Brain Barrier Transcytosis. Cell Rep. 21, 3256-3270 (2017).
42. Zuchero, Y. J. Y. et al. Discovery of Novel Blood-Brain Barrier Targets to Enhance Brain Uptake of Therapeutic Antibodies. Neuron (2016). doi:10.1016/j.neuron.2015.11.024
43. Couch, J. A. et al. Addressing safety liabilities of TfR bispecific antibodies that cross the blood-brain barrier. Sci. Transl. Med. (2013). doi:10.1126/scitranslmed.3005338
44. Atwal, J. K. et al. A therapeutic antibody targeting BACE1 inhibits amyloid-β production in vivo. Sci. Transl. Med. (2011). doi:10.1126/scitranslmed.3002254
45. Yu, Y. J. et al. Boosting brain uptake of a therapeutic antibody by reducing its affinity for a transcytosis target. Sci. Transl. Med. (2011). doi:10.1126/scitranslmed.3002230
46. Niewoehner, J. et al. Increased Brain Penetration and Potency of a Therapeutic Antibody Using a Monovalent Molecular Shuttle. Neuron (2014). doi:10.1016/j.neuron.2013.10.061
47. Jefferies, W. A. et al. Transferrin receptor on endothelium of brain capillaries. Nature (1984). doi:10.1038/312162a0
48. Broadwell, R. D. Transcytosis of macromolecules through the blood-brain barrier: a cell biological perspective and critical appraisal. Acta Neuropathologica (1989). doi:10.1007/BF00294368
49. Daneman, R. The blood-brain barrier in health and disease. Ann. Neurol. (2012). doi:10.1002/ana.23648
50. Zlokovic, B. V. The blood-brain barrier in health and chronic neurodegenerative disorders. Neuron (2008). doi:10.1016/j.neuron.2008.01.003
51. Zuchero, Y. J. Y. et al. Discovery of Novel Blood-Brain Barrier Targets to Enhance Brain Uptake of Therapeutic Antibodies. Neuron (2016). doi:10.1016/j.neuron.2015.11.024
52. Friden, P. M. et al. Anti-transferrin receptor antibody and antibody-drug conjugates cross the blood-brain barrier. Proc. Natl. Acad. Sci. (2006). doi:10.1073/pnas.88.11.4771
53. Bell, R. D. et al. Apolipoprotein e controls cerebrovascular integrity via cyclophilin A. Nature (2012). doi:10.1038/nature11087
54. Zlokovic, B. V. Cerebrovascular effects of apolipoprotein E: Implications for Alzheimer disease. JAMA Neurol. (2013). doi:10.1001/jamaneurol.2013.2152
55. Andreone, B. J. et al. Blood-Brain Barrier Permeability Is Regulated by Lipid Transport-Dependent Suppression of Caveolae-Mediated Transcytosis. Neuron 94, 581-594.e5 (2017).
56. Ben-Zvi, A. et al. Mfsd2a is critical for the formation and function of the blood-brain barrier. Nature (2014). doi:10.1038/nature13324
57. Daneman, R. et al. The mouse blood-brain barrier transcriptome: A new resource for understanding the development and function of brain endothelial cells. PLoS One (2010). doi:10.1371/journal.pone.0013741
58. Vanlandewijck, M. et al. A molecular atlas of cell types and zonation in the brain vasculature. Nature 554, 475-480 (2018).
59. Chen, M B. et al. Brain endothelial cells are exquisite sensors of age-related circulatory cues. bioRxiv (2019). doi:10.1101/617258
60. Simionescu, M., Simionescu, N. & Palade, G. E. Segmental differentiations of cell junctions in the vascular endothelium: Arteries and veins. J. Cell Biol. (1976). doi:10.1083/jcb.68.3.705
61. Tsafrir, D. et al. Sorting points into neighborhoods (SPIN): Data analysis and visualization by ordering distance matrices. Bioinformatics (2005). doi:10.1093/bioinformatics/bti329
62. Montagne, A. et al. Blood-Brain barrier breakdown in the aging human hippocampus. Neuron (2015). doi:10.1016/j.neuron.2014.12.032
63. Yousef, H. et al. Aged blood impairs hippocampal neural precursor activity and activates microglia via brain endothelial cell VCAM1. Nat. Med. (2019). doi:10.1038/s41591-019-0440-4
64. Ben-Zvi, A. et al. Mfsd2a is critical for the formation and function of the blood-brain barrier. Nature (2014). doi:10.1038/nature13324
65. Nguyen, L. N. et al. Mfsd2a is a transporter for the essential omega-3 fatty acid docosahexaenoic acid. Nature (2014). doi:10.1038/nature13241
66. Fishman, J. B., Rubin, J. B., Handrahan, J. V., Connor, J. R. & Fine, R. E. Receptor-mediated transcytosis of transferrin across the blood-brain barrier. J. Neurosci. Res. (1987). doi:10.1002/jnr.490180206
67. Roberts, R. L., Fine, R. E. & Sandra, A. Receptor-mediated endocytosis of transferrin at the bloodbrain barrier. J. Cell Sci. (1993).
68. Lajoie, J. M. & Shusta, E. V. Targeting Receptor-Mediated Transport for Delivery of Biologics Across the Blood-Brain Barrier. Annu. Rev. Pharmacol. Toxicol. (2014). doi:10.1146/annurevpharmtox-010814-124852
69. Schubert, W. et al. Caveolae-deficient Endothelial Cells Show Defects in the Uptake and Transport of Albumin in Vivo. J. Biol. Chem. (2001). doi:10.1074/jbc.C100613200
70. Nabi, I. R. & Le, P. U. Caveolae/raft-dependent endocytosis. Journal of Cell Biology (2003). doi:10.1083/jcb.200302028
71. Villasenor, R., Lampe, J., Schwaninger, M. & Collin, L. Intracellular transport and regulation of transcytosis across the blood-brain barrier. Cellular and Molecular Life Sciences (2019). doi:10.1007/s00018-018-2982-x
72. Spitzer, N., Sammons, G. S. & Price, E. M. Autofluorescent cells in rat brain can be convincing impostors in green fluorescent reporter studies. J. Neurosci. Methods (2011). doi:10.1016/j.jneumeth.2011.01.029
73. Schnell, S. A., Staines, W. A. & Wessendorf, M. W. Reduction of lipofuscin-like autofluorescence in fluorescently labeled tissue. J. Histochem. Cytochem. (1999). doi:10.1177/002215549904700601
74. Sonoda, H. et al. A Blood-Brain-Barrier-Penetrating Anti-human Transferrin Receptor Antibody Fusion Protein for Neuronopathic Mucopolysaccharidosis II. Mol. Ther. (2018). doi:10.1016/j.ymthe.2018.02.032
75. Bien-Ly, N. et al. Transferrin receptor (TfR) trafficking determines brain uptake of TfR antibody affinity variants. J. Exp. Med. (2014). doi:10.1084/jem.20131660
76. Oller-Salvia, B., Sanchez-Navarro, M., Giralt, E. & Teixido, M. Blood-brain barrier shuttle peptides: An emerging paradigm for brain delivery. Chemical Society Reviews (2016). doi:10.1039/c6cs00076b
77. Debray, J. et al. Inhibitors of tissue-nonspecific alkaline phosphatase: Design, synthesis, kinetics, biomineralization and cellular tests. Bioorganic Med. Chem. (2013). doi:10.1016/j.bmc.2013.09.053
78. Sheen, C. R. et al. Pathophysiological role of vascular smooth muscle alkaline phosphatase in medial artery calcification. J. Bone Miner. Res. (2015). doi:10.1002/jbmr.2420
79. Murshed, M., Harmey, D., Millan, J. L., McKee, M. D. & Karsenty, G. Unique coexpression in osteoblasts of broadly expressed genes accounts for the spatial restriction of ECM mineralization to bone. Genes Dev. (2005). doi:10.1101/gad.1276205
80. Villa-Bellosta, R. & O'Neill, W. C. Pyrophosphate deficiency in vascular calcification. Kidney International (2018). doi:10.1016/j.kint.2017.11.035
81. Savinov, A. Y. et al. Transgenic overexpression of tissue-nonspecific alkaline phosphatase (TNAP) in vascular endothelium results in generalized arterial calcification. J. Am. Heart Assoc. (2015). doi:10.1161/JAHA.115.002499
82. Romanelli, F. et al. Overexpression of tissue-nonspecific alkaline phosphatase (TNAP) in endothelial cells accelerates coronary artery disease in a mouse model of familial hypercholesterolemia. PLoS One (2017). doi:10.1371/journal.pone.0186426
83. Keller, A. et al. Mutations in the gene encoding PDGF-B cause brain calcifications in humans and mice. Nat. Genet. (2013). doi:10.1038/ng.2723
84. Vanlandewijck, M. et al. Functional characterization of germline mutations in PDGFB and PDGFRB in primary familial brain calcification. PLoS One (2015). doi:10.1371/journal.pone.0143407
85. Zarb, Y. et al. Ossified blood vessels in primary familial brain calcification elicit a neurotoxic astrocyte response. Brain (2019). doi:10.1093/brain/awz032
86. Sagare, A. P. et al. Pericyte loss influences Alzheimer-like neurodegeneration in mice. Nat. Commun. (2013). doi:10.1038/ncomms3932
87. Montagne, A. et al. Pericyte degeneration causes white matter dysfunction in the mouse central nervous system. Nat. Med. (2018). doi:10.1038/nm.4482
88. Bell, R. D. et al. Pericytes Control Key Neurovascular Functions and Neuronal Phenotype in the Adult Brain and during Brain Aging. Neuron (2010). doi:10.1016/j.neuron.2010.09.043
89. Dahl, R. et al. Discovery and validation of a series of aryl sulfonamides as selective inhibitors of tissue-nonspecific alkaline phosphatase (TNAP). J. Med. Chem. (2009). doi:10.1021/jm900383s
90. Armulik, A. et al. Pericytes regulate the blood-brain barrier. Nature 468, 557-561 (2010).
91. Armulik, A. et al. Pericytes regulate the blood-brain barrier. Nature (2010). doi:10.1038/nature09522
92. Su, E. J. et al. Activation of PDGF-CC by tissue plasminogen activator impairs blood-brain barrier integrity during ischemic stroke. Nat. Med. (2008). doi:10.1038/nm1787
93. Junger, M. A. & Aebersold, R. Mass spectrometry-driven phosphoproteomics: Patterning the systems biology mosaic. Wiley Interdisciplinary Reviews: Developmental Biology (2014). doi:10.1002/wdev.121
94. Olsen, J. V et al. Quantitative phosphoproteomics reveals widespread full phosphorylation site occupancy during mitosis. Sci. Signal. (2010). doi:10.1126/scisignal.2000475
95. Sharma, K. et al. Ultradeep Human Phosphoproteome Reveals a Distinct Regulatory Nature of Tyr and Ser/Thr-Based Signaling. Cell Rep. (2014). doi:10.1016/j.celrep.2014.07.036
96. Haqqani, A. S. et al. Intracellular sorting and transcytosis of the rat transferrin receptor antibody OX26 across the blood-brain barrier in vitro is dependent on its binding affinity. J. Neurochem. (2018). doi:10.1111/jnc.14482
97. Johnsen, K. B. et al. Antibody affinity and valency impact brain uptake of transferrin receptortargeted gold nanoparticles. Theranostics (2018). doi:10.7150/thno.25228
98. Pardridge, W. M. Drug transport across the blood-brain barrier. Journal of Cerebral Blood Flow and Metabolism (2012). doi:10.1038/jcbfm.2012.126
99. Banks, W. A. From blood-brain barrier to blood-brain interface: New opportunities for CNS drug delivery. Nature Reviews Drug Discovery (2016). doi:10.1038/nrd.2015.21
100. Karnovsky, M. J. The ultrastructural basis of capillary permeability studied with peroxidase as a tracer. J. Cell Biol. (1967). doi:10.1083/jcb.35.1.213
101. Saunders, N. R., Habgood, M. D., Mollgard, K. & Dziegielewska, K. M. The biological significance of brain barrier mechanisms: help or hindrance in drug delivery to the central nervous system? F1000Researeh (2016). doi:10.12688/f1000research.7378.1
102. Liddelow, S. A. Fluids and barriers of the CNS: A historical viewpoint. Fluids and Barriers of the CNS (2011). doi:10.1186/2045-8118-8-2
103. Nadal, A., Fuentes, E., Pastor, J. & McNaughton, P. A. Plasma albumin is a potent trigger of calcium signals and DNA synthesis in astrocytes. Proc. Natl. Acad. Sci. (2006). doi:10.1073/pnas.92.5.1426
104. Gingrich, M. B. & Traynelis, S. F. Serine proteases and brain damage - Is there a link? Trends in Neurosciences (2000). doi:10.1016/S0166-2236(00)01617-9
105. Petersen, M. A., Ryu, J. K. & Akassoglou, K. Fibrinogen in neurological diseases: Mechanisms, imaging and therapeutics. Nature Reviews Neuroscience (2018). doi:10.1038/nrn.2018.13
106. Davalos, D. et al. Fibrinogen-induced perivascular microglial clustering is required for the development of axonal damage in neuroinflammation. Nat. Commun. (2012). doi:10.103 8/ncomms2230
107. Davalos, D. & Akassoglou, K. Fibrinogen as a key regulator of inflammation in disease. Seminars in Immunopathology (2012). doi:10.1007/s00281-011-0290-8
108. Sweeney, M. D., Sagare, A. P. & Zlokovic, B. V. Blood-brain barrier breakdown in Alzheimer disease and other neurodegenerative disorders. Nat. Rev. Neurol. (2018). doi:10.1038/nrneurol.2017.188
109. Xiao, G. & Gan, L.-S. Receptor-Mediated Endocytosis and Brain Delivery of Therapeutic Biologics. Int. J. Cell Biol. (2013). doi:10.1155/2013/703545
110. Daneman, R., Zhou, L., Kebede, A. A. & Barres, B. A. Pericytes are required for blood-brain barrier integrity during embryogenesis. Nature 468, 562-566 (2010).
111. Ammanamanchi, M., Li, X., Li, X., Qin, Z. & Hayenga, H. Substrate stiffness modulates the route of transport and not the permeability of the human brain endothelium. in P2574 (8th World Congress of Biomechanics, 2018).
112. Zlokovic, B. V. Neurovascular pathways to neurodegeneration in Alzheimer's disease and other disorders. Nature Reviews Neuroscience (2011). doi:10.1038/nrn3114
113. Nelson, A. R., Sweeney, M. D., Sagare, A. P. & Zlokovic, B. V. Neurovascular dysfunction and neurodegeneration in dementia and Alzheimer's disease. Biochim. Biophys. Acta - Mol. Basis Dis. (2016). doi:10.1016/j.bbadis.2015.12.016
114. Harold, D. et al. Genome-wide association study identifies variants at CLU and PICALM associated with Alzheimer's disease. Nat. Genet. (2009). doi:10.1038/ng.440
115. Vardy, E. R. L. C., Kellett, K. A. B., Cocklin, S. L. & Hooper, N. M. Alkaline Phosphatase Is Increased in both Brain and Plasma in Alzheimer's Disease. Neurodegener. Dis. (2011). doi:10.1159/000329722
116. Kellett, K. A. B. & Hooper, N. M. The role of tissue non-specific alkaline phosphatase (TNAP) in neurodegenerative diseases: Alzheimer's disease in the focus. Subcell. Biochem. (2015). doi:10.1007/978-94-017-7197-9_17
117. Murakami, Y. et al. Mechanism for release of alkaline phosphatase caused by glycosylphosphatidylinositol deficiency in patients with hyperphosphatasia mental retardation syndrome. J. Biol. Chem. (2012). doi:10.1074/jbc.M111.331090
118. Iadecola, C. The Pathobiology of Vascular Dementia. Neuron (2013). doi:10.1016/j.neuron.2013.10.008
119. Muhire, G. et al. Arterial Stiffness Due to Carotid Calcification Disrupts Cerebral Blood Flow Regulation and Leads to Cognitive Deficits. J. Am. Heart Assoc. 8, (2019).
120. Kapasi, A. & Schneider, J. A. Vascular contributions to cognitive impairment, clinical Alzheimer's disease, and dementia in older persons. Biochim. Biophys. Acta - Mol. Basis Dis. (2016). doi:10.1016/j.bbadis.2015.12.023
121. Iulita, M. F., Noriega de la Colina, A. & Girouard, H. Arterial stiffness, cognitive impairment and dementia: confounding factor or real risk? Journal of Neurochemistry (2018). doi:10.1111/jnc.14235
122. Iadecola, C. & Davisson, R. L. Hypertension and Cerebrovascular Dysfunction. Cell Metabolism (2008). doi:10.1016/j.cmet.2008.03.010
123. Louveau, A., Harris, T. H. & Kipnis, J. Revisiting the Mechanisms of CNS Immune Privilege. Trends in Immunology (2015). doi:10.1016/j.it.2015.08.006
124. Da Mesquita, S., Fu, Z. & Kipnis, J. The Meningeal Lymphatic System: A New Player in Neurophysiology. Neuron (2018). doi:10.1016/j.neuron.2018.09.022
125. Zlokovic, B. V., Deane, R., Sagare, A. P., Bell, R. D. & Winkler, E. A. Low-density lipoprotein receptor-related protein-1: A serial clearance homeostatic mechanism controlling Alzheimer's amyloid β-peptide elimination from the brain. J. Neurochem. (2010). doi:10.1111/j. 1471-4159.2010.07002.x
126. Ben-Zvi, A. et al. Mfsd2a is critical for the formation and function of the blood-brain barrier. Nature 509, 507-511 (2014).
127. Yang, A. C. et al. Multiple Click-Selective tRNA Synthetases Expand Mammalian Cell-Specific Proteomics. J. Am. Chem. Soc. (2018). doi:10.1021/jacs.8b03074
128. Hughes, C. S. et al. Ultrasensitive proteome analysis using paramagnetic bead technology. Mol. Syst. Biol. (2014). doi:10.15252/msb.20145625
129. Cox, J. & Mann, M. MaxQuant enables high peptide identification rates, individualized p.p.b.-range mass accuracies and proteome-wide protein quantification. Nat. Biotechnol. (2008). doi:10.1038/nbt.1511
130. Tyanova, S., Temu, T. & Cox, J. The MaxQuant computational platform for mass spectrometrybased shotgun proteomics. Nat. Protoc. (2016). doi:10.1038/nprot.2016.136
131. Treweek, J. B. et al. Whole-body tissue stabilization and selective extractions via tissue-hydrogel hybrids for high-resolution intact circuit mapping and phenotyping. Nat. Protoc. (2015). doi:10.1038/nprot.2015.122
132. Tournoux, F. et al. Validation of noninvasive measurements of cardiac output in mice using echocardiography. J. Am. Soc. Echocardiogr. (2011). doi:10.1016/j.echo.2010.12.019
133. Dominguez, E. et al. Non-invasive in vivo measurement of cardiac output in C57BL/6 mice using high frequency transthoracic ultrasound: evaluation of gender and body weight effects. Int. J. Cardiovasc. Imaging (2014). doi:10.1007/s105 54-014-0454-4
134. Ferreira, C. L. et al. Comparison of bifunctional chelates for 64Cu antibody imaging. Eur. J. Nucl. Med. Mol. Imaging (2010). doi:10.1007/s00259-010-1506-1
135. Ilovich, O. et al. Development and Validation of an Immuno-PET Tracer as a Companion Diagnostic Agent for Antibody-Drug Conjugate Therapy to Target the CA6 Epitope. Radiology (2015). doi:10.1148/radiol.15140058
136. Chaney, A. et al. 11 C-DPA-713 Versus 18 F-GE-180: A Preclinical Comparison of Translocator Protein 18 kDa PET Tracers to Visualize Acute and Chronic Neuroinflammation in a Mouse Model of Ischemic Stroke . J. Nucl. Med. (2018). doi:10.2967/jnumed.118.209155
137. Chaney, A. M., Johnson, E. M., Cropper, H. C. & James, M. L. PET Imaging of Neuroinflammation Using [11C]DPA-713 in a Mouse Model of Ischemic Stroke. J. Vis. Exp. (2018). doi:10.3791/57243
138. James, M. L. et al. New positron emission tomography (PET) radioligand for imaging σ-1 receptors in living subjects. J. Med. Chem. (2012). doi:10.1021/jm300371c
139. Yousef, H. et al. Aged blood inhibits hippocampal neurogenesis and activates microglia through VCAM1 at the blood-brain barrier. bioRxiv (2018). doi:1 0.1101/242198
140. Schaum, N. et al. Single-cell transcriptomics of 20 mouse organs creates a Tabula Muris. Nature (2018). doi:10.1038/s41586-018-0590-4
141. Picelli, S. et al. Full-length RNA-seq from single cells using Smart-seq2. Nat. Protoc. (2014). doi:10.1038/nprot.2014.006
142. Wu, A. R. et al. Quantitative assessment of single-cell RNA-sequencing methods. Nat. Methods (2014). doi:10.1038/nmeth.2694
143. Yanagida, K. et al. Size-selective opening of the blood-brain barrier by targeting endothelial sphingosine 1-phosphate receptor 1. Proc. Natl. Acad. Sci. (2017). doi:10.1073/pnas.1618659114
144. Triguero, D., Buciak, J. & Pardridge, W. M. Capillary Depletion Method for Quantification of Blood-Brain Barrier Transport of Circulating Peptides and Plasma Proteins. J. Neurochem. (1990). doi:10.1111/j.1471-4159.1990.tb04886.x
145. Singh, I. et al. Low levels of copper disrupt brain amyloid-β homeostasis by altering its production and clearance. Proc. Natl. Acad. Sci. (2013). doi:10.1073/pnas.1302212110
146. Paris-Robidas, S., Brouard, D., Emond, V., Parent, M. & Calon, F. Internalization of targeted quantum dots by brain capillary endothelial cells in vivo. J. Cereb. Blood Flow Metab. (2015). doi:10.1177/0271678X15608201
147. Preston, J. E., Al-Sarraf, H. & Segal, M. B. Permeability of the developing blood-brain barrier to 14C-mannitol using the rat in situ brain perfusion technique. Dev. Brain Res. (1995). doi:10.1016/0165-3806(95)00060-Q
148. Dan, M., Cochran, D. B., Yokel, R. A. & Dziubla, T. D. Binding, transcytosis and biodistribution of anti-PECAM-1 iron oxide nanoparticles for brain-targeted delivery. PLoS One (2013). doi:10.1371/journal.pone.0081051
149. Cajka, T., Smilowitz, J. T. & Fiehn, O. Validating Quantitative Untargeted Lipidomics Across Nine Liquid Chromatography-High-Resolution Mass Spectrometry Platforms. Anal. Chem. (2017). doi:10.1021/acs.analchem.7b03404
150. Contrepois, K. et al. Cross-Platform Comparison of Untargeted and Targeted Lipidomics Approaches on Aging Mouse Plasma. Sci. Rep. (2018). doi:10.1038/s41598-018-35807-4
151. Luo, J. et al. Long-term cognitive impairments and pathological alterations in a mouse model of repetitive mild traumatic brain injury. Front. Neurol. (2014). doi:10.3389/fneur.2014.00012
152. Kadakkuzha, B. M. et al. Transcriptome analyses of adult mouse brain reveal enrichment of lncRNAs in specific brain regions and neuronal populations. Front. Cell. Neurosci. (2015). doi:10.3389/fncel.2015.00063
153. Sharma, K. et al. Cell type- and brain region-resolved mouse brain proteome. Nat. Neurosci. 18, 1819-1831 (2015).
154. Dougherty, J. D., Schmidt, E. F., Nakajima, M. & Heintz, N. Analytical approaches to RNA profiling data for the identification of genes enriched in specific cells. Nucleic Acids Res. (2010). doi:10.1093/nar/gkq130
155. Ashburner, M. et al. Gene ontology: Tool for the unification of biology. Nature Genetics (2000). doi:10.1038/75556
156. Gene Ontology Consortium. The Gene Ontology (GO) database and informatics resource. Nucleic Acids Res. (2004). doi:10.1093/nar/gkh036
157. Zhang, X. D. et al. Traumatic Brain Injury Imaging in the Second Near-Infrared Window with a Molecular Fluorophore. Adv. Mater. (2016). doi:10.1002/adma.201600706
158. Kiick, K. L., Saxon, E., Tirrell, D. A. & Bertozzi, C. R. Incorporation of azides into recombinant proteins for chemoselective modification by the Staudinger ligation. Proc. Natl. Acad. Sci. 99, 19-24 (2002).
159. Calve, S., Witten, A. J., Ocken, A. R. & Kinzer-Ursem, T. L. Incorporation of non-canonical amino acids into the developing murine proteome. Sci. Rep. (2016). doi:10.1038/srep32377
160. Liu, A. P., Aguet, F., Danuser, G. & Schmid, S. L. Local clustering of transferrin receptors promotes clathrin-coated pit initiation. J. Cell Biol. (2010). doi:10.1083/jcb.201008117
161. Sabbagh, M. F. et al. Transcriptional and epigenomic landscapes of CNS and non-CNS vascular endothelial cells. Elife (2018). doi:10.7554/elife.36187
162. Simionescu, M. et al. The cerebral microvasculature of the rat: structure and luminal surface properties during early development. J. Submicrosc. Cytol. Pathol. (1988).
163. Herve, F., Ghinea, N. & Scherrmann, J.-M. CNS Delivery Via Adsorptive Transcytosis. AAPSJ. (2008). doi:10.1208/s12248-008-9055-2
164. Montagne, A., Zhao, Z. & Zlokovic, B. V. Alzheimer's disease: A matter of blood-brain barrier dysfunction? J. Exp. Med. (2017). doi:10.1084/jem.20171406
165. Anstrom, J. A. et al. Anatomical analysis of the developing cerebral vasculature in premature neonates: Absence of precapillary arteriole-to-venous shunts. Pediatr. Res. (2002). doi:10.1203/00006450-200210000-00015
166. Norman, M. G. & O'Kusky, J. R. The growth and development of microvasculature in human cerebral cortex. J. Neuropathol. Exp. Neurol. (1986). doi:10.1097/00005072-198605000-00004
167. Bien-Ly, N. et al. Lack of Widespread BBB Disruption in Alzheimer's Disease Models: Focus on Therapeutic Antibodies. Neuron 88, 289-297 (2015).
168. Pardridge, W. M, Buciak, J. L. & Friden, P. M. Selective transport of an anti-transferrin receptor antibody through the blood-brain barrier in vivo. J. Pharmacol. Exp. Ther. (1991).
169. Villasenor, R. et al. Trafficking of Endogenous Immunoglobulins by Endothelial Cells at the Blood-Brain Barrier. Sci. Rep. 6, 1-10 (2016).
170. Pappolla, M. A. & Andorn, A. C. Serum protein leakage in aged human brain and inhibition of ligand binding at alpha2- adrenergic and cholinergic binding sites. Synapse (1987). doi:10.1002/syn. 890010111
171. Harrison, L. et al. Fluorescent blood-brain barrier tracing shows intact leptin transport in obese mice. International Journal of Obesity (2018). doi:10.1038/s41366-018-0221-z
172. Vazana, U. et al. Glutamate-Mediated Blood-Brain Barrier Opening: Implications for Neuroprotection and Drug Delivery. J. Neurosci. (2016). doi:10.1523/jneurosci.0587-16.2016
173. Armulik, A., Genove, G. & Betsholtz, C. Pericytes: Developmental, Physiological, and Pathological Perspectives, Problems, and Promises. Developmental Cell (2011). doi:10.1016/j.devcel.2011.07.001
174. Nagatsu-Ishibashi, I., Nagatsu, T. & Yagi, K. Changes of quantity of flavin in the brain after peripheral administration of flavins. Nature (1961). doi:10.1038/190728a0
175. Tiwary, S. et al. Metastatic brain tumors disrupt the blood-brain barrier and alter lipid metabolism by inhibiting expression of the endothelial cell fatty acid transporter Mfsd2a. Sci. Rep. (2018). doi:10.1038/s41598-018-26636-6
176. Schlageter, N. L., Carson, R. E. & Rapoport, S. I. Examination of blood-brain barrier permeability in dementia of the Alzheimer type with [68Ga]EDTA and positron emission tomography. J. Cereb. Blood Flow Metab. (1987). doi:10.1038/jcbfm.1987.1
177. Montagne, A. et al. Blood-Brain barrier breakdown in the aging human hippocampus. Neuron 85, 296-302 (2015).
178. van de Haar, H. J. et al. Neurovascular unit impairment in early Alzheimer's disease measured with magnetic resonance imaging. Neurobiol. Aging (2016). doi:10.1016/j.neurobiolaging.2016.06.006
179. Miller, J. J. et al. 13C Pyruvate Transport Across the Blood-Brain Barrier in Preclinical Hyperpolarised MRI. Sci. Rep. (2018). doi:10.1038/s41598-018-33363-5
180. Hunt, A. et al. Reduced cerebral glucose metabolism in patients at risk for Alzheimer's Disease Psychiatry Res. - Neuroimaging (2007). doi:10.1016/j.pscychresns.2006.12.003
181. Allen, R. P. et al. Restless Legs Syndrome Prevalence and Impact. Arch. Intern. Med. (2005). doi:10.1001/archinte.165.11.1286
182. Innes, K. E., Selfe, T. K. & Agarwal, P. Prevalence of restless legs syndrome in North American and Western European populations: A systematic review. Sleep Medicine (2011). doi:10.1016/j.sleep.2010.12.018
183. Ohayon, M. M., O'Hara, R. & Vitiello, M. V. Epidemiology of restless legs syndrome: A synthesis of the literature. Sleep Medicine Reviews (2012). doi:10.1016/j.smrv.2011.05.002
184. Milligan, S. A. & Chesson, A. L. Restless legs syndrome in the older adult: Diagnosis and management. Drugs and Aging (2002). doi:10.2165/00002512-200219100-00003
185. Mills, E., Dong, X. P., Wang, F. & Xu, H. Mechanisms of brain iron transport: Insight into neurodegeneration and CNS disorders. Future Medicinal Chemistry (2010). doi:10.4155/fmc.09.140
186. Chiou, B. et al. Pharmaceutical iron formulations do not cross a model of the human blood-brain barrier. PLoS One (2018). doi:10.1371/journal.pone.0198775
187. Mizuno, S., Mihara, T., Miyaoka, T., Inagaki, T. & Horiguchi, J. CSF iron, ferritin and transferrin levels in restless legs syndrome. J. Sleep Res. (2005). doi:10.1111/j.1365-2869.2004.00403.x
189. Connor, J. R. et al. Profile of altered brain iron acquisition in restless legs syndrome. Brain (2011). doi:10.1093/brain/awr012

## Claims

1. SLC16a1 for use in increasing blood-brain barrier permeability in a subject, wherein said subject is a patient in need thereof.

2. The SLC16a1 for use of claim 1, wherein said SLC16a1 is further for use in delivering a therapeutic agent to the brain of said subject in need thereof.

3. The SLC16a1 for use of claim 1 or claim 2, wherein the subject suffers from a central nervous system (CNS) disease.

4. The SLC16a1 for use of claim 3, wherein the central nervous system disease is a metabolic disease, trauma, a behavioral disorder, a personality disorder, dementia, a cancer, an infection, an autoimmune disease, a vascular disease, a sleep disorder, a genetic disease, or a neurodegenerative disease.

5. The SLC16a1 for use of claim 4, wherein the central nervous system disease is amyotrophic lateral sclerosis (ALS).

6. Monocarboxylate transporter 1 for use in increasing blood-brain barrier permeability in a subject, wherein said subject is a patient in need thereof.

7. The monocarboxylate transporter 1 for use of claim 6, wherein said monocarboxylate transporter is further for use in delivering a therapeutic agent to the brain of said subject in need thereof.

8. The monocarboxylate transporter 1 for use of claim 6 or claim 7, wherein the subject suffers from a central nervous system (CNS) disease.

9. The monocarboxylate transporter! for use of claim 8, wherein the central nervous system disease is a metabolic disease, trauma, a behavioral disorder, a personality disorder, dementia, a cancer, an infection, an autoimmune disease, a vascular disease, a sleep disorder, a genetic disease, or a neurodegenerative disease.

10. The monocarboxylate transporter 1 for use of claim 9, wherein the central nervous system disease is amyotrophic lateral sclerosis (ALS).
